# EUROPEAN PATENT APPLICATION

(11) **EP 4 603 562 A2**
(43) Date of publication of application: **20.08.2025**
(21) Application number: 25186179.5
(22) Date of filing: 25.07.2018
(51) Int. Cl.: C09K 11/06

(54) **SPIROBIFLUORENE DERIVATIVES FOR USE IN ELECTRONIC DEVICES**

(30) Priority: 28.07.2017 EP 17183663
(62) Divisional of application: 18753070.4
(71) Applicant: Merck Patent GmbH, 64293 Darmstadt (DE)
(72) Inventor: Montenegro, Elvira, 64293 Darmstadt (DE); Mujica-Fernaud, Teresa, 64293 Darmstadt (DE); Maier-Flaig, Florian, 64293 Darmstadt (DE); Voges, Frank, 64293 Darmstadt (DE)
(74) Representative: Merck Patent Association

(57) **Abstract**

The present application relates to a spirobifluorene derivative of a specific formula which is suitable for use in electronic devices.

## Description

The present application relates to a spirobifluorene derivative of a formula (I) defined hereinafter which is suitable for use in electronic devices, especially organic electroluminescent devices (OLEDs).

Electronic devices in the context of this application are understood to mean what are called organic electronic devices, which contain organic semiconductor materials as functional materials. More particularly, these are understood to mean OLEDs.

The construction of OLEDs in which organic compounds are used as functional materials is common knowledge in the prior art. In general, the term OLEDs is understood to mean electronic devices which have one or more layers comprising organic compounds and emit light on application of electrical voltage.

In electronic devices, especially OLEDs, there is great interest in improving the performance data, especially lifetime, efficiency and operating voltage. In these aspects, it has not yet been possible to find any entirely satisfactory solution.

A great influence on the performance data of electronic devices is possessed by layers having a hole-transporting function, for example hole-injecting layers, hole transport layers, electron blocking layers and also emitting layers. For use in these layers, there is a continuous search for new materials having hole-transporting properties.

In the course of the present invention, it has been found that spirobifluorene derivatives which have an amine or bridged amine group in the 2-position, and a further substituent which is selected from particular chemical groups in one of the 5, 6, and 8-position of the spirobifluorene, are very well suited for use as materials with hole transporting function, in particular for use as materials of the hole transporting layer, the electron blocking layer and the emitting layer, more particularly for use in the electron blocking layer. An electron blocking layer is understood in this context to be a layer which is directly adjacent to the emitting layer on the anode side, and which serves to block electrons which are present in the emitting layer from entering the hole transporting layers of the OLED.

When used in electronic devices, in particular in OLEDs, they lead to excellent results in terms of lifetime, operating voltage and quantum efficiency of the devices. The compounds are also characterized by very good hole-conducting properties, very good electron-blocking properties, high glass transition temperature, high oxidation stability, good solubility, high thermal stability, and low sublimation temperature.

The present application therefore relates to a compound of the formula (I) 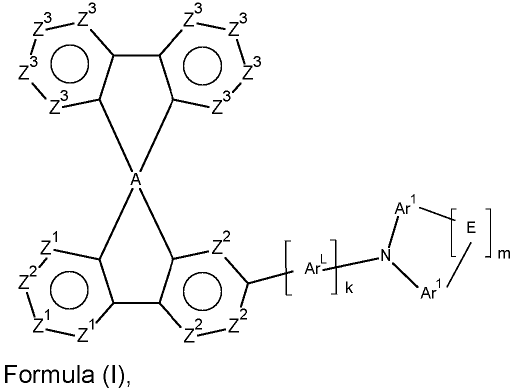 where the variables are defined as follows:
- A: is C or Si;
- Z¹: is, identically or differently on each occurrence, selected from CR¹, CR² and N;
- Z²: is, identically or differently on each occurrence, selected from CR² and N;
- Z³: is, identically or differently on each occurrence, selected from CR³ and N;
- Ar^{L}: is, identically or differently on each occurrence, selected from aromatic ring systems having 6 to 40 aromatic ring atoms, which may be substituted by one or more radicals R⁴, and heteroaromatic ring systems having 5 to 40 aromatic ring atoms, which may be substituted by one or more radicals R⁴;
- Ar¹: is, identically or differently, selected from aromatic ring systems having 6 to 40 aromatic ring atoms, which may be substituted by one or more radicals R⁴, and heteroaromatic ring systems having 5 to 40 aromatic ring atoms, which may be substituted by one or more radicals R⁴;
- E: is a single bond or is a divalent group selected from C(R⁴)₂, N(R⁴), O, and S;
- R¹: is selected, identically or differently on each occurrence, from 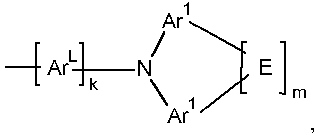 Si(R⁵)₃, straight-chain alkyl, alkoxy or thioalkyl groups having 1 to 20 C atoms, branched or cyclic alkyl, alkoxy or thioalkyl groups having 3 to 20 C atoms, aromatic ring systems having 6 to 40 aromatic ring atoms, and heteroaromatic ring systems having 5 to 40 aromatic ring atoms, where the said alkyl, alkoxy and thioalkyl groups and the said aromatic and heteroaromatic ring systems may in each case be substituted by one or more radicals R⁵;
- R², R³: are selected, identically or differently on each occurrence, from H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, SCN, SF₅, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals selected from radicals R² and R³ may be connected to each other to form a ring; where the said alkyl, alkoxy, alkenyl and alkynyl groups and the said aromatic and heteroaromatic ring systems may in each case be substituted by one or more radicals R⁵, and where one or more CH₂ groups in the said alkyl, alkoxy, alkenyl and alkynyl groups may in each case be replaced by -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO or SO₂;
- R⁴: is, identically or differently at each occurrence, selected from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁴ may be connected to each other to form a ring; where the said alkyl, alkoxy, alkenyl and alkynyl groups and the said aromatic and heteroaromatic ring systems may in each case be substituted by one or more radicals R⁵, and where one or more CH₂ groups in the said alkyl, alkoxy, alkenyl and alkynyl groups may in each case be replaced by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂;
- R⁵: is, identically or differently at each occurrence, selected from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁵ may be connected to each other to form a ring; where the said alkyl, alkoxy, alkenyl and alkynyl groups and the said aromatic and heteroaromatic ring systems may in each case be substituted by one or more radicals R⁶, and where one or more CH₂ groups in the said alkyl, alkoxy, alkenyl and alkynyl groups may in each case be replaced by -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO or SO₂;
- R⁶: is selected, identically or differently at each occurrence, from H, D, F, CN, alkyl groups having 1 to 20 C atoms, aromatic ring systems having 6 to 40 C atoms, and heteroaromatic ring systems having 5 to 40 aromatic ring atoms; where two or more radicals R⁶ may be connected to each other to form a ring; and where the said alkyl groups, aromatic ring systems and heteroaromatic ring systems may be substituted by F and CN;
- k: is on each occurrence, identically or differently, 0 or 1; where in the case of k=0, the group Ar^{L} is not present and the nitrogen atom and the spirobifluorene group are directly connected;
- m: is on each occurrence, identically or differently, 0 or 1, where in the case of m=0, the group E is not present and the groups Ar¹ are not connected;
characterized in that at least one of groups Z¹ is CR¹.

The circles drawn in the six-rings of formula (I) mean that the respective rings have aromaticity, resulting from alternation of double bonds and single bonds between the atoms forming the rings.

The following definitions apply to the chemical groups used as general definitions. They only apply insofar as no more specific definitions are given.

An aryl group in the sense of this invention contains 6 to 40 aromatic ring atoms, of which none is a heteroatom. An aryl group here is taken to mean either a simple aromatic ring, for example benzene, or a condensed aromatic polycycle, for example naphthalene, phenanthrene, or anthracene. A condensed aromatic polycycle in the sense of the present application consists of two or more simple aromatic rings condensed with one another.

A heteroaryl group in the sense of this invention contains 5 to 40 aromatic ring atoms, at least one of which is a heteroatom. The heteroatoms are preferably selected from N, O and S. A heteroaryl group here is taken to mean either a simple heteroaromatic ring, such as pyridine, pyrimidine or thiophene, or a condensed heteroaromatic polycycle, such as quinoline or carbazole. A condensed heteroaromatic polycycle in the sense of the present application consists of two or more simple heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benz-anthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, pheno-thiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aromatic ring system in the sense of this invention contains 6 to 40 C atoms in the ring system and does not comprise any heteroatoms as aromatic ring atoms. An aromatic ring system in the sense of this application therefore does not comprise any heteroaryl groups. An aromatic ring system in the sense of this invention is intended to be taken to mean a system which does not necessarily contain only aryl groups, but instead in which, in addition, a plurality of aryl groups may be connected by a non-aromatic unit such as one or more optionally substituted C, Si, N, O or S atoms. The non-aromatic unit in such case comprises preferably less than 10% of the atoms other than H, relative to the total number of atoms other than H of the whole aromatic ring system. Thus, for example, systems such as 9,9'-spirobifluorene, 9,9'-diarylfluorene, triarylamine, diaryl ether, and stilbene are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl groups are linked to one another via single bonds are also taken to be aromatic ring systems in the sense of this invention, such as, for example, systems such as biphenyl and terphenyl.

Preferably, an aromatic ring system is understood to be a chemical group, in which the aryl groups which constitute the chemical group are conjugated with each other. This means that the aryl groups are connected with each other via single bonds or via connecting units which have a free pi electron pair which can take part in the conjugation. The connecting units are preferably selected from nitrogen atoms, single C=C units, single C≡C units, multiple C=C units and/or C≡C units which are conjugated with each other, -O-, and -S-.

A heteroaromatic ring system in the sense of this invention contains 5 to 40 aromatic ring atoms, at least one of which is a heteroatom. The heteroatoms are preferably selected from N, O or S. A heteroaromatic ring system is defined as an aromatic ring system above, with the difference that it must obtain at least one heteroatom as one of the aromatic ring atoms. It thereby differs from an aromatic ring system according to the definition of the present application, which cannot comprise any heteroatom as aromatic ring atom.

An aromatic ring system having 6 to 40 aromatic ring atoms or a heteroaromatic ring system having 5 to 40 aromatic ring atoms is in particular a group which is derived from the above mentioned aryl or heteroaryl groups, or from biphenyl, terphenyl, quaterphenyl, fluorene, spirobifluorene, dihydrophenanthrene, dihydropyrene, tetrahydropyrene, indenofluorene, truxene, isotruxene, spirotruxene, spiroisotruxene, and indenocarbazole.

For the purposes of the present invention, a straight-chain alkyl group having 1 to 20 C atoms or a branched or cyclic alkyl group having 3 to 20 C atoms or an alkenyl or alkynyl group having 2 to 20 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl.

An alkoxy or thioalkyl group having 1 to 20 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyl-oxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptyl-thio, cycloheptylthio, n-octylthio, cyclooctylthio, 2-ethylhexylthio, trifluoro-methylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenyl-thio, ethynylthio, propynylthio, butynylthio, pentynylthio, hexynylthio, heptynylthio or octynylthio.

Preferably, in compounds of formula (I), group A is C.

Furthermore, preferably, Z¹ is selected from CR¹ and CR².

Furthermore, preferably, Z² is CR².

Furthermore, preferably, Z³ is CR³.

Furthermore, it is preferred that a maximum of three groups which are selected from groups Z¹, Z² and Z³ per aromatic ring of the compound of formula (I) is N. More preferably, in the compound of formula (I), a maximum of three groups selected from groups Z¹, Z² and Z³ is N.

Preferably, group Ar^{L} is selected from aromatic ring systems having 6 to 30 aromatic ring atoms, which may be substituted by one or more radicals R⁴. It is particularly preferred if Ar^{L} is selected from divalent groups derived from benzene, biphenyl, terphenyl, naphthyl, fluorenyl, indenofluorenyl, spirobifluorenyl, dibenzofuranyl, dibenzothiophenyl, and carbazolyl, which may each be substituted by one or more radicals R⁴. Most preferably, Ar^{L} is a divalent group derived from benzene, which may be substituted by one or more radicals R⁴.

Preferred groups Ar^{L} conform to the following formulae

| | | |
|---|---|---|
| 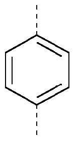 | 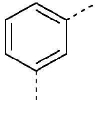 | 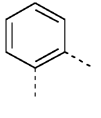 |
| Ar^{L}-1 | Ar^{L}-2 | Ar^{L}-3 |
| 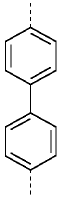 | 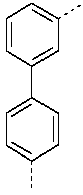 | 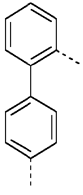 |
| Ar^{L}-4 | Ar^{L}-5 | Ar^{L}-6 |
| 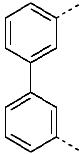 | 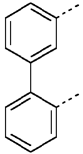 | 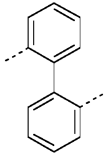 |
| Ar^{L}-7 | Ar^{L}-8 | Ar^{L}-9 |
| 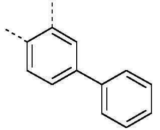 | 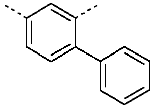 | 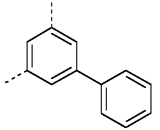 |
| Ar^{L}-10 | Ar^{L}-11 | Ar^{L}-12 |
| 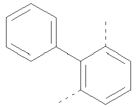 | 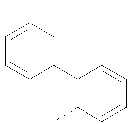 | 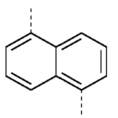 |
| Ar^{L}-13 | Ar^{L}-14 | Ar^{L}-15 |
| 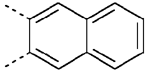 | 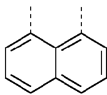 | 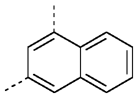 |
| Ar^{L}-16 | Ar^{L}-17 | Ar^{L}-18 |
| 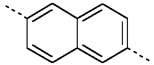 | 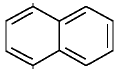 | 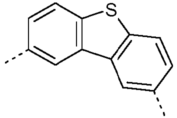 |
| Ar^{L}-19 | Ar^{L}-20 | Ar^{L}-21 |
| 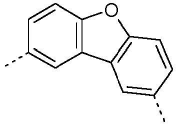 | 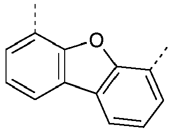 | 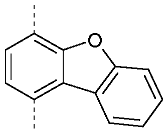 |
| Ar^{L}-22 | Ar^{L}-23 | Ar^{L}-24 |
| 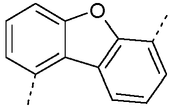 | 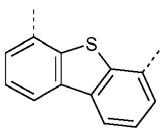 | 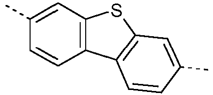 |
| Ar^{L}-25 | Ar^{L}-26 | Ar^{L}-27 |
| 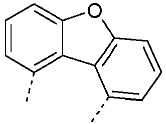 | 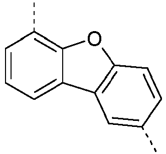 | 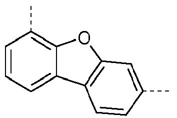 |
| Ar^{L}-28 | Ar^{L}-29 | Ar^{L}-30 |
| 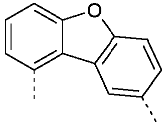 | 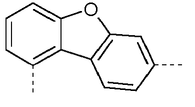 | 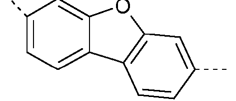 |
| Ar^{L}-31 | Ar^{L}-32 | Ar^{L}-33 |
| 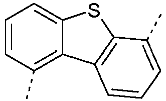 | 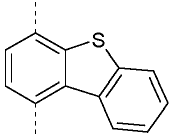 | 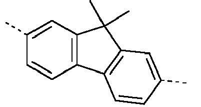 |
| Ar^{L}-34 | Ar^{L}-35 | Ar^{L}-36 |
| 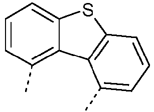 | 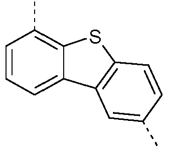 | 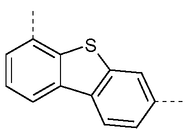 |
| Ar^{L}-37 | Ar^{L}-38 | Ar^{L}-39 |
| 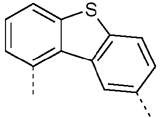 | 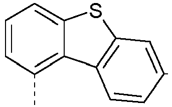 | 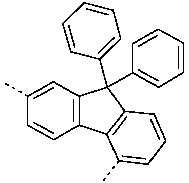 |
| Ar^{L}-40 | Ar^{L}-41 | Ar^{L}-42 |
| 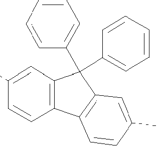 | 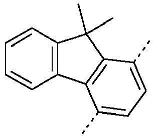 | 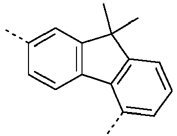 |
| Ar^{L}-43 | Ar^{L}-44 | Ar^{L}-45 |
| 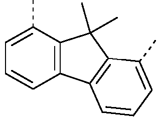 | 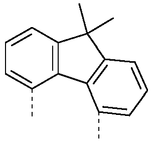 | 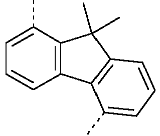 |
| Ar^{L}-46 | Ar^{L}-47 | Ar^{L}-48 |
| 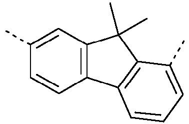 | 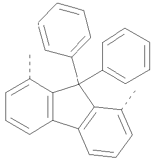 | 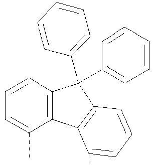 |
| Ar^{L}-49 | Ar^{L}-50 | Ar^{L}-51 |
| 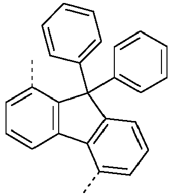 | 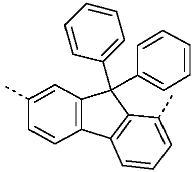 | 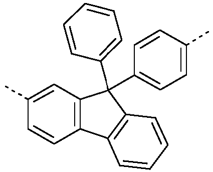 |
| Ar^{L}-52 | Ar^{L}-53 | Ar^{L}-54 |
| 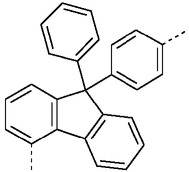 | 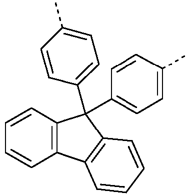 | 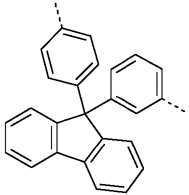 |
| Ar^{L}-55 | Ar^{L}-56 | Ar^{L}-57 |
| 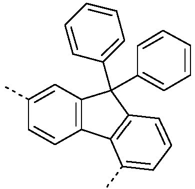 | 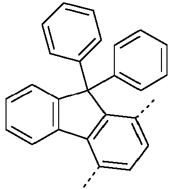 | 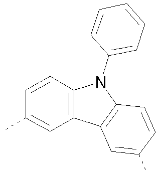 |
| Ar^{L}-58 | Ar^{L}-59 | Ar^{L}-60 |
| 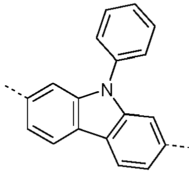 | 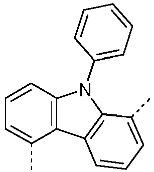 | 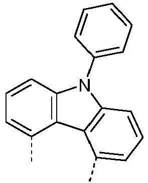 |
| Ar^{L}-61 | Ar^{L}-62 | Ar^{L}-63 |
| 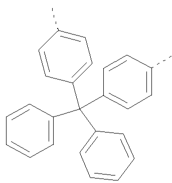 | 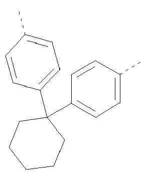 | 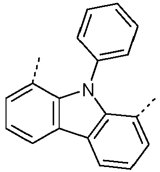 |
| Ar^{L}-64 | Ar^{L}-65 | Ar^{L}-66 |
| 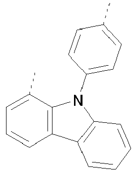 | 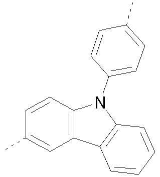 | 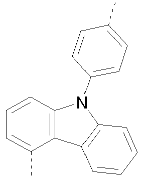 |
| Ar^{L}-67 | Ar^{L}-68 | Ar^{L}-69 |
| 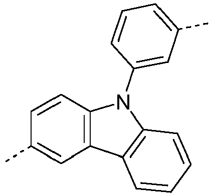 | 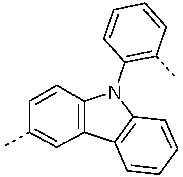 | 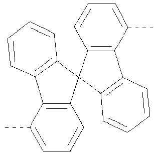 |
| Ar^{L}-70 | Ar^{L}-71 | Ar^{L}-72 |
| 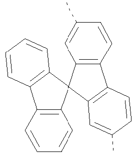 | 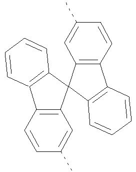 | 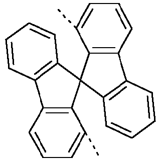 |
| Ar^{L}-73 | Ar^{L}-74 | Ar^{L}-75 |

where the dotted lines represent the bonds of the divalent group to the rest of the formula (I).

Particularly preferred among the groups above are the groups according to one of formulae Ar^{L}-1, Ar^{L}-2, Ar^{L}-3, Ar^{L}-4, Ar^{L}-15, Ar^{L}-20, Ar^{L}-25, and Ar^{L}-36.

It is preferred that index k is 0, meaning that the group Ar^{L} is not present, so that the spirobifluorene and the nitrogen atom of the amine are directly connected with each other.

Preferably, groups Ar¹ are, identically or differently, selected from radicals derived from the following groups, which are each optionally substituted by one or more radicals R⁴, or from combinations of 2 or 3 radicals derived from the following groups, which are each optionally substituted by one or more radicals R⁴: phenyl, biphenyl, terphenyl, quaterphenyl, naphthyl, fluorenyl, especially 9,9'-dimethylfluorenyl and 9,9'-diphenylfluorenyl, benzofluorenyl, spirobifluorenyl, indenofluorenyl, dibenzofuranyl, dibenzothiophenyl, carbazolyl, benzofuranyl, benzothiophenyl, indolyl, quinolinyl, pyridyl, pyrimidyl, pyrazinyl, pyridazinyl and triazinyl.

Particularly preferred groups Ar¹ are, identically or differently, selected from phenyl, biphenyl, terphenyl, quaterphenyl, naphthyl, fluorenyl, especially 9,9'-dimethylfluorenyl and 9,9'-diphenylfluorenyl, benzofluorenyl, spirobifluorenyl, indenofluorenyl, dibenzofuranyl, dibenzothiophenyl, carbazolyl, benzofuranyl, benzothiophenyl, benzofused dibenzofuranyl, benzofused dibenzothiophenyl, naphthyl-substituted phenyl, fluorenyl-substituted phenyl, spirobifluorenyl-substituted phenyl, dibenzofuranyl-substituted phenyl, dibenzothiophenyl-substituted phenyl, carbazolyl-substituted phenyl, pyridyl-substituted phenyl, pyrimidyl-substituted phenyl, and triazinyl-substituted phenyl, each of which may optionally be substituted by one or more radicals R⁴.

Preferably, groups Ar¹ are, at each occurrence, selected differently.

Preferred groups Ar¹ are, identically or differently, selected from groups of the following formulae

| | | |
|---|---|---|
| 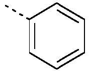 | 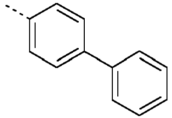 | 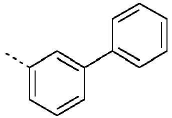 |
| Ar-1 | Ar-2 | Ar-3 |
| 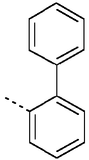 | 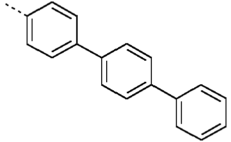 | 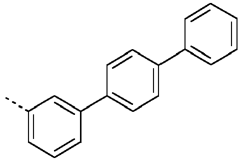 |
| Ar-4 | Ar-5 | Ar-6 |
| 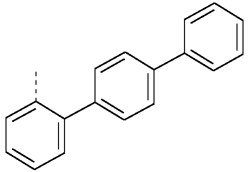 | 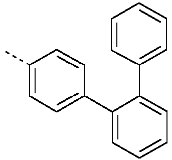 | 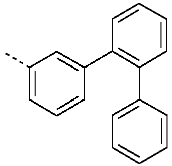 |
| Ar-7 | Ar-8 | Ar-9 |
| 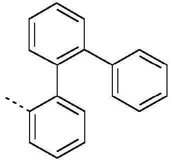 | 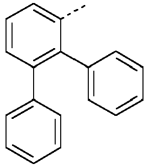 | 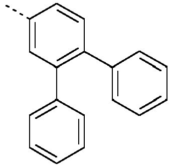 |
| Ar-10 | Ar-11 | Ar-12 |
| 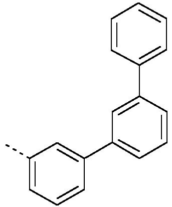 | 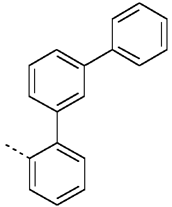 | 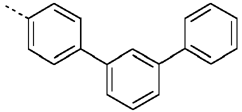 |
| Ar-13 | Ar-14 | Ar-15 |
| 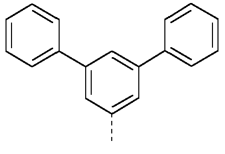 | 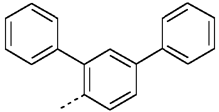 | 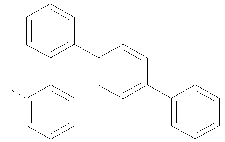 |
| Ar-16 | Ar-17 | Ar-18 |
| 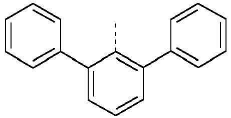 | | |
| Ar-19 | | |
| 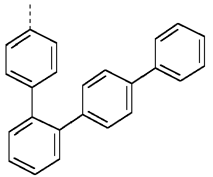 | 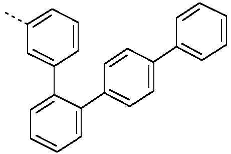 | 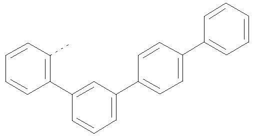 |
| Ar-20 | Ar-21 | Ar-22 |
| 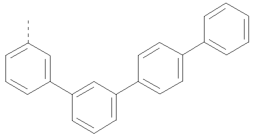 | 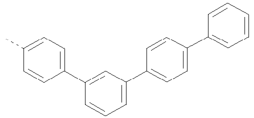 | 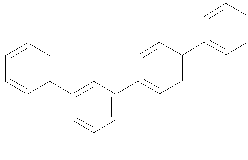 |
| Ar-23 | Ar-24 | Ar-25 |
| 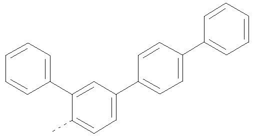 | 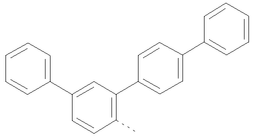 | 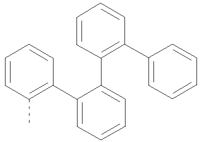 |
| Ar-26 | Ar-27 | Ar-28 |
| 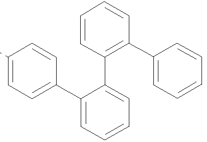 | 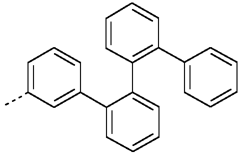 | 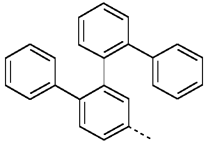 |
| Ar-29 | Ar-30 | Ar-31 |
| 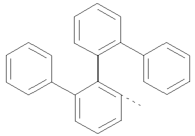 | 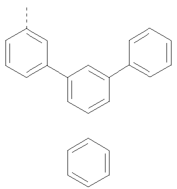 | 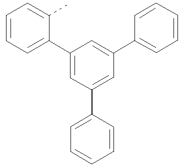 |
| Ar-32 | Ar-33 | Ar-34 |
| 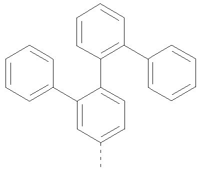 | 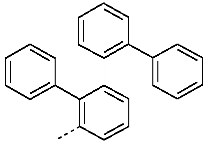 | 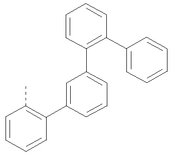 |
| Ar-35 | Ar-36 | Ar-37 |
| 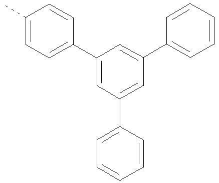 | 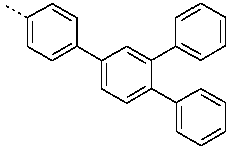 | 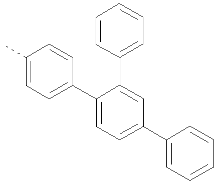 |
| Ar-38 | Ar-39 | Ar-40 |
| 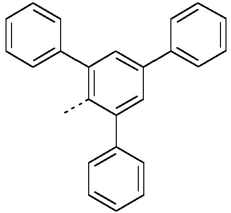 | 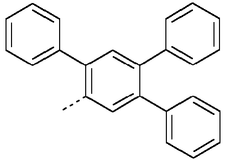 | 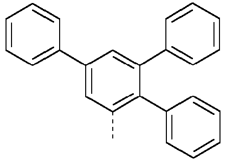 |
| Ar-41 | Ar-42 | Ar-43 |
| 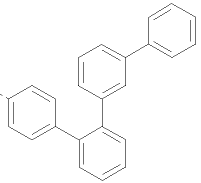 | 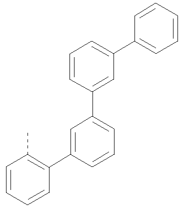 | 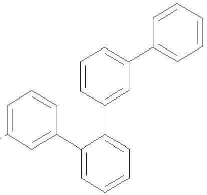 |
| Ar-44 | Ar-45 | Ar-46 |
| 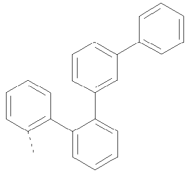 | | |
| Ar-47 | | |
| 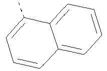 | 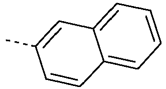 | 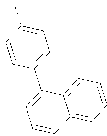 |
| Ar-48 | Ar-49 | Ar-50 |
| 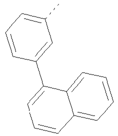 | 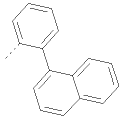 | 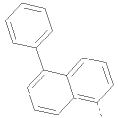 |
| Ar-51 | Ar-52 | Ar-53 |
| 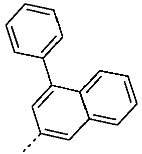 | 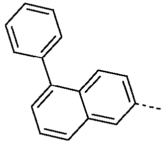 | 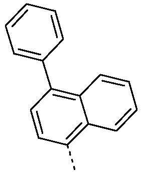 |
| Ar-54 | Ar-55 | Ar-56 |
| 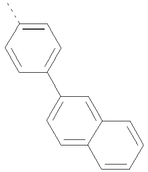 | 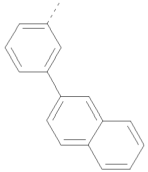 | 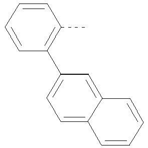 |
| Ar-57 | Ar-58 | Ar-59 |
| 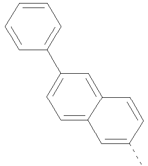 | 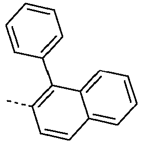 | 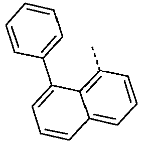 |
| Ar-60 | Ar-61 | Ar-62 |
| 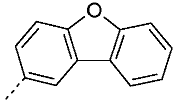 | 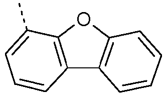 | 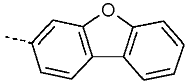 |
| Ar-63 | Ar-64 | Ar-65 |
| 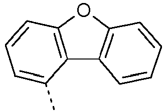 | 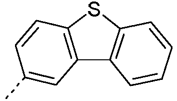 | 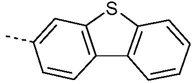 |
| Ar-66 | Ar-67 | Ar-68 |
| 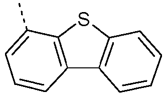 | 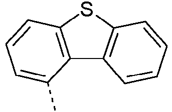 | 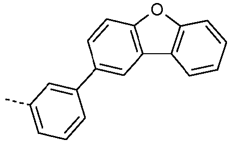 |
| Ar-69 | Ar-70 | Ar-71 |
| 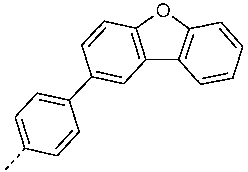 | 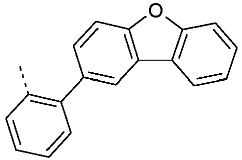 | 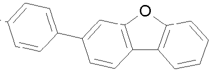 |
| Ar-72 | Ar-73 | Ar-74 |
| 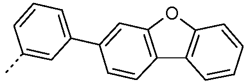 | 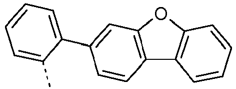 | 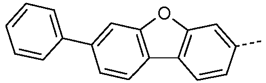 |
| Ar-75 | Ar-76 | Ar-77 |
| 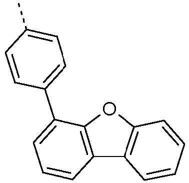 | 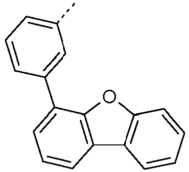 | 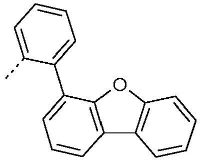 |
| Ar-78 | Ar-79 | Ar-80 |
| 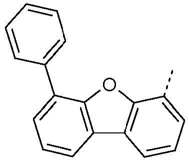 | 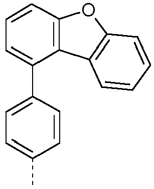 | 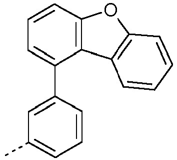 |
| Ar-81 | Ar-82 | Ar-83 |
| 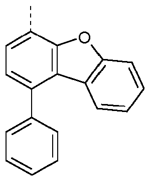 | 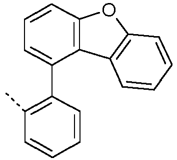 | 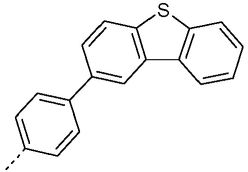 |
| Ar-84 | Ar-85 | Ar-86 |
| 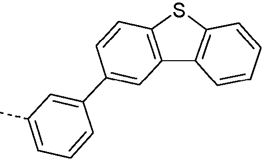 | 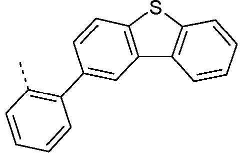 | 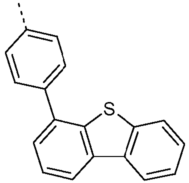 |
| Ar-87 | Ar-88 | Ar-89 |
| 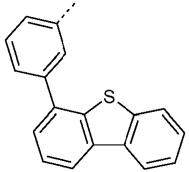 | 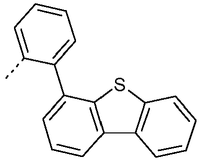 | 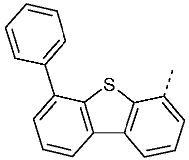 |
| Ar-90 | Ar-91 | Ar-92 |
| 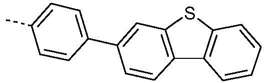 | 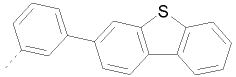 | 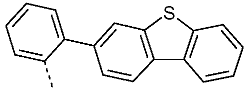 |
| Ar-93 | Ar-94 | Ar-95 |
| 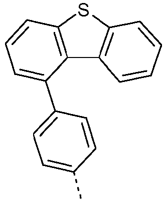 | 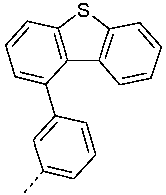 | 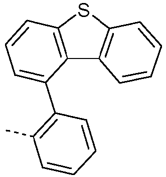 |
| Ar-96 | Ar-97 | Ar-98 |
| 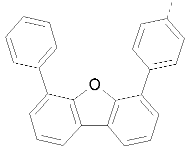 | 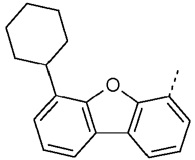 | 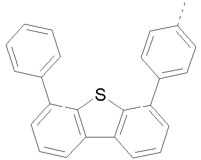 |
| Ar-99 | Ar-100 | Ar-101 |
| 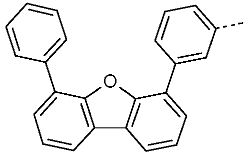 | 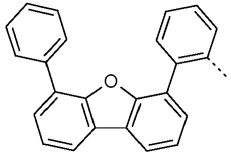 | 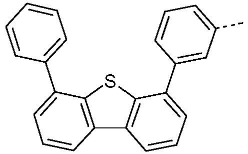 |
| Ar-102 | Ar-103 | Ar-104 |
| 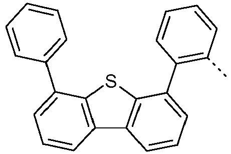 | | |
| Ar-105 | | |
| 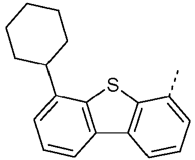 | 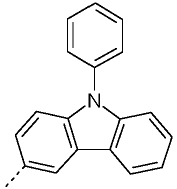 | 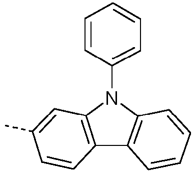 |
| Ar-106 | Ar-107 | Ar-108 |
| 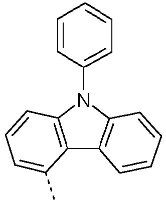 | 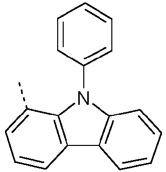 | 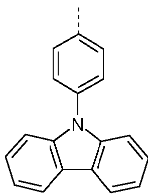 |
| Ar-109 | Ar-110 | Ar-111 |
| 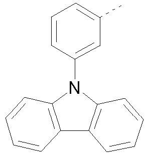 | 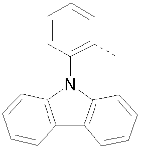 | |
| Ar-112 | Ar-113 | |
| 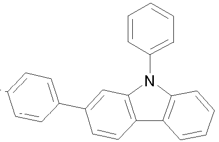 | 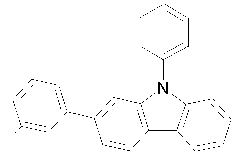 | 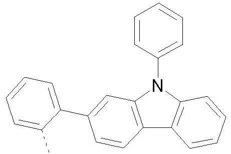 |
| Ar-114 | Ar-115 | Ar-116 |
| 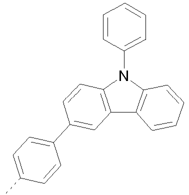 | 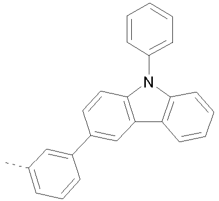 | 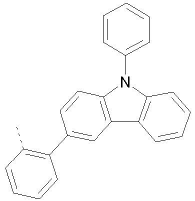 |
| Ar-117 | Ar-118 | Ar-119 |
| 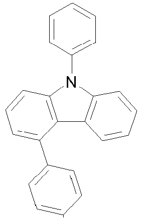 | 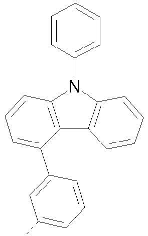 | 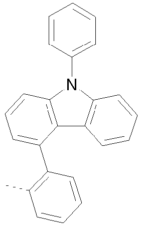 |
| Ar-120 | Ar-121 | Ar-122 |
| 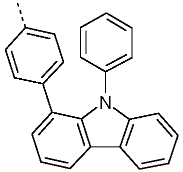 | 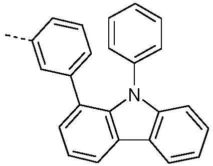 | 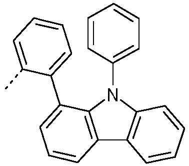 |
| Ar-123 | Ar-124 | Ar-125 |
| 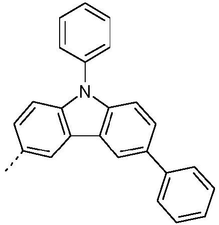 | 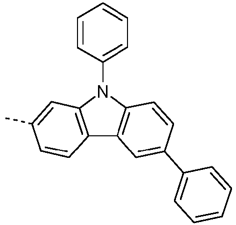 | 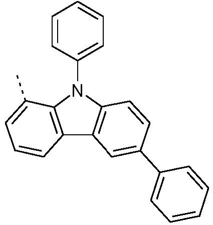 |
| Ar-126 | Ar-127 | Ar-128 |
| 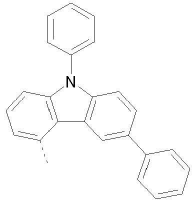 | 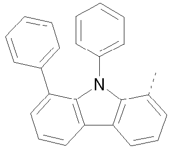 | 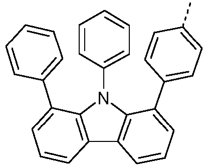 |
| Ar-129 | Ar-130 | Ar-131 |
| 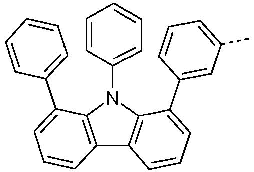 | 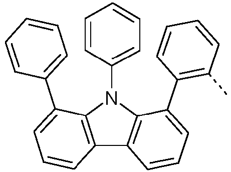 | |
| Ar-132 | Ar-133 | |
| 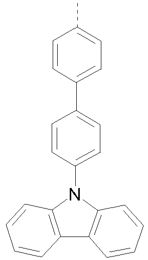 | 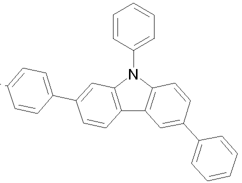 | 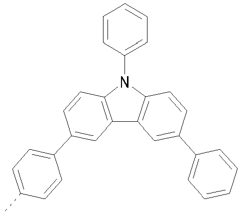 |
| Ar-134 | Ar-135 | Ar-136 |
| 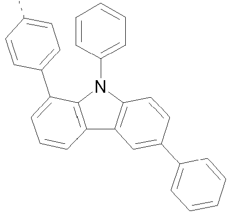 | 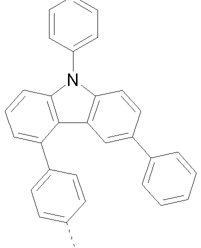 | 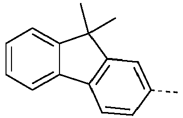 |
| Ar-137 | Ar-138 | Ar-139 |
| 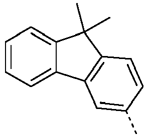 | 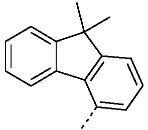 | 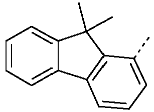 |
| Ar-140 | Ar-141 | Ar-142 |
| 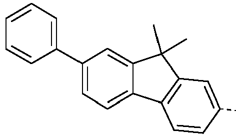 | 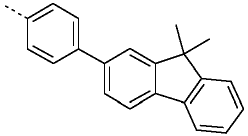 | 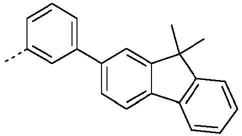 |
| Ar-143 | Ar-144 | Ar-145 |
| 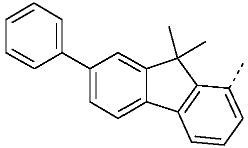 | 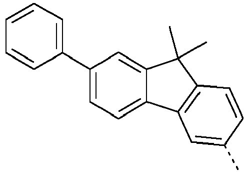 | 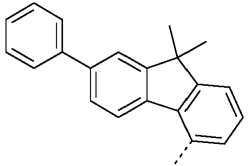 |
| Ar-146 | Ar-147 | Ar-148 |
| 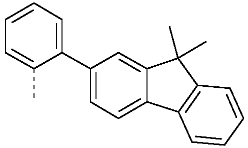 | 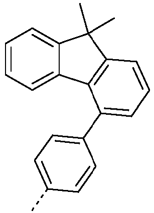 | 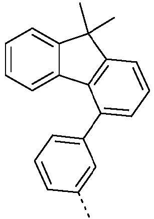 |
| Ar-149 | Ar-150 | Ar-151 |
| 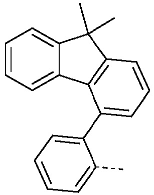 | 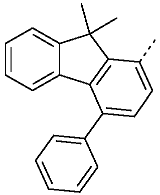 | 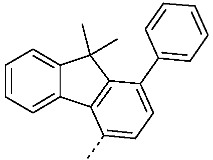 |
| Ar-152 | Ar-153 | Ar-154 |
| 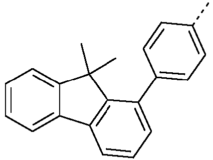 | 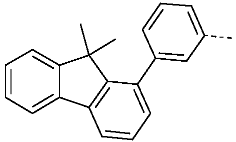 | 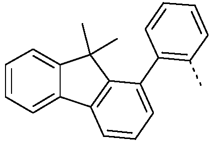 |
| Ar-155 | Ar-156 | Ar-157 |
| 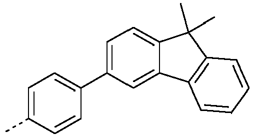 | 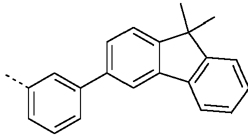 | 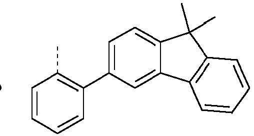 |
| Ar-158 | Ar-159 | Ar-160 |
| 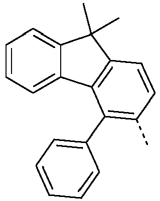 | 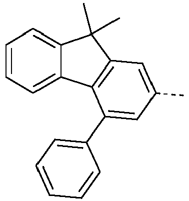 | 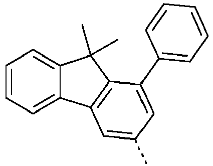 |
| Ar-161 | Ar-162 | Ar-163 |
| 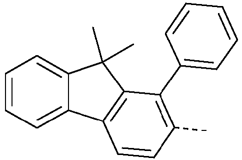 | 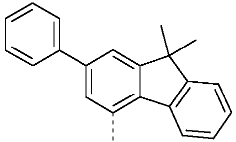 | 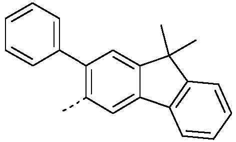 |
| Ar-164 | Ar-165 | Ar-166 |
| 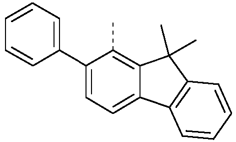 | 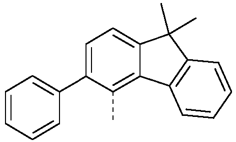 | 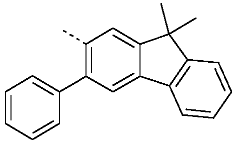 |
| Ar-167 | Ar-168 | Ar-169 |
| 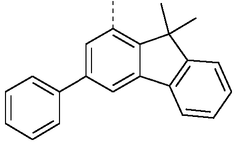 | 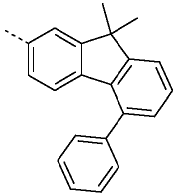 | 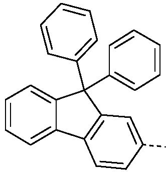 |
| Ar-170 | Ar-171 | Ar-172 |
| 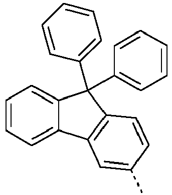 | 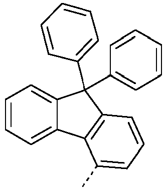 | 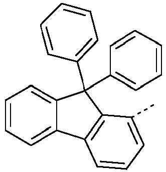 |
| Ar-173 | Ar-174 | Ar-175 |
| 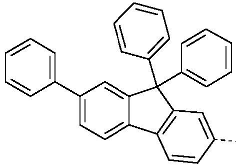 | 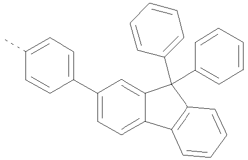 | 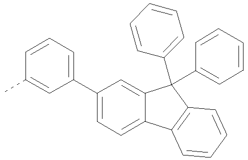 |
| Ar-176 | Ar-177 | Ar-178 |
| 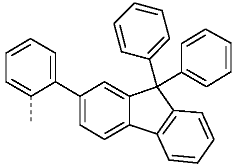 | 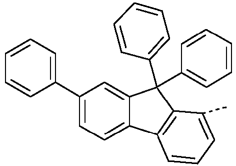 | 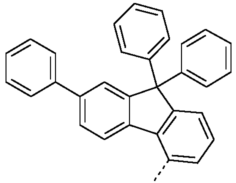 |
| Ar-179 | Ar-180 | Ar-181 |
| 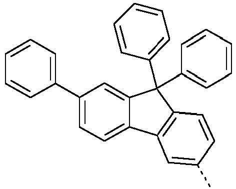 | 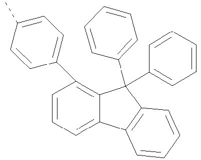 | 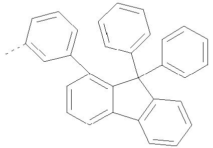 |
| Ar-182 | Ar-183 | Ar-184 |
| 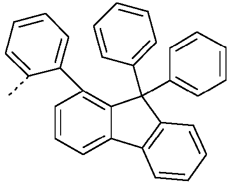 | 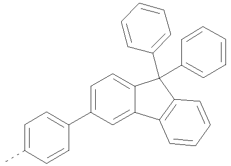 | 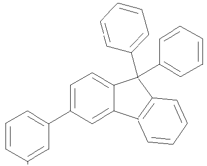 |
| Ar-185 | Ar-186 | Ar-187 |
| 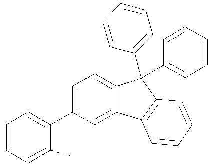 | 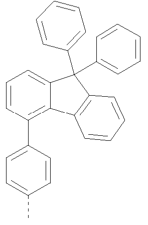 | 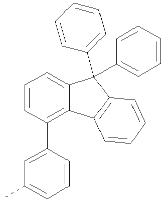 |
| Ar-188 | Ar-189 | Ar-190 |
| 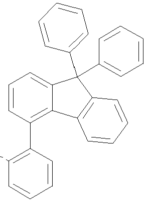 | | |
| Ar-191 | | |
| 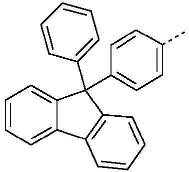 | 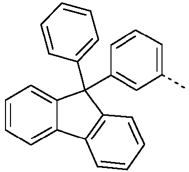 | 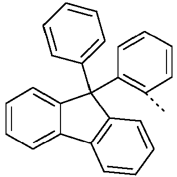 |
| Ar-192 | Ar-193 | Ar-194 |
| 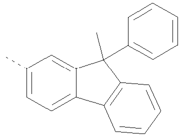 | 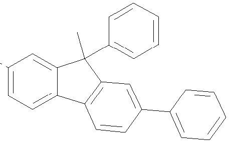 | 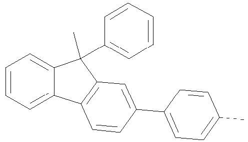 |
| Ar-195 | Ar-196 | Ar-197 |
| 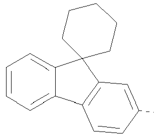 | 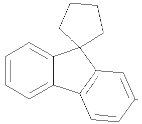 | 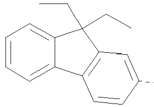 |
| Ar-198 | Ar-199 | Ar-200 |
| 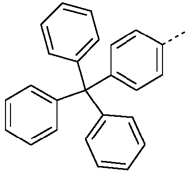 | 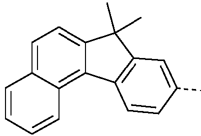 | 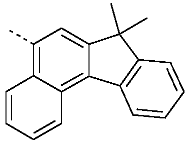 |
| Ar-201 | Ar-202 | Ar-203 |
| 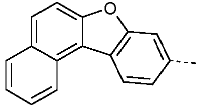 | 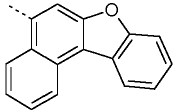 | 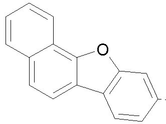 |
| Ar-204 | Ar-205 | Ar-206 |
| 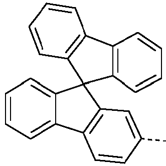 | 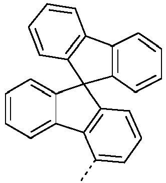 | 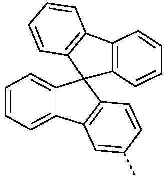 |
| Ar-207 | Ar-208 | Ar-209 |
| 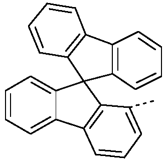 | 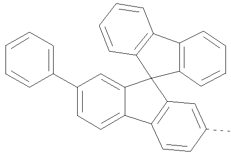 | 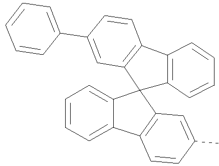 |
| Ar-210 | Ar-211 | Ar-212 |
| 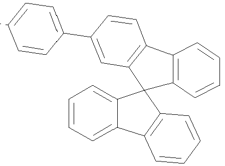 | 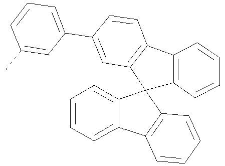 | 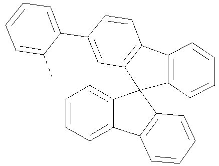 |
| Ar-213 | Ar-214 | Ar-215 |
| 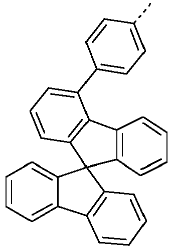 | 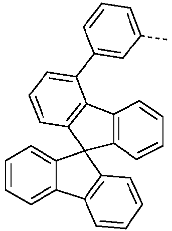 | 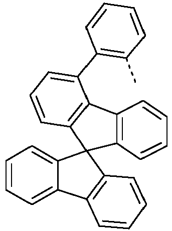 |
| Ar-216 | Ar-217 | Ar-218 |
| 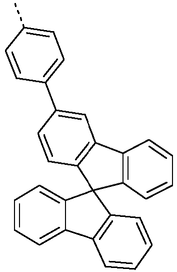 | 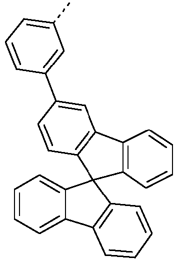 | 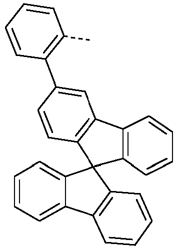 |
| Ar-219 | Ar-220 | Ar-221 |
| 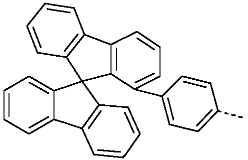 | 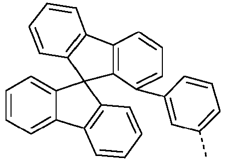 | 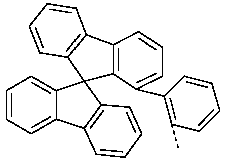 |
| Ar-222 | Ar-223 | Ar-224 |
| 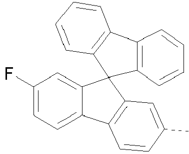 | 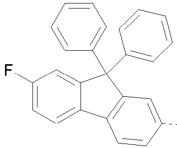 | 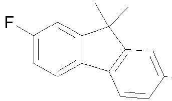 |
| Ar-225 | Ar-226 | Ar-227 |
| 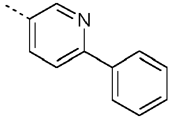 | 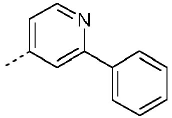 | 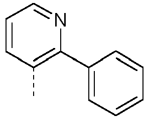 |
| Ar-228 | Ar-229 | Ar-230 |
| 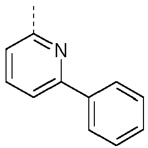 | 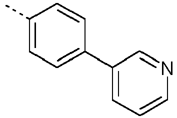 | 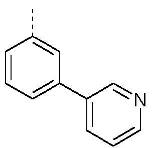 |
| Ar-231 | Ar-232 | Ar-233 |
| 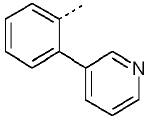 | 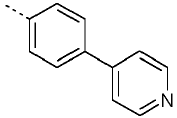 | 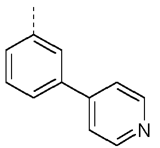 |
| Ar-234 | Ar-235 | Ar-236 |
| 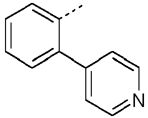 | 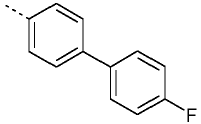 | 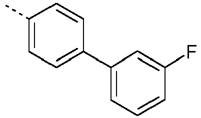 |
| Ar-237 | Ar-238 | Ar-239 |
| 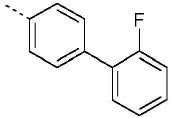 | 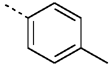 | 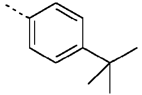 |
| Ar-240 | Ar-241 | Ar-242 |
| 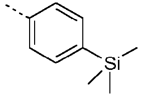 | 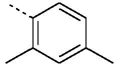 | 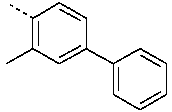 |
| Ar-243 | Ar-244 | Ar-245 |
| 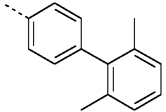 | 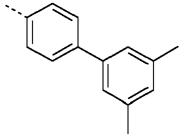 | 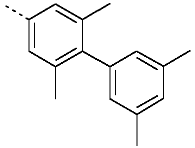 |
| Ar-246 | Ar-247 | Ar-248 |
| 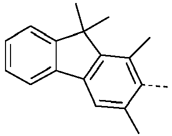 | 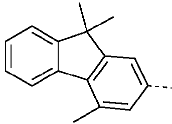 | 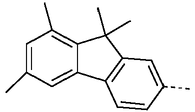 |
| Ar-250 | Ar-251 | Ar-252 |

where the groups may be substituted at the free positions with groups R⁴, but are preferably unsubstituted in these positions, and where the dotted line symbolizes the bonding position to the nitrogen atom.

Particularly preferred groups Ar¹ are groups which conform to one of above formulae Ar-1, Ar-2, Ar-4, Ar-5, Ar-74, Ar-78, Ar-82, Ar-117, Ar-134, Ar-139, Ar-150, and Ar-172.

According to a preferred embodiment, index m is 0, meaning that groups Ar¹ are not connected by a group E.

According to an alternative embodiment, which may be preferred under certain conditions, index m is 1, meaning that groups Ar¹ are connected by a group E.

In the case that groups Ar¹ are connected by a group E, it is preferred that groups Ar¹ are selected, identically or differently, from phenyl and fluorenyl, each of which may be substituted by one or more groups R⁴. Furthermore, in such case, it is preferred that the group E which connects the groups Ar¹ is located on the respective group Ar¹, preferably on the respective group Ar¹ which is phenyl or fluorenyl, in ortho-position to the bond of the group Ar¹ to the amine nitrogen atom. Furthermore, preferably, in such case a six-ring with the amine nitrogen atom is formed of the groups Ar¹ and E if E is selected from C(R⁴)₂, NR⁴, O and S; and a five-ring is formed if E is a single bond.

In the case that groups Ar¹ are connected by a group E, particularly preferred embodiments of the moieties 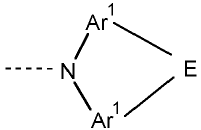 are selected from the following formulae

| | | |
|---|---|---|
| 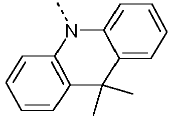 | 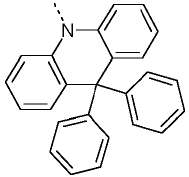 | 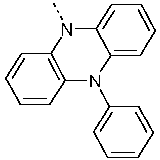 |
| N-1 | N-2 | N-3 |
| 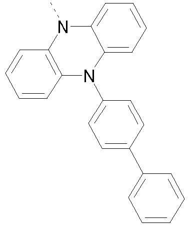 | 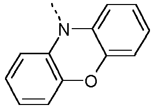 | 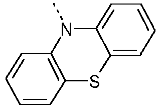 |
| N-4 | N-5 | N-6 |
| 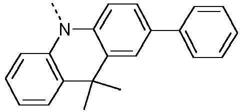 | 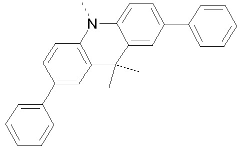 | 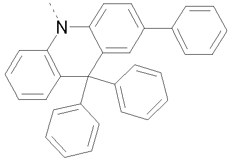 |
| N-7 | N-8 | N-9 |
| 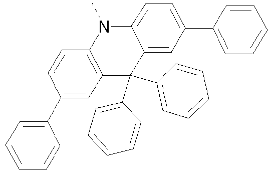 | 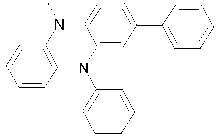 | 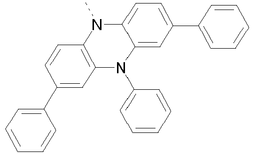 |
| N-10 | N-11 | N-12 |
| 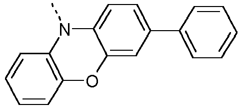 | 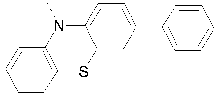 | 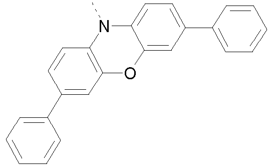 |
| N-13 | N-14 | N-15 |
| 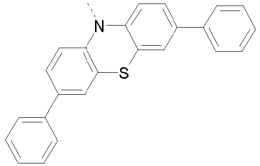 | 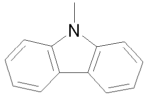 | 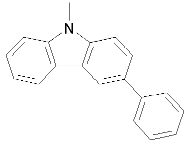 |
| N-16 | N-17 | N-18 |
| 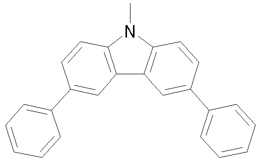 | 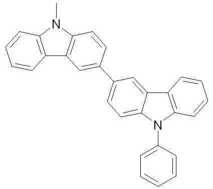 | 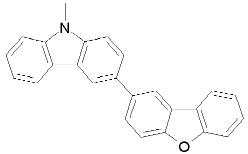 |
| | N-20 | N-21 |
| 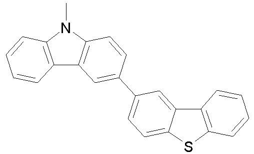 | 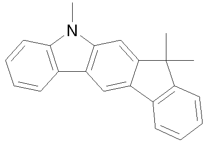 | 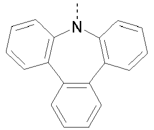 |
| N-22 | N-23 | N-24 |
| 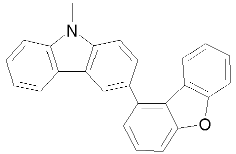 | 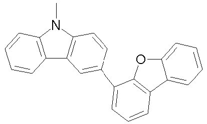 | 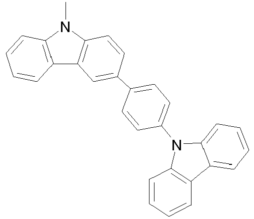 |
| N-25 | N-26 | N-27 |
| 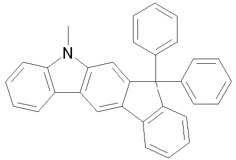 | 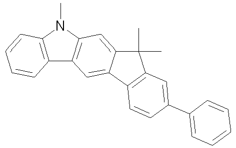 | 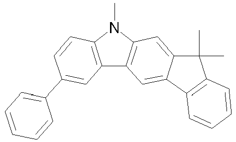 |
| N-28 | N-29 | N-30 |
| 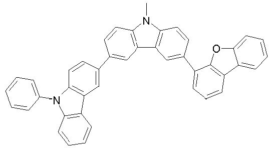 | 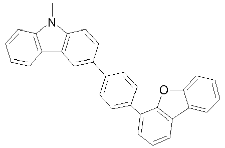 | |
| N-31 | N-32 | |

where the groups may be substituted at the free positions with groups R⁴, but are preferably unsubstituted in these positions, and where the dotted line symbolizes the bonding position to the nitrogen atom.

For the case m=0, particularly preferable moieties 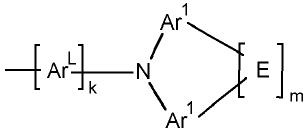 in formula (I) conform to the following formulae

| | |
|---|---|
| 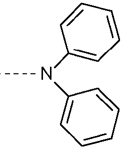 | 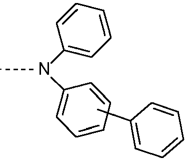 |
| A-1 | A-2 |
| 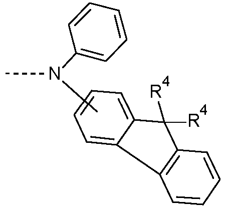 | 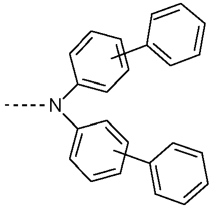 |
| A-3 | A-4 |
| 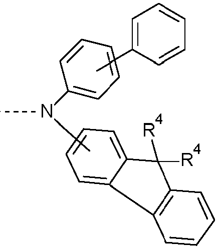 | 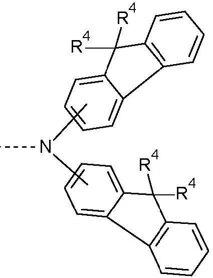 |
| A-5 | A-6 |
| 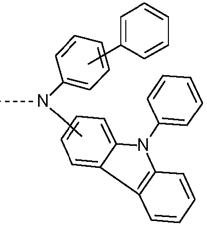 | 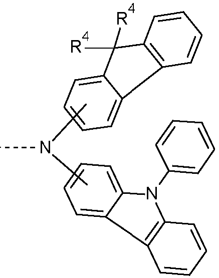 |
| A-7 | A-8 |
| 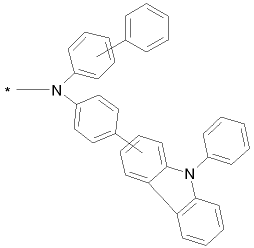 | 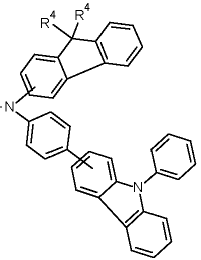 |
| A-9 | A-10 |
| 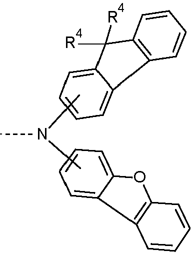 | 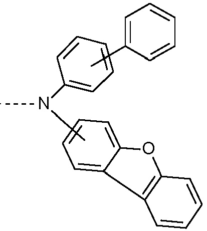 |
| A-11 | A-12 |
| 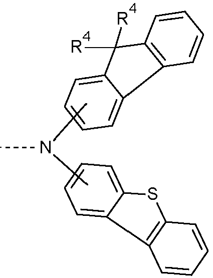 | 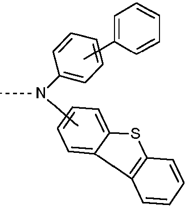 |
| A-13 | A-14 |
| 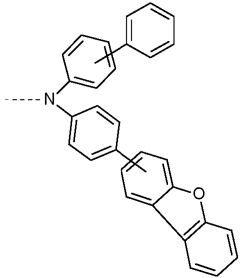 | 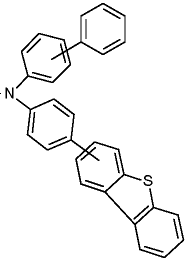 |
| A-15 | A-16 |
| 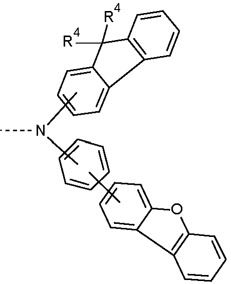 | 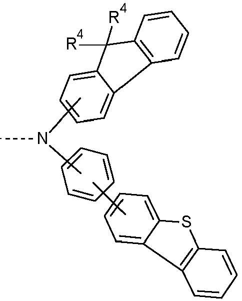 |
| A-17 | A-18 |
| 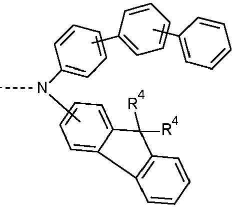 | 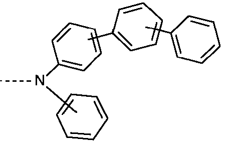 |
| A-19 | A-20 |
| 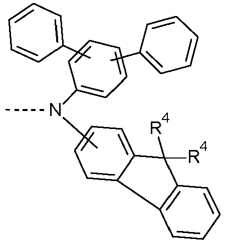 | 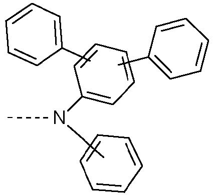 |
| A-21 | A-22 |
| 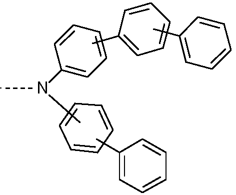 | 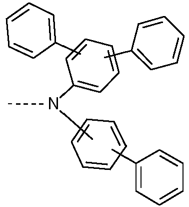 |
| A-23 | A-24 |
| 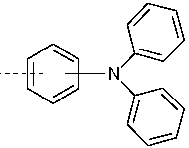 | 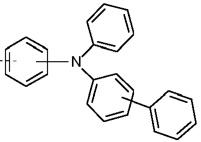 |
| A-25 | A-26 |
| 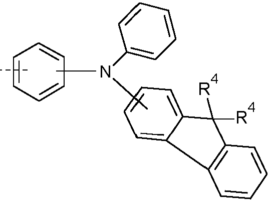 | 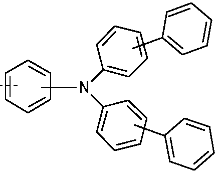 |
| A-27 | A-28 |
| 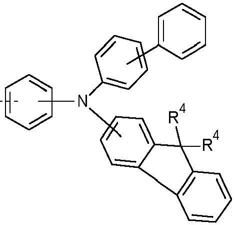 | 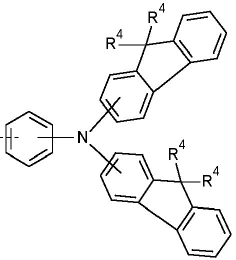 |
| A-29 | A-30 |
| 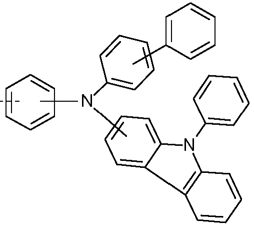 | 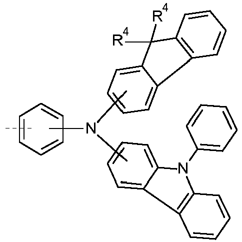 |
| A-31 | A-32 |
| 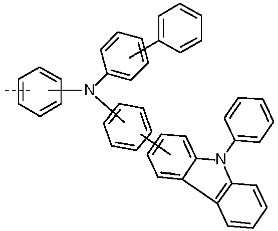 | 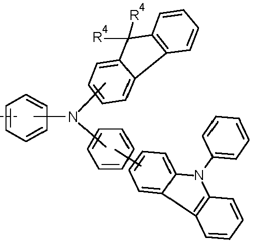 |
| A-33 | A-34 |
| 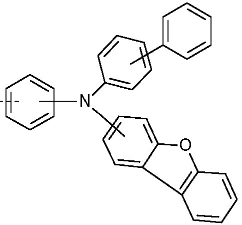 | 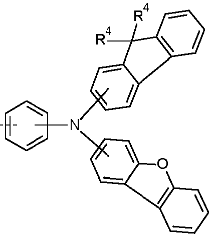 |
| A-35 | A-36 |
| 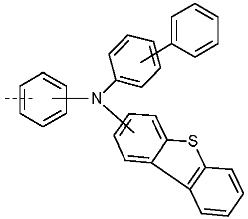 | 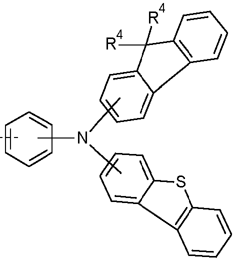 |
| A-37 | A-38 |
| 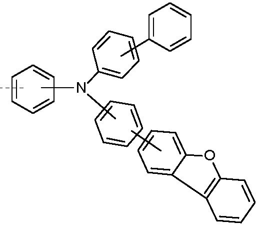 | 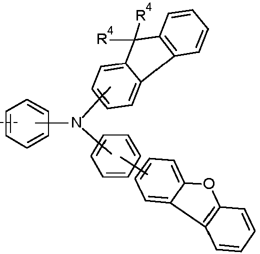 |
| A-39 | A-40 |
| 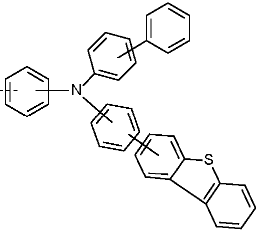 | 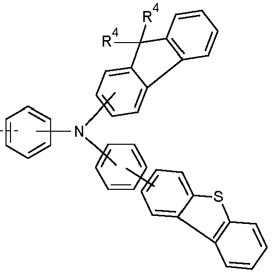 |
| A-41 | A-42 |
| 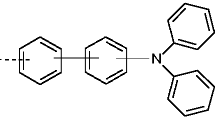 | 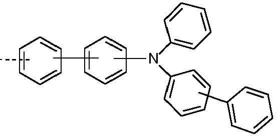 |
| A-43 | A-44 |
| 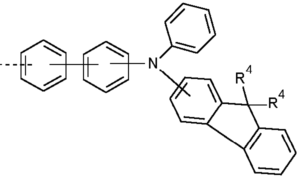 | 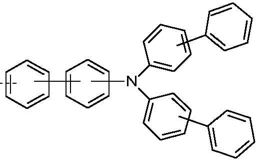 |
| A-45 | A-46 |
| 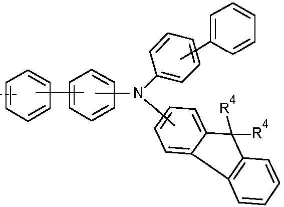 | 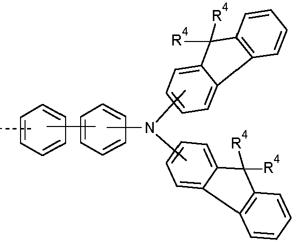 |
| A-47 | A-48 |

where the groups may be substituted at the free positions with groups R⁴, but are preferably unsubstituted in these positions, and where the dotted line symbolizes the bonding position to the spirobifluorene moiety of formula (I).

Groups R¹ are preferably selected, identically or differently, from 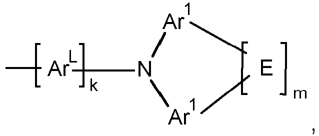 aromatic ring systems having 6 to 30 aromatic ring atoms, and heteroaromatic ring systems having 5 to 30 aromatic ring atoms, where the said aromatic and heteroaromatic ring systems may in each case be substituted by one or more radicals R⁵; particularly preferably selected, identically or differently, from 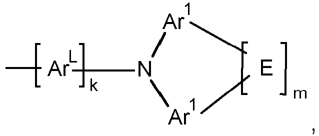 phenyl, biphenyl, terphenyl, quaterphenyl, naphthyl, fluorenyl, especially 9,9'-dimethylfluorenyl and 9,9'-diphenylfluorenyl, benzofluorenyl, spirobifluorenyl, indenofluorenyl, dibenzofuranyl, dibenzothiophenyl, carbazolyl, benzofuranyl, benzothiophenyl, benzofused dibenzofuranyl, benzofused dibenzothiophenyl, naphthyl-substituted phenyl, fluorenyl-substituted phenyl, spirobifluorenyl-substituted phenyl, dibenzofuranyl-substituted phenyl, dibenzothiophenyl-substituted phenyl, carbazolyl-substituted phenyl, pyridyl-substituted phenyl, pyrimidyl-substituted phenyl, and triazinyl-substituted phenyl, each of which may optionally be substituted by one or more radicals R⁵.

For embodiments 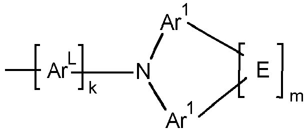 as groups R¹, the same preferred embodiments regarding groups Ar^{L}, Ar¹, E, and indices k and m apply, as mentioned above in the context of groups 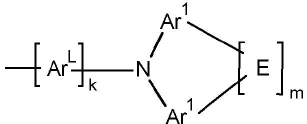 of formula (I).

Particularly preferred groups R¹ are groups which conform to the following groups

| | | | |
|---|---|---|---|
| 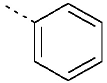 | 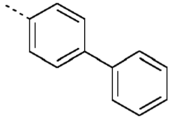 | 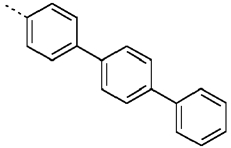 | |
| R-1 | R-2 | R-3 | |
| 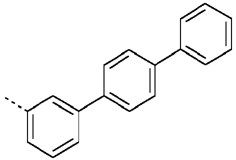 | 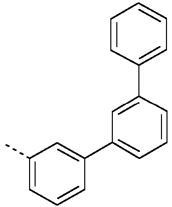 | 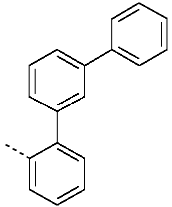 | |
| R-4 | R-5 | R-6 | |
| 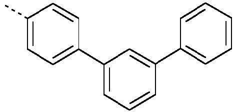 | 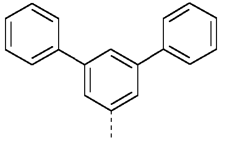 | 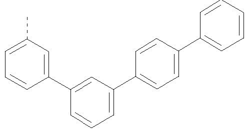 | |
| R-7 | R-8 | R-9 | |
| 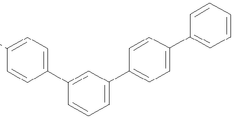 | 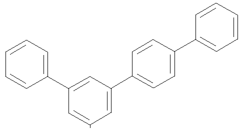 | 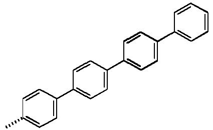 | |
| R-10 | R-11 | R-12 | |
| 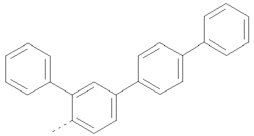 | 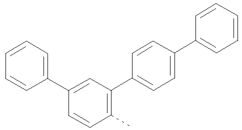 | 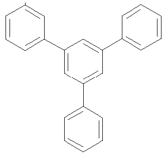 | |
| R-13 | R-14 | R-15 | |
| 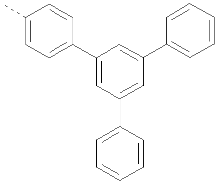 | 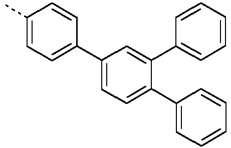 | 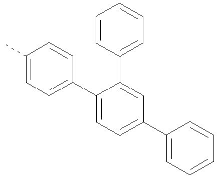 | |
| R-16 | R-17 | R-18 | |
| 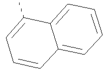 | 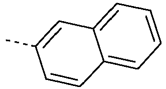 | 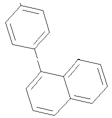 | |
| R-19 | R-20 | R-21 | |
| 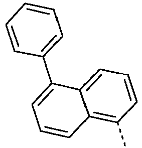 | 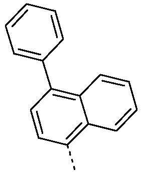 | 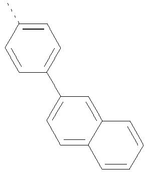 | |
| R-22 | R-23 | R-24 | |
| 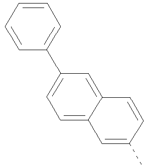 | 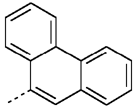 | 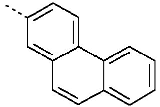 | |
| R-25 | R-26 | R-27 | |
| 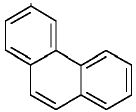 | 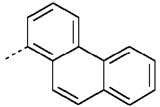 | 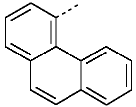 | |
| R-28 | R-29 | R-30 | |
| 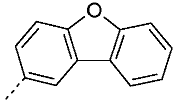 | 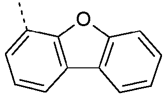 | 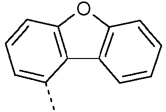 | |
| R-31 | R-32 | R-33 | |
| 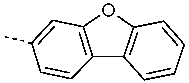 | 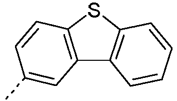 | 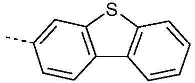 | |
| R-34 | R-35 | R-36 | |
| 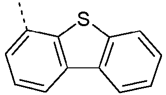 | 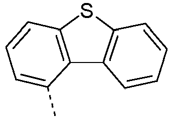 | 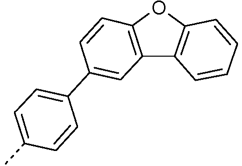 | |
| R-37 | R-38 | R-39 | |
| 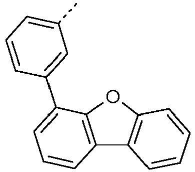 | 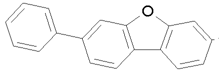 | 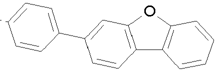 | |
| R-40 | R-41 | R-42 | |
| 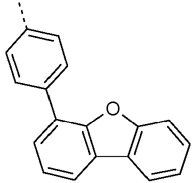 | 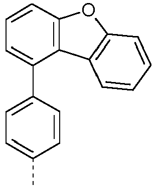 | 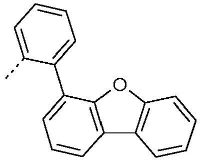 | |
| R-43 | R-44 | R-45 | |
| 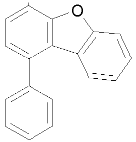 | 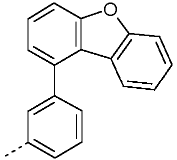 | 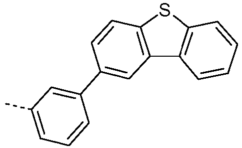 | |
| R-46 | R-47 | R-48 | |
| 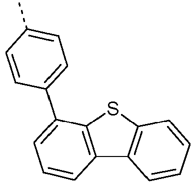 | 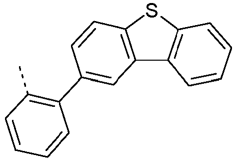 | 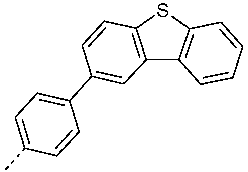 | |
| R-49 | R-50 | R-51 | |
| 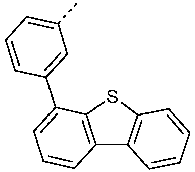 | 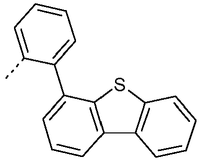 | 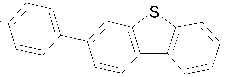 | |
| R-52 | R-53 | R-54 | |
| 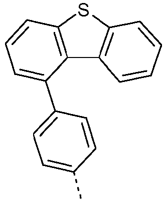 | 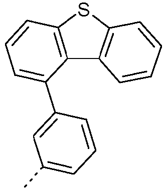 | 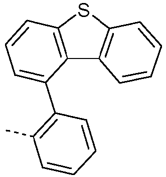 | |
| R-55 | R-56 | R-57 | |
| 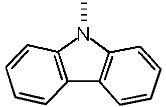 | 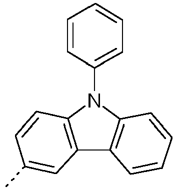 | 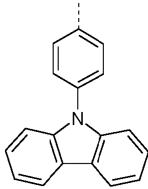 | |
| R-58 | R-59 | R-60 | |
| 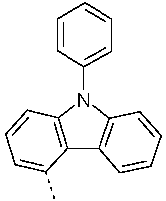 | 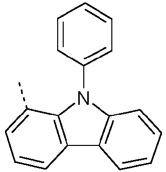 | 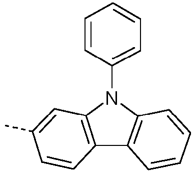 | |
| R-61 | R-62 | R-63 | |
| 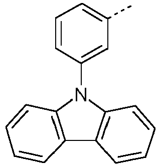 | 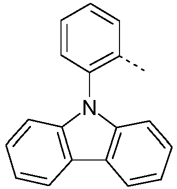 | 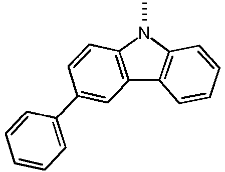 | |
| R-64 | R-65 | R-66 | |
| 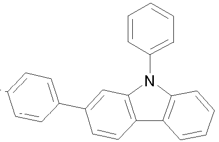 | 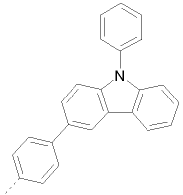 | 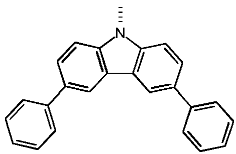 | |
| R-67 | R-68 | R-69 | |
| 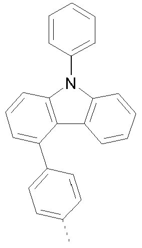 | 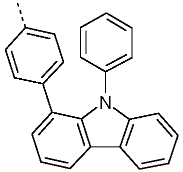 | 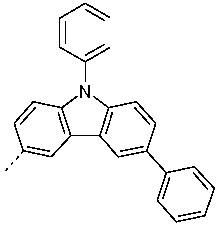 | |
| R-70 | R-71 | R-72 | |
| 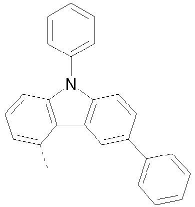 | 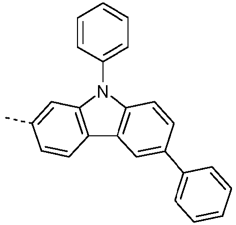 | 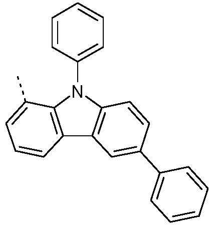 | |
| R-73 | R-74 | R-75 | |
| 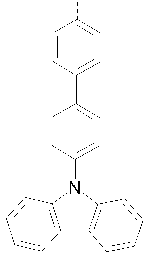 | 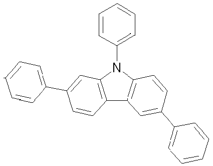 | 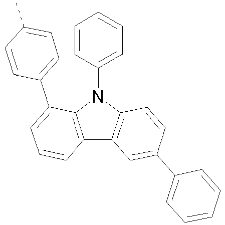 | |
| R-76 | R-77 | R-78 | |
| 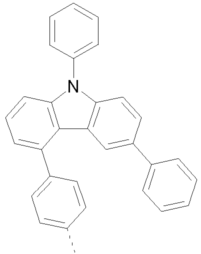 | 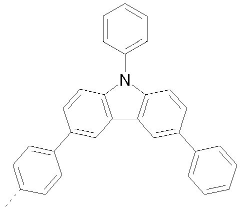 | 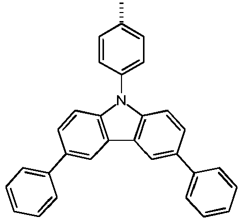 | |
| R-79 | R-80 | R-81 | |
| 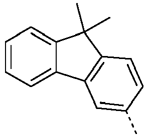 | 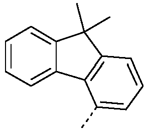 | 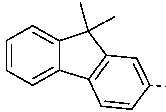 | |
| R-82 | R-83 | R-84 | |
| 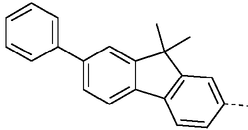 | 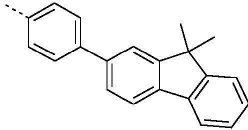 | 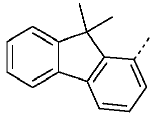 | |
| R-85 | R-86 | R-87 | |
| 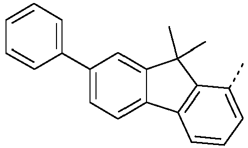 | 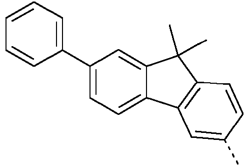 | 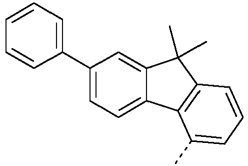 | |
| R-88 | R-89 | R-90 | |
| 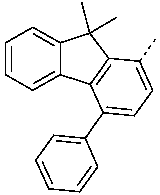 | 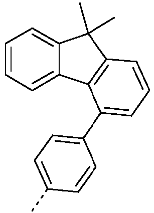 | 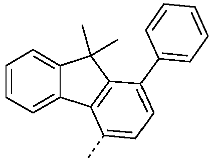 | |
| R-91 | R-92 | R-93 | |
| 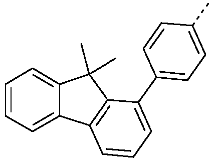 | 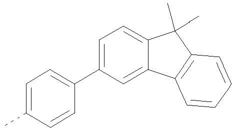 | 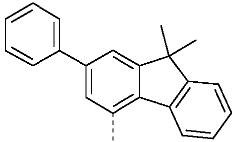 | |
| R-94 | R-95 | R-96 | |
| 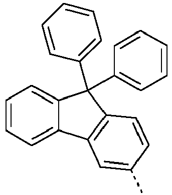 | 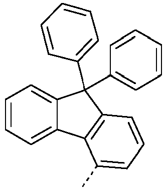 | 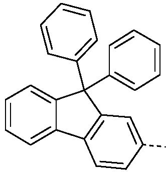 | |
| R-97 | R-98 | R-99 | |
| 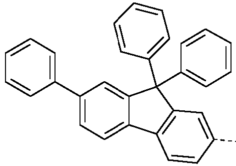 | 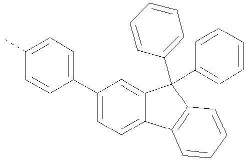 | 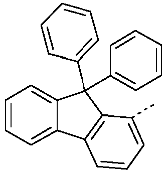 | |
| R-100 | R-101 | R-102 | |
| 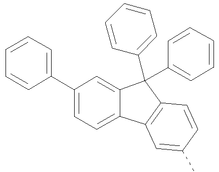 | 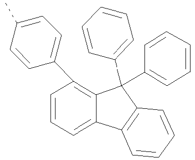 | 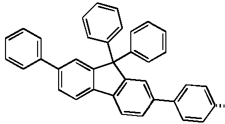 | |
| R-103 | R-104 | R-105 | |
| 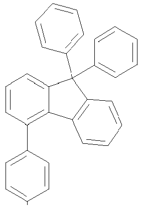 | 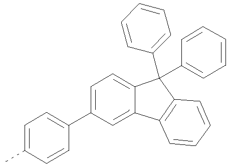 | 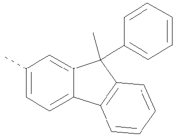 | |
| R-106 | R-107 | R-108 | |
| 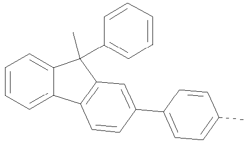 | 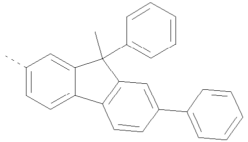 | 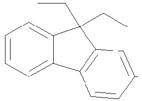 | |
| R-109 | R-110 | R-111 | |
| 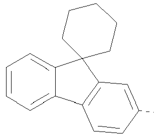 | 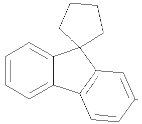 | 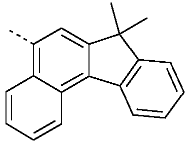 | |
| R-112 | R-113 | R-114 | |
| 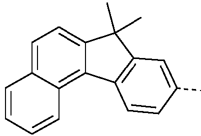 | 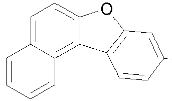 | 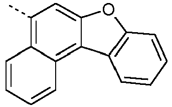 | |
| R-115 | R-116 | R-117 | |
| 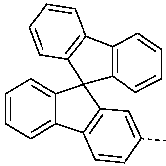 | 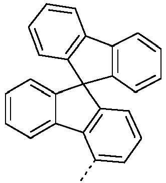 | 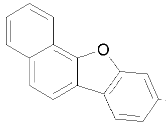 | |
| R-118 | R-119 | R-120 | |
| 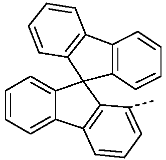 | 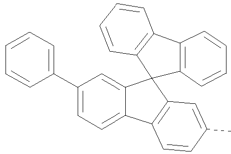 | 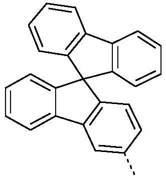 | |
| R-121 | R-122 | R-123 | |
| 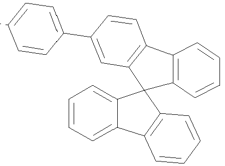 | 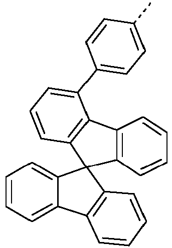 | 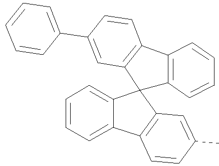 | |
| R-124 | R-125 | R-126 | |
| 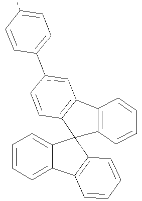 | 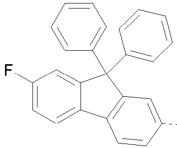 | 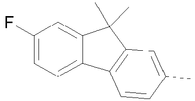 | |
| R-127 | R-128 | R-129 | |
| 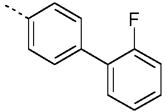 | 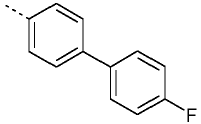 | 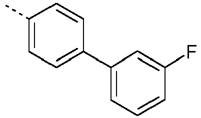 | |
| R-130 | R-131 | R-132 | |
| 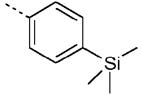 | 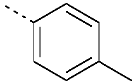 | 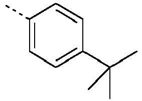 | |
| R-133 | R-134 | R-135 | |
| 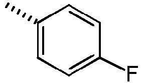 | 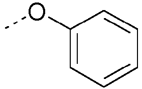 | 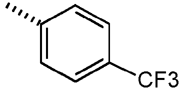 | |
| R-136 | R-137 | R-138 | |
| 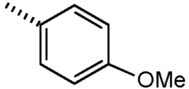 | 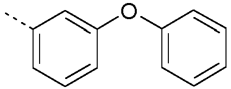 | 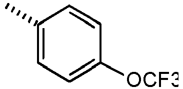 | |
| R-139 | R-140 | R-141 | |
| 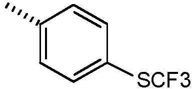 | -CH3 | -CH₂CH₃ | |
| R-142 | R-143 | R-144 | |
| 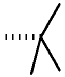 | 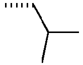 | | |
| R-145 | R-146 | R-147 | |
| -CF₂CF₃ | -CF₃ | -SCF₃ | |
| R-148 | R-149 | R-150 | |
| -OCF₃ | -OCF₂CF₃ | -SCF₂CF₃ | |
| R-151 | R-152 | R-153 | |
| 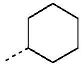 | 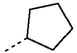 | 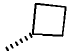 | |
| R-154 | R-155 | R-156 | |
| 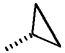 | -SCH₃ | | -OCH₂CH₃ |
| R-157 | R-158 | | R-159 |
| -OCH₃ | -Si(CH₃)₂Ph | | -Si(CH₃)₃ |
| R-160 | R-161 | | R-162 |
| -Si(CH3)₂t-Bu | -Si(iPr)₃ | | |
| R-163 | R-164 | | |
| 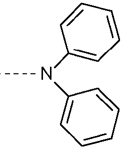 | | 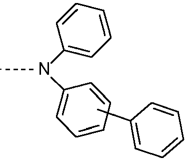 | |
| R-165 | | R-166 | |
| 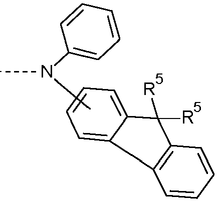 | | 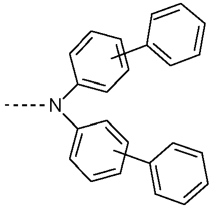 | |
| R-167 | | R-168 | |
| 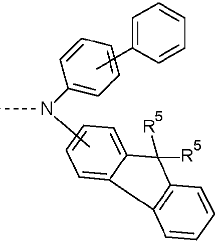 | | 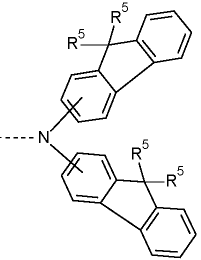 | |
| R-169 | | R-170 | |
| 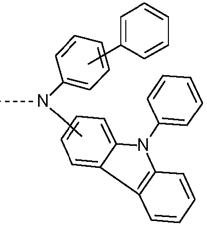 | 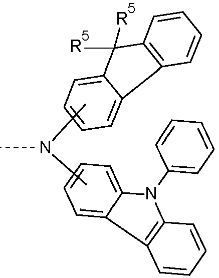 | | |
| R-171 | R-172 | | |
| 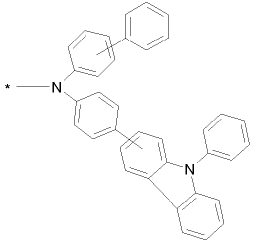 | 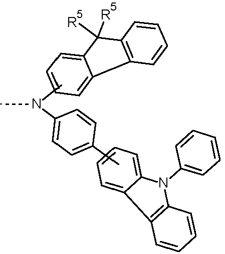 | | |
| R-173 | R-174 | | |
| 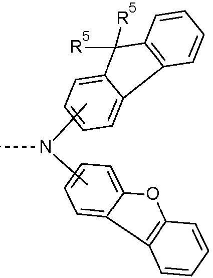 | 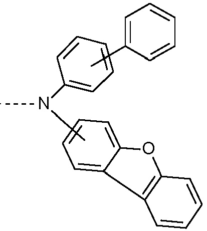 | | |
| R-175 | R-176 | | |
| 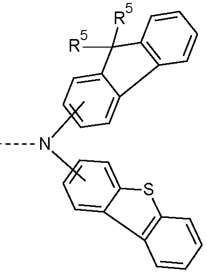 | 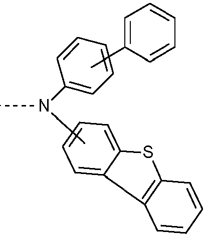 | | |
| R-177 | R-178 | | |
| 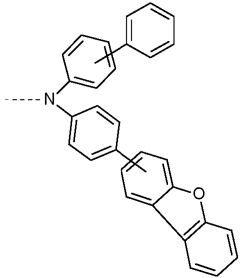 | 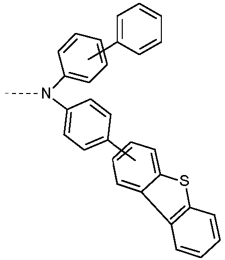 | | |
| R-179 | R-180 | | |
| 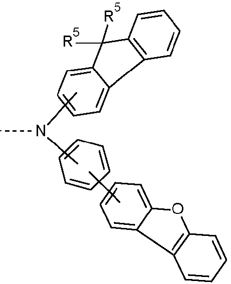 | 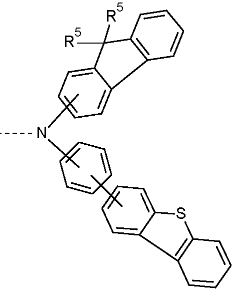 | | |
| R-181 | R-182 | | |
| 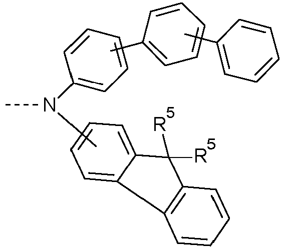 | 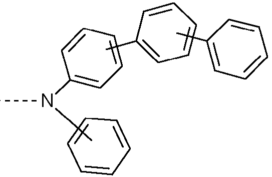 | | |
| R-183 | R-184 | | |
| 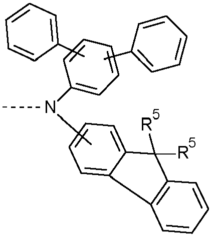 | 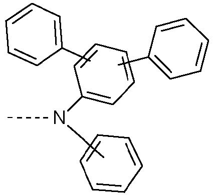 | | |
| R-185 | R-186 | | |
| 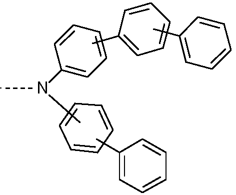 | 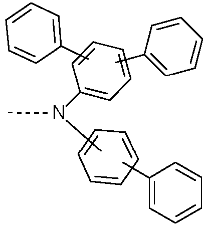 | | |
| R-187 | R-188 | | |
| 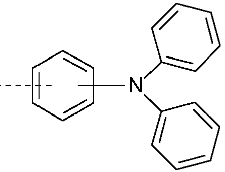 | 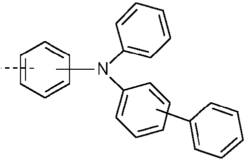 | | |
| R-189 | R-190 | | |
| 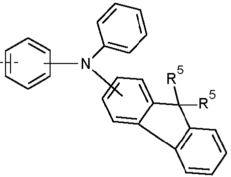 | 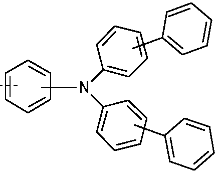 | | |
| R-191 | R-192 | | |
| 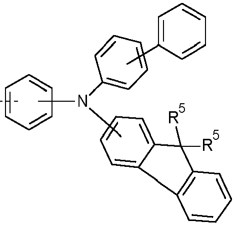 | 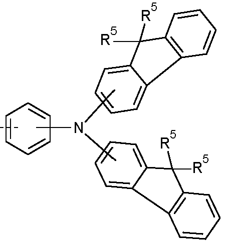 | | |
| R-193 | R-194 | | |
| 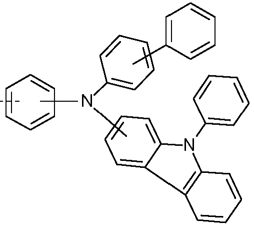 | 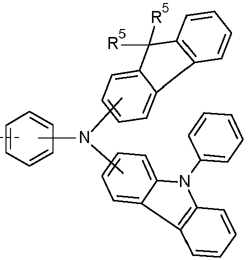 | | |
| R-195 | R-196 | | |
| 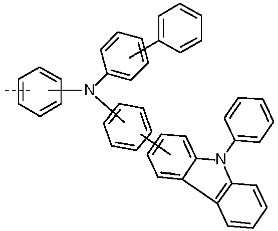 | 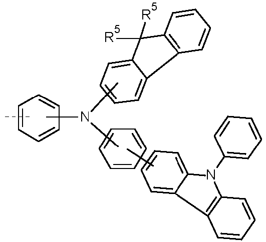 | | |
| R-197 | R-198 | | |
| 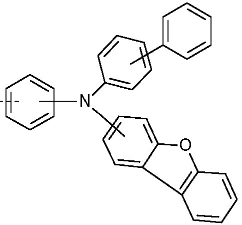 | 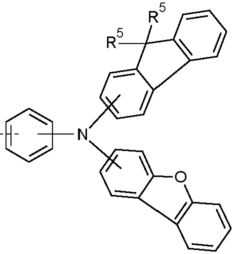 | | |
| R-199 | R-200 | | |
| 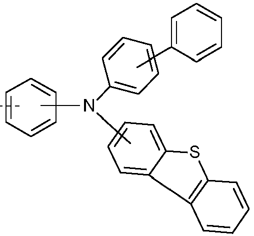 | 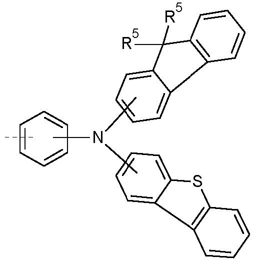 | | |
| R-201 | R-202 | | |
| 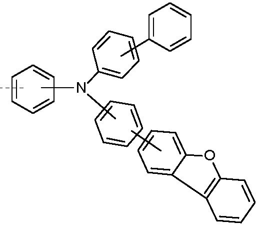 | 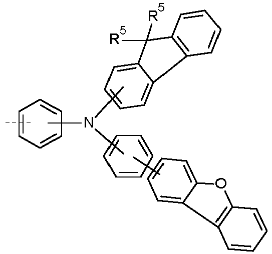 | | |
| R-203 | R-204 | | |
| 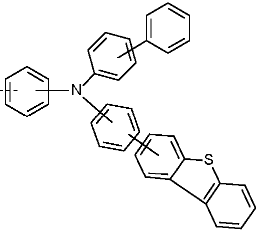 | 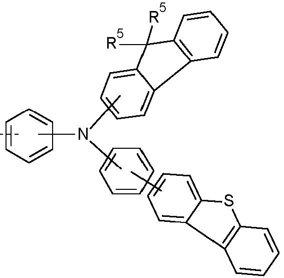 | | |
| R-205 | R-206 | | |
| 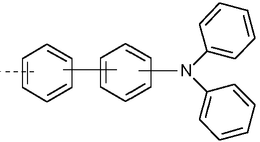 | 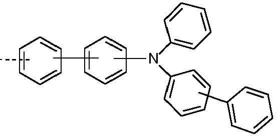 | | |
| R-207 | R-208 | | |
| 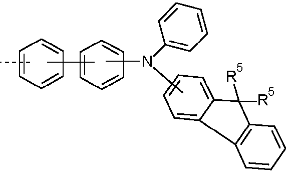 | 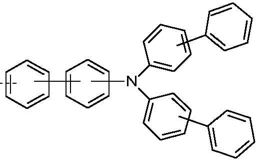 | | |
| R-209 | R-210 | | |
| 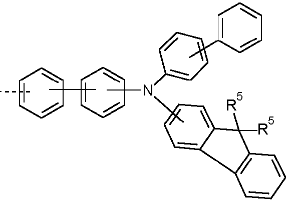 | 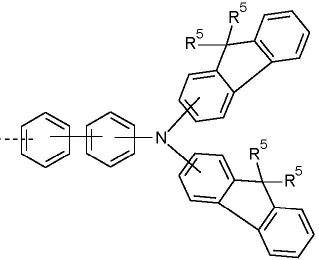 | | |
| R-211 | R-212 | | |

where the groups may be substituted at the free positions with groups R⁵, but are preferably unsubstituted in these positions, and where the dotted line symbolizes the bonding position to the spirobifluorene moiety of formula (1).

Particularly preferred groups R¹ are groups conforming to one of formulae R-1, R-2, R-21, R-58, and R-66.

Groups R² and R³ are preferably selected, identically or differently, from H, F, straight-chain alkyl groups having 1 to 20 C atoms, branched or cyclic alkyl groups having 3 to 20 C atoms, aromatic ring systems having 6 to 30 aromatic ring atoms, and heteroaromatic ring systems having 5 to 30 aromatic ring atoms, where the said alkyl groups, aromatic ring systems and heteroaromatic ring systems may in each case be substituted by one or more radicals R⁵. More preferably, groups R² are H. More preferably, groups R³ are selected, identically or differently, from H, F, methyl, tert-butyl, and phenyl. Most preferably, groups R³ are H.

Groups R⁴ are preferably selected, identically or differently, from H, F, CN, Si(R⁵)₃, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where two or more radicals R⁴ may be connected to each other to form a ring; where the said alkyl groups and the said aromatic and heteroaromatic ring systems may in each case be substituted by one or more radicals R⁵.

Groups R⁵ are preferably selected, identically or differently, from H, F, CN, Si(R⁶)₃, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where two or more radicals R⁵ may be connected to each other to form a ring; where the said alkyl groups and the said aromatic and heteroaromatic ring systems may in each case be substituted by one or more radicals R⁶.

Preferably, one, and not more than one group Z¹ in formula (I) is CR¹. Furthermore, preferably, the group Z¹ which is located in the ortho-position to the bond between the two six-rings is CR¹. Preferably, in this case, the other groups Z¹ are CR².

Formula (I) preferably conforms to one of formulae (I-A) to (I-C)

| | |
|---|---|
| 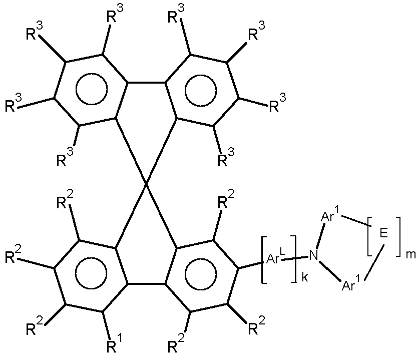 | 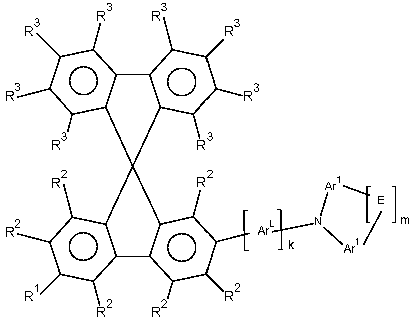 |
| Formula (I-A) | Formula (I-B) |
| 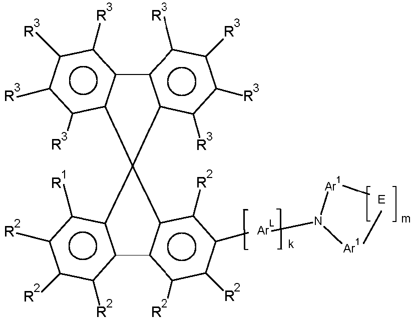 | |
| Formula (I-C) | |

where the variables occurring are defined as above. Preferably, the variables conform to their above-described embodiments.

Among formulae (I-A), (I-B) and (I-C), formula (I-A) is preferred.

Preferred embodiments of formula (I) conform to one of formulae (I-A-1) to (I-C-2)

| | |
|---|---|
| 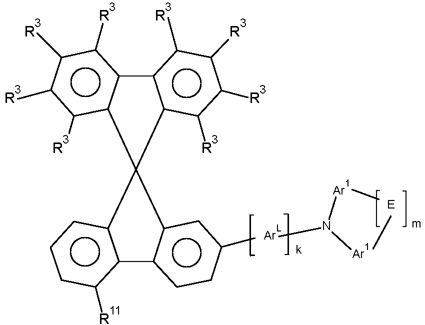 | 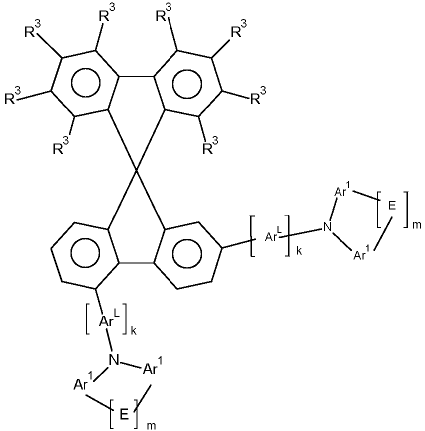 |
| Formula (I-A-1) | Formula (I-A-2) |
| 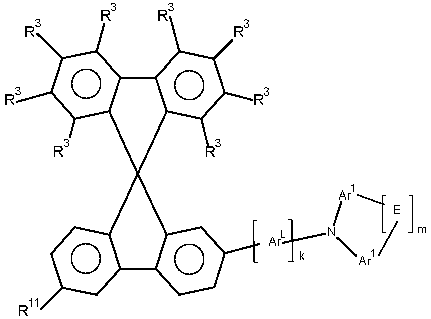 | 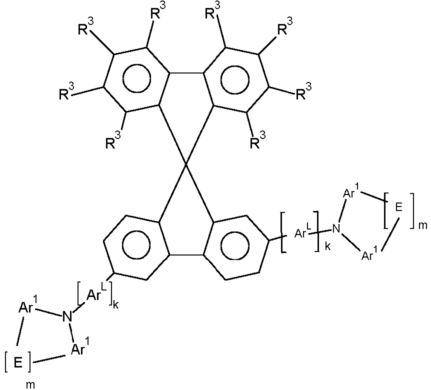 |
| Formula (I-B-1) | Formula (I-B-2) |
| 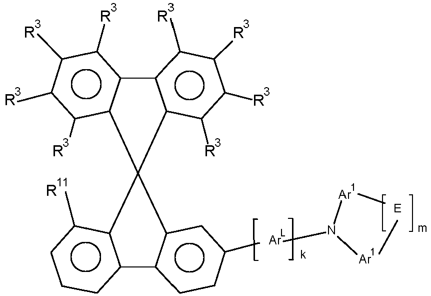 | 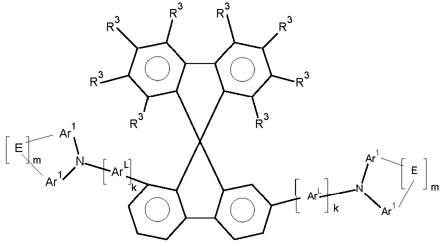 |
| Formula (I-C-1) | Formula (I-C-2) |

where the variables occurring are as defined above, and R¹¹ is selected from F, Cl, Br, I, CN, SCN, SF₅, Si(R⁵)₃, straight-chain alkyl, alkoxy or thioalkyl groups having 1 to 20 C atoms, branched or cyclic alkyl, alkoxy or thioalkyl groups having 3 to 20 C atoms, aromatic ring systems having 6 to 40 aromatic ring atoms, and heteroaromatic ring systems having 5 to 40 aromatic ring atoms, where the said alkyl, alkoxy and thioalkyl groups and the said aromatic and heteroaromatic ring systems may in each case be substituted by one or more radicals R⁵, and is preferably selected from aromatic ring systems having 6 to 30 aromatic ring atoms, and heteroaromatic ring systems having 5 to 30 aromatic ring atoms, where the said aromatic and heteroaromatic ring systems may in each case be substituted by one or more radicals R⁵.

Among the above formulae, formulae (I-A-1) and (I-A-2) are preferred.

Particularly preferably embodiments of formula (I) conform to one of formulae (I-A-1-1) to (I-C-2-2)

| | |
|---|---|
| 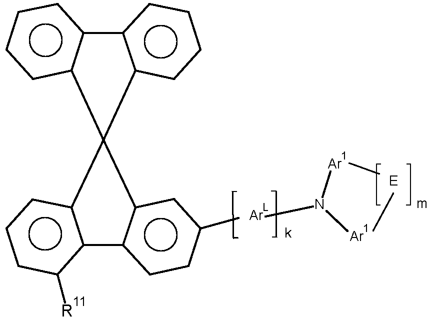 | 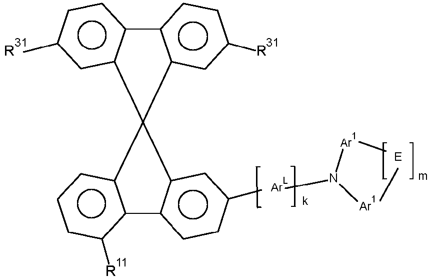 |
| Formula (I-A-1-1) | Formula (I-A-1-2) |
| 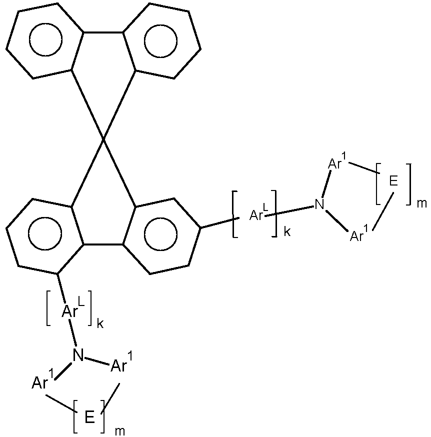 | 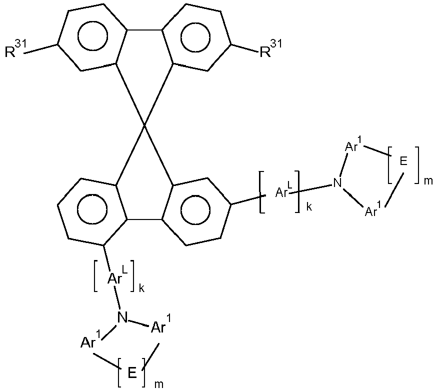 |
| Formula (I-A-2-1) | Formula (I-A-2-2) |
| 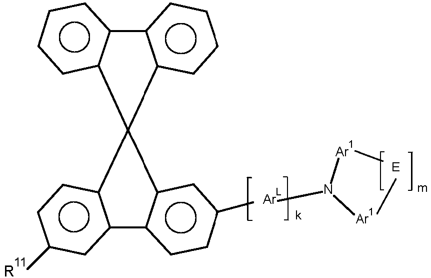 | 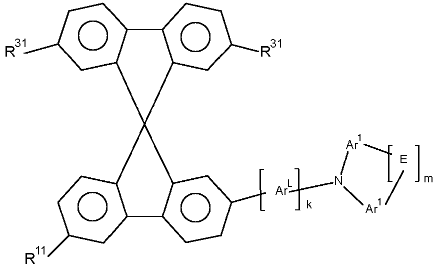 |
| Formula (I-B-1-1) | Formula (I-B-1-2) |
| 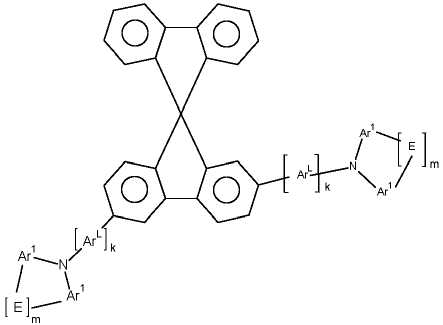 | 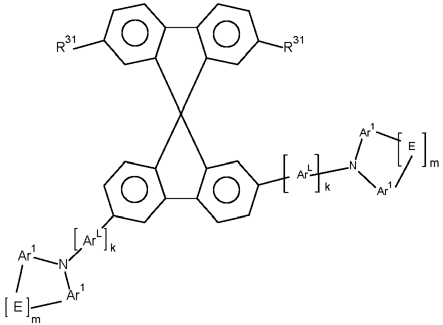 |
| Formula (I-B-2-1) | Formula (I-B-2-2) |
| 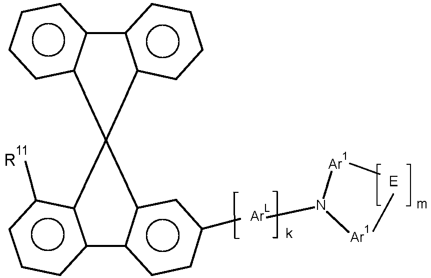 | 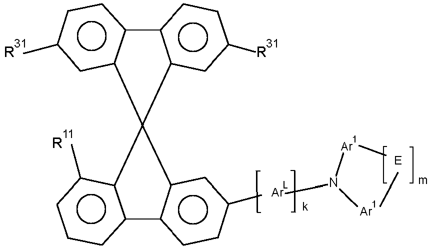 |
| Formula (I-C-1-1) | Formula (I-C-1-2) |
| 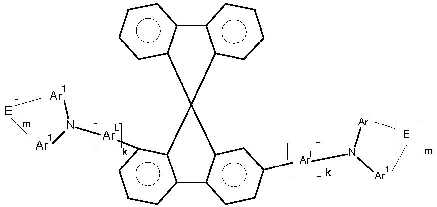 | 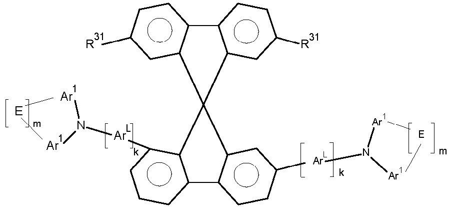 |
| Formula (I-C-2-1) | Formula (I-C-2-2) |

where the variables occurring are defined as above, and where R³¹ is selected, identically or differently, from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³¹ may be connected to each other to form a ring; where the said alkyl, alkoxy, alkenyl and alkynyl groups and the said aromatic and heteroaromatic ring systems may in each case be substituted by one or more radicals R⁵, and where one or more CH₂ groups in the said alkyl, alkoxy, alkenyl and alkynyl groups may in each case be replaced by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂, where at least one group R³¹ is different from H and D. Preferably, R³¹ is selected, identically or differently on each occurrence, from H, F, straight-chain alkyl groups having 1 to 20 C atoms, branched or cyclic alkyl groups having 3 to 20 C atoms, aromatic ring systems having 6 to 30 aromatic ring atoms, and heteroaromatic ring systems having 5 to 30 aromatic ring atoms, where the said alkyl groups, aromatic ring systems and heteroaromatic ring systems may in each case be substituted by one or more radicals R⁵, where at least one group R³¹ is different from H. More preferably, R³¹ is selected, identically or differently on each occurrence, from H, F, methyl, tert-butyl, and phenyl, where at least one group R³¹ is different from H. Most preferably, both groups R³¹ are different from H and D for the above embodiments.

Among the above formulae, formulae (I-A-1-1), (I-A-1-2), (I-A-2-1) and (I-A-2-2) are preferred. Particularly preferred are formulae (I-A-1-1) and (I-A-2-1).

Particularly preferred specific compounds are the following compounds, which conform to the formula (I-A-2-2-1) below 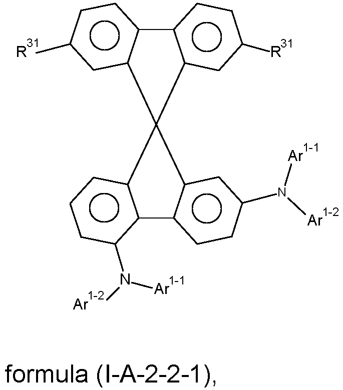 in which R³¹, Ar¹⁻¹ and Ar¹⁻² are specified as shown in the list below (formulae Ar-1 to Ar-172 are as specified above):

| **No.** | **R³¹** | **Ar¹⁻¹** | **Ar¹⁻²** | **No.** | **R³¹** | **Ar¹⁻¹** | **Ar¹⁻²** |
|---|---|---|---|---|---|---|---|
| C-1 | H | Ar-1 | Ar-1 | C-157 | Methyl | Ar-1 | Ar-1 |
| C-2 | " | " | Ar-2 | C-158 | " | " | Ar-2 |
| C-3 | " | " | Ar-4 | C-159 | " | " | Ar-4 |
| C-4 | " | " | Ar-5 | C-160 | " | " | Ar-5 |
| C-5 | " | " | Ar-74 | C-161 | " | " | Ar-74 |
| C-6 | " | " | Ar-78 | C-162 | " | " | Ar-78 |
| C-7 | " | " | Ar-82 | C-163 | " | " | Ar-82 |
| C-8 | " | " | Ar-117 | C-164 | " | " | Ar-117 |
| C-9 | " | " | Ar-134 | C-165 | " | " | Ar-134 |
| C-10 | " | " | Ar-139 | C-166 | " | " | Ar-139 |
| C-11 | " | " | Ar-150 | C-167 | " | " | Ar-150 |
| C-12 | " | " | Ar-172 | C-168 | " | " | Ar-172 |
| C-13 | " | Ar-2 | Ar-2 | C-169 | " | Ar-2 | Ar-2 |
| C-14 | " | " | Ar-4 | C-170 | " | " | Ar-4 |
| C-15 | " | " | Ar-5 | C-171 | " | " | Ar-5 |
| C-16 | " | " | Ar-74 | C-172 | " | " | Ar-74 |
| C-17 | " | " | Ar-78 | C-173 | " | " | Ar-78 |
| C-18 | " | " | Ar-82 | C-174 | " | " | Ar-82 |
| C-19 | " | " | Ar-117 | C-175 | " | " | Ar-117 |
| C-20 | " | " | Ar-134 | C-176 | " | " | Ar-134 |
| C-21 | " | " | Ar-139 | C-177 | " | " | Ar-139 |
| C-22 | " | " | Ar-150 | C-178 | " | " | Ar-150 |
| C-23 | " | " | Ar-172 | C-179 | " | " | Ar-172 |
| C-24 | " | Ar-4 | Ar-4 | C-180 | " | Ar-4 | Ar-4 |
| C-25 | " | " | Ar-5 | C-181 | " | " | Ar-5 |
| C-26 | " | " | Ar-74 | C-182 | " | " | Ar-74 |
| C-27 | " | " | Ar-78 | C-183 | " | " | Ar-78 |
| C-28 | " | " | Ar-82 | C-184 | " | " | Ar-82 |
| C-29 | " | " | Ar-117 | C-185 | " | " | Ar-117 |
| C-30 | " | " | Ar-134 | C-186 | " | " | Ar-134 |
| C-31 | " | " | Ar-139 | C-187 | " | " | Ar-139 |
| C-32 | " | " | Ar-150 | C-188 | " | " | Ar-150 |
| C-33 | " | " | Ar-172 | C-189 | " | " | Ar-172 |
| C-34 | " | Ar-5 | Ar-5 | C-190 | " | Ar-5 | Ar-5 |
| C-35 | " | " | Ar-74 | C-191 | " | " | Ar-74 |
| C-36 | " | " | Ar-78 | C-192 | " | " | Ar-78 |
| C-37 | " | " | Ar-82 | C-193 | " | " | Ar-82 |
| C-38 | " | " | Ar-117 | C-194 | " | " | Ar-117 |
| C-39 | " | " | Ar-134 | C-195 | " | " | Ar-134 |
| C-40 | " | " | Ar-139 | C-196 | " | " | Ar-139 |
| C-41 | " | " | Ar-150 | C-197 | " | " | Ar-150 |
| C-42 | " | " | Ar-172 | C-198 | " | " | Ar-172 |
| C-43 | " | Ar-74 | Ar-74 | C-199 | " | Ar-74 | Ar-74 |
| C-44 | " | " | Ar-78 | C-200 | " | " | Ar-78 |
| C-45 | " | " | Ar-82 | C-201 | " | " | Ar-82 |
| C-46 | " | " | Ar-117 | C-202 | " | " | Ar-117 |
| C-47 | " | " | Ar-134 | C-203 | " | " | Ar-134 |
| C-48 | " | " | Ar-139 | C-204 | " | " | Ar-139 |
| C-49 | " | " | Ar-150 | C-205 | " | " | Ar-150 |
| C-50 | " | " | Ar-172 | C-206 | " | " | Ar-172 |
| C-51 | " | Ar-78 | Ar-78 | C-207 | " | Ar-78 | Ar-78 |
| C-52 | " | " | Ar-82 | C-208 | " | " | Ar-82 |
| C-53 | " | " | Ar-117 | C-209 | " | " | Ar-117 |
| C-54 | " | " | Ar-134 | C-210 | " | " | Ar-134 |
| C-55 | " | " | Ar-139 | C-211 | " | " | Ar-139 |
| C-56 | " | " | Ar-150 | C-212 | " | " | Ar-150 |
| C-57 | " | " | Ar-172 | C-213 | " | " | Ar-172 |
| C-58 | " | Ar-82 | Ar-82 | C-214 | " | Ar-82 | Ar-82 |
| C-59 | " | " | Ar-117 | C-215 | " | " | Ar-117 |
| C-60 | " | " | Ar-134 | C-216 | " | " | Ar-134 |
| C-61 | " | " | Ar-139 | C-217 | " | " | Ar-139 |
| C-62 | " | " | Ar-150 | C-218 | " | " | Ar-150 |
| C-63 | " | " | Ar-172 | C-219 | " | " | Ar-172 |
| C-64 | " | Ar-117 | Ar-117 | C-220 | " | Ar-117 | Ar-117 |
| C-65 | " | " | Ar-134 | C-221 | " | " | Ar-134 |
| C-66 | " | " | Ar-139 | C-222 | " | " | Ar-139 |
| C-67 | " | " | Ar-150 | C-223 | " | " | Ar-150 |
| C-68 | " | " | Ar-172 | C-224 | " | " | Ar-172 |
| C-69 | " | Ar-134 | Ar-134 | C-225 | " | Ar-134 | Ar-134 |
| C-70 | " | " | Ar-139 | C-226 | " | " | Ar-139 |
| C-71 | " | " | Ar-150 | C-227 | " | " | Ar-150 |
| C-72 | " | " | Ar-172 | C-228 | " | " | Ar-172 |
| C-73 | " | Ar-139 | Ar-139 | C-229 | " | Ar-139 | Ar-139 |
| C-74 | " | " | Ar-150 | C-230 | " | " | Ar-150 |
| C-75 | " | " | Ar-172 | C-231 | " | " | Ar-172 |
| C-76 | " | Ar-150 | Ar-150 | C-232 | " | Ar-150 | Ar-150 |
| C-77 | " | " | Ar-172 | C-233 | " | " | Ar-172 |
| C-78 | " | Ar-172 | Ar-172 | C-234 | " | Ar-172 | Ar-172 |
| C-79 | F | Ar-1 | Ar-1 | C-235 | tert-Butyl | Ar-1 | Ar-1 |
| C-80 | " | " | Ar-2 | C-236 | " | " | Ar-2 |
| C-81 | " | " | Ar-4 | C-237 | " | " | Ar-4 |
| C-82 | " | " | Ar-5 | C-238 | " | " | Ar-5 |
| C-83 | " | " | Ar-74 | C-239 | " | " | Ar-74 |
| C-84 | " | " | Ar-78 | C-240 | " | " | Ar-78 |
| C-85 | " | " | Ar-82 | C-241 | " | " | Ar-82 |
| C-86 | " | " | Ar-117 | C-242 | " | " | Ar-117 |
| C-87 | " | " | Ar-134 | C-243 | " | " | Ar-134 |
| C-88 | " | " | Ar-139 | C-244 | " | " | Ar-139 |
| C-89 | " | " | Ar-150 | C-245 | " | " | Ar-150 |
| C-90 | " | " | Ar-172 | C-246 | " | " | Ar-172 |
| C-91 | " | Ar-2 | Ar-2 | C-247 | " | Ar-2 | Ar-2 |
| C-92 | " | " | Ar-4 | C-248 | " | " | Ar-4 |
| C-93 | " | " | Ar-5 | C-249 | " | " | Ar-5 |
| C-94 | " | " | Ar-74 | C-250 | " | " | Ar-74 |
| C-95 | " | " | Ar-78 | C-251 | " | " | Ar-78 |
| C-96 | " | " | Ar-82 | C-252 | " | " | Ar-82 |
| C-97 | " | " | Ar-117 | C-253 | " | " | Ar-117 |
| C-98 | " | " | Ar-134 | C-254 | " | " | Ar-134 |
| C-99 | " | " | Ar-139 | C-255 | " | " | Ar-139 |
| C-100 | " | " | Ar-150 | C-256 | " | " | Ar-150 |
| C-101 | " | " | Ar-172 | C-257 | " | " | Ar-172 |
| C-102 | " | Ar-4 | Ar-4 | C-258 | " | Ar-4 | Ar-4 |
| C-103 | " | " | Ar-5 | C-259 | " | " | Ar-5 |
| C-104 | " | " | Ar-74 | C-260 | " | " | Ar-74 |
| C-105 | " | " | Ar-78 | C-261 | " | " | Ar-78 |
| C-106 | " | " | Ar-82 | C-262 | " | " | Ar-82 |
| C-107 | " | " | Ar-117 | C-263 | " | " | Ar-117 |
| C-108 | " | " | Ar-134 | C-264 | " | " | Ar-134 |
| C-109 | " | " | Ar-139 | C-265 | " | " | Ar-139 |
| C-110 | " | " | Ar-150 | C-266 | " | " | Ar-150 |
| C-111 | " | " | Ar-172 | C-267 | " | " | Ar-172 |
| C-112 | " | Ar-5 | Ar-5 | C-268 | " | Ar-5 | Ar-5 |
| C-113 | " | " | Ar-74 | C-269 | " | " | Ar-74 |
| C-114 | " | " | Ar-78 | C-270 | " | " | Ar-78 |
| C-115 | " | " | Ar-82 | C-271 | " | " | Ar-82 |
| C-116 | " | " | Ar-117 | C-272 | " | " | Ar-117 |
| C-117 | " | " | Ar-134 | C-273 | " | " | Ar-134 |
| C-118 | " | " | Ar-139 | C-274 | " | " | Ar-139 |
| C-119 | " | " | Ar-150 | C-275 | " | " | Ar-150 |
| C-120 | " | " | Ar-172 | C-276 | " | " | Ar-172 |
| C-121 | " | Ar-74 | Ar-74 | C-277 | " | Ar-74 | Ar-74 |
| C-122 | " | " | Ar-78 | C-278 | " | " | Ar-78 |
| C-123 | " | " | Ar-82 | C-279 | " | " | Ar-82 |
| C-124 | " | " | Ar-117 | C-280 | " | " | Ar-117 |
| C-125 | " | " | Ar-134 | C-281 | " | " | Ar-134 |
| C-126 | " | " | Ar-139 | C-282 | " | " | Ar-139 |
| C-127 | " | " | Ar-150 | C-283 | " | " | Ar-150 |
| C-128 | " | " | Ar-172 | C-284 | " | " | Ar-172 |
| C-129 | " | Ar-78 | Ar-78 | C-285 | " | Ar-78 | Ar-78 |
| C-130 | " | " | Ar-82 | C-286 | " | " | Ar-82 |
| C-131 | " | " | Ar-117 | C-287 | " | " | Ar-117 |
| C-132 | " | " | Ar-134 | C-288 | " | " | Ar-134 |
| C-133 | " | " | Ar-139 | C-289 | " | " | Ar-139 |
| C-134 | " | " | Ar-150 | C-290 | " | " | Ar-150 |
| C-135 | " | " | Ar-172 | C-291 | " | " | Ar-172 |
| C-136 | " | Ar-82 | Ar-82 | C-292 | " | Ar-82 | Ar-82 |
| C-137 | " | " | Ar-117 | C-293 | " | " | Ar-117 |
| C-138 | " | " | Ar-134 | C-294 | " | " | Ar-134 |
| C-139 | " | " | Ar-139 | C-295 | " | " | Ar-139 |
| C-140 | " | " | Ar-150 | C-296 | " | " | Ar-150 |
| C-141 | " | " | Ar-172 | C-297 | " | " | Ar-172 |
| C-142 | " | Ar-117 | Ar-117 | C-298 | " | Ar-117 | Ar-117 |
| C-143 | " | " | Ar-134 | C-299 | " | " | Ar-134 |
| C-144 | " | " | Ar-139 | C-300 | " | " | Ar-139 |
| C-145 | " | " | Ar-150 | C-301 | " | " | Ar-150 |
| C-146 | " | " | Ar-172 | C-302 | " | " | Ar-172 |
| C-147 | " | Ar-134 | Ar-134 | C-303 | " | Ar-134 | Ar-134 |
| C-148 | " | " | Ar-139 | C-304 | " | " | Ar-139 |
| C-149 | " | " | Ar-150 | C-305 | " | " | Ar-150 |
| C-150 | " | " | Ar-172 | C-306 | " | " | Ar-172 |
| C-151 | " | Ar-139 | Ar-139 | C-307 | " | Ar-139 | Ar-139 |
| C-152 | " | " | Ar-150 | C-308 | " | " | Ar-150 |
| C-153 | " | " | Ar-172 | C-309 | " | " | Ar-172 |
| C-154 | " | Ar-150 | Ar-150 | C-310 | " | Ar-150 | Ar-150 |
| C-155 | " | " | Ar-172 | C-311 | " | " | Ar-172 |
| C-156 | " | Ar-172 | Ar-172 | C-312 | " | Ar-172 | Ar-172 |

Furthermore preferred are compounds which correspond to the compounds C-1 to C-312 above, with the exception that they are derived from the following formulae

| | |
|---|---|
| 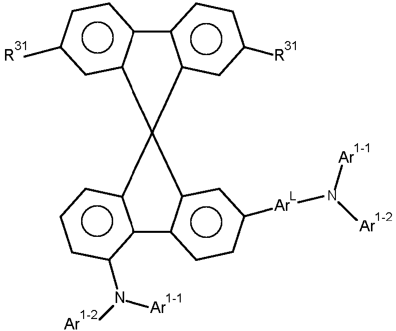 | 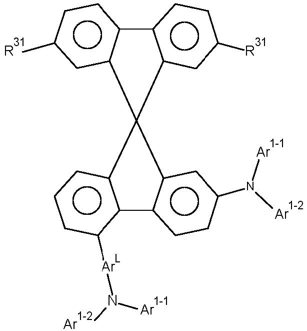 |
| Formula (I-A-2-2-2) | Formula (I-A-2-2-3) |

| | |
|---|---|
| 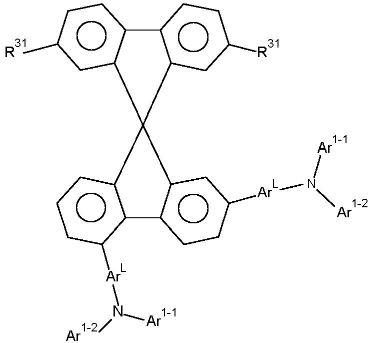 | |
| Formula (I-A-2-2-4) | |

where Ar^{L} is phenylene, preferably 1,4-phenylene, and where R³¹, Ar¹⁻¹ and Ar¹⁻² are specified as shown for the corresponding compounds C-1 to C-312.

Particularly preferred specific compounds are the following compounds, which conform to the formula (I-A-1-2-1) below 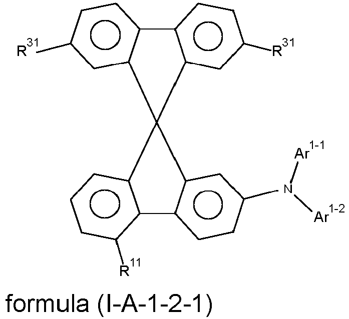 in which R¹¹, R³¹, Ar¹⁻¹ and Ar¹⁻² are specified as shown in the list below (formulae Ar-1 to Ar-172 and R-1 to R-66 are as specified above):

| **No.** | **R¹¹** | **R³¹** | **Ar¹⁻¹** | **Ar¹⁻²** | **No.** | **R¹¹** | **R³¹** | **Ar¹⁻¹** | **Ar¹⁻²** |
|---|---|---|---|---|---|---|---|---|---|
| C-313 | R-1 | H | Ar-1 | Ar-1 | C-1093 | " | Methyl | Ar-1 | Ar-1 |
| C-314 | " | " | " | Ar-2 | C-1094 | " | " | " | Ar-2 |
| C-315 | " | " | " | Ar-4 | C-1095 | " | " | " | Ar-4 |
| C-316 | " | " | " | Ar-5 | C-1096 | " | " | " | Ar-5 |
| C-317 | " | " | " | Ar-74 | C-1097 | " | " | " | Ar-74 |
| C-318 | " | " | " | Ar-78 | C-1098 | " | " | " | Ar-78 |
| C-319 | " | " | " | Ar-82 | C-1099 | " | " | " | Ar-82 |
| C-320 | " | " | " | Ar-117 | C-1100 | " | " | " | Ar-117 |
| C-321 | " | " | " | Ar-134 | C-1101 | " | " | " | Ar-134 |
| C-322 | " | " | " | Ar-139 | C-1102 | " | " | " | Ar-139 |
| C-323 | " | " | " | Ar-150 | C-1103 | " | " | " | Ar-150 |
| C-324 | " | " | " | Ar-172 | C-1104 | " | " | " | Ar-172 |
| C-325 | " | " | Ar-2 | Ar-2 | C-1105 | " | " | Ar-2 | Ar-2 |
| C-326 | " | " | " | Ar-4 | C-1106 | " | " | " | Ar-4 |
| C-327 | " | " | " | Ar-5 | C-1107 | " | " | " | Ar-5 |
| C-328 | " | " | " | Ar-74 | C-1108 | " | " | " | Ar-74 |
| C-329 | " | " | " | Ar-78 | C-1109 | " | " | " | Ar-78 |
| C-330 | " | " | " | Ar-82 | C-1110 | " | " | " | Ar-82 |
| C-331 | " | " | " | Ar-117 | C-1111 | " | " | " | Ar-117 |
| C-332 | " | " | " | Ar-134 | C-1112 | " | " | " | Ar-134 |
| C-333 | " | " | " | Ar-139 | C-1113 | " | " | " | Ar-139 |
| C-334 | " | " | " | Ar-150 | C-1114 | " | " | " | Ar-150 |
| C-335 | " | " | " | Ar-172 | C-1115 | " | " | " | Ar-172 |
| C-336 | " | " | Ar-4 | Ar-4 | C-1116 | " | " | Ar-4 | Ar-4 |
| C-337 | " | " | " | Ar-5 | C-1117 | " | " | " | Ar-5 |
| C-338 | " | " | " | Ar-74 | C-1118 | " | " | " | Ar-74 |
| C-339 | " | " | " | Ar-78 | C-1119 | " | " | " | Ar-78 |
| C-340 | " | " | " | Ar-82 | C-1120 | " | " | " | Ar-82 |
| C-341 | " | " | " | Ar-117 | C-1121 | " | " | " | Ar-117 |
| C-342 | " | " | " | Ar-134 | C-1122 | " | " | " | Ar-134 |
| C-343 | " | " | " | Ar-139 | C-1123 | " | " | " | Ar-139 |
| C-344 | " | " | " | Ar-150 | C-1124 | " | " | " | Ar-150 |
| C-345 | " | " | " | Ar-172 | C-1125 | " | " | " | Ar-172 |
| C-346 | " | " | Ar-5 | Ar-5 | C-1126 | " | " | Ar-5 | Ar-5 |
| C-347 | " | " | " | Ar-74 | C-1127 | " | " | " | Ar-74 |
| C-348 | " | " | " | Ar-78 | C-1128 | " | " | " | Ar-78 |
| C-349 | " | " | " | Ar-82 | C-1129 | " | " | " | Ar-82 |
| C-350 | " | " | " | Ar-117 | C-1130 | " | " | " | Ar-117 |
| C-351 | " | " | " | Ar-134 | C-1131 | " | " | " | Ar-134 |
| C-352 | " | " | " | Ar-139 | C-1132 | " | " | " | Ar-139 |
| C-353 | " | " | " | Ar-150 | C-1133 | " | " | " | Ar-150 |
| C-354 | " | " | " | Ar-172 | C-1134 | " | " | " | Ar-172 |
| C-355 | " | " | Ar-74 | Ar-74 | C-1135 | " | " | Ar-74 | Ar-74 |
| C-356 | " | " | " | Ar-78 | C-1136 | " | " | " | Ar-78 |
| C-357 | " | " | " | Ar-82 | C-1137 | " | " | " | Ar-82 |
| C-358 | " | " | " | Ar-117 | C-1138 | " | " | " | Ar-117 |
| C-359 | " | " | " | Ar-134 | C-1139 | " | " | " | Ar-134 |
| C-360 | " | " | " | Ar-139 | C-1140 | " | " | " | Ar-139 |
| C-361 | " | " | " | Ar-150 | C-1141 | " | " | " | Ar-150 |
| C-362 | " | " | " | Ar-172 | C-1142 | " | " | " | Ar-172 |
| C-363 | " | " | Ar-78 | Ar-78 | C-1143 | " | " | Ar-78 | Ar-78 |
| C-364 | " | " | " | Ar-82 | C-1144 | " | " | " | Ar-82 |
| C-365 | " | " | " | Ar-117 | C-1145 | " | " | " | Ar-117 |
| C-366 | " | " | " | Ar-134 | C-1146 | " | " | " | Ar-134 |
| C-367 | " | " | " | Ar-139 | C-1147 | " | " | " | Ar-139 |
| C-368 | " | " | " | Ar-150 | C-1148 | " | " | " | Ar-150 |
| C-369 | " | " | " | Ar-172 | C-1149 | " | " | " | Ar-172 |
| C-370 | " | " | Ar-82 | Ar-82 | C-1150 | " | " | Ar-82 | Ar-82 |
| C-371 | " | " | " | Ar-117 | C-1151 | " | " | " | Ar-117 |
| C-372 | " | " | " | Ar-134 | C-1152 | " | " | " | Ar-134 |
| C-373 | " | " | " | Ar-139 | C-1153 | " | " | " | Ar-139 |
| C-374 | " | " | " | Ar-150 | C-1154 | " | " | " | Ar-150 |
| C-375 | " | " | " | Ar-172 | C-1155 | " | " | " | Ar-172 |
| C-376 | " | " | Ar-117 | Ar-117 | C-1156 | " | " | Ar-117 | Ar-117 |
| C-377 | " | " | " | Ar-134 | C-1157 | " | " | " | Ar-134 |
| C-378 | " | " | " | Ar-139 | C-1158 | " | " | " | Ar-139 |
| C-379 | " | " | " | Ar-150 | C-1159 | " | " | " | Ar-150 |
| C-380 | " | " | " | Ar-172 | C-1160 | " | " | " | Ar-172 |
| C-381 | " | " | Ar-134 | Ar-134 | C-1161 | " | " | Ar-134 | Ar-134 |
| C-382 | " | " | " | Ar-139 | C-1162 | " | " | " | Ar-139 |
| C-383 | " | " | " | Ar-150 | C-1163 | " | " | " | Ar-150 |
| C-384 | " | " | " | Ar-172 | C-1164 | " | " | " | Ar-172 |
| C-385 | " | " | Ar-139 | Ar-139 | C-1165 | " | " | Ar-139 | Ar-139 |
| C-386 | " | " | " | Ar-150 | C-1166 | " | " | " | Ar-150 |
| C-387 | " | " | " | Ar-172 | C-1167 | " | " | " | Ar-172 |
| C-388 | " | " | Ar-150 | Ar-150 | C-1168 | " | " | Ar-150 | Ar-150 |
| C-389 | " | " | " | Ar-172 | C-1169 | " | " | " | Ar-172 |
| C-390 | " | " | Ar-172 | Ar-172 | C-1170 | " | " | Ar-172 | Ar-172 |
| C-391 | " | F | Ar-1 | Ar-1 | C-1171 | " | tert-Butyl | Ar-1 | Ar-1 |
| C-392 | " | " | " | Ar-2 | C-1172 | " | " | " | Ar-2 |
| C-393 | " | " | " | Ar-4 | C-1173 | " | " | " | Ar-4 |
| C-394 | " | " | " | Ar-5 | C-1174 | " | " | " | Ar-5 |
| C-395 | " | " | " | Ar-74 | C-1175 | " | " | " | Ar-74 |
| C-396 | " | " | " | Ar-78 | C-1176 | " | " | " | Ar-78 |
| C-397 | " | " | " | Ar-82 | C-1177 | " | " | " | Ar-82 |
| C-398 | " | " | " | Ar-117 | C-1178 | " | " | " | Ar-117 |
| C-399 | " | " | " | Ar-134 | C-1179 | " | " | " | Ar-134 |
| C-400 | " | " | " | Ar-139 | C-1180 | " | " | " | Ar-139 |
| C-401 | " | " | " | Ar-150 | C-1181 | " | " | " | Ar-150 |
| C-402 | " | " | " | Ar-172 | C-1182 | " | " | " | Ar-172 |
| C-403 | " | " | Ar-2 | Ar-2 | C-1183 | " | " | Ar-2 | Ar-2 |
| C-404 | " | " | " | Ar-4 | C-1184 | " | " | " | Ar-4 |
| C-405 | " | " | " | Ar-5 | C-1185 | " | " | " | Ar-5 |
| C-406 | " | " | " | Ar-74 | C-1186 | " | " | " | Ar-74 |
| C-407 | " | " | " | Ar-78 | C-1187 | " | " | " | Ar-78 |
| C-408 | " | " | " | Ar-82 | C-1188 | " | " | " | Ar-82 |
| C-409 | " | " | " | Ar-117 | C-1189 | " | " | " | Ar-117 |
| C-410 | " | " | " | Ar-134 | C-1190 | " | " | " | Ar-134 |
| C-411 | " | " | " | Ar-139 | C-1191 | " | " | " | Ar-139 |
| C-412 | " | " | " | Ar-150 | C-1192 | " | " | " | Ar-150 |
| C-413 | " | " | " | Ar-172 | C-1193 | " | " | " | Ar-172 |
| C-414 | " | " | Ar-4 | Ar-4 | C-1194 | " | " | Ar-4 | Ar-4 |
| C-415 | " | " | " | Ar-5 | C-1195 | " | " | " | Ar-5 |
| C-416 | " | " | " | Ar-74 | C-1196 | " | " | " | Ar-74 |
| C-417 | " | " | " | Ar-78 | C-1197 | " | " | " | Ar-78 |
| C-418 | " | " | " | Ar-82 | C-1198 | " | " | " | Ar-82 |
| C-419 | " | " | " | Ar-117 | C-1199 | " | " | " | Ar-117 |
| C-420 | " | " | " | Ar-134 | C-1200 | " | " | " | Ar-134 |
| C-421 | " | " | " | Ar-139 | C-1201 | " | " | " | Ar-139 |
| C-422 | " | " | " | Ar-150 | C-1202 | " | " | " | Ar-150 |
| C-423 | " | " | " | Ar-172 | C-1203 | " | " | " | Ar-172 |
| C-424 | " | " | Ar-5 | Ar-5 | C-1204 | " | " | Ar-5 | Ar-5 |
| C-425 | " | " | " | Ar-74 | C-1205 | " | " | " | Ar-74 |
| C-426 | " | " | " | Ar-78 | C-1206 | " | " | " | Ar-78 |
| C-427 | " | " | " | Ar-82 | C-1207 | " | " | " | Ar-82 |
| C-428 | " | " | " | Ar-117 | C-1208 | " | " | " | Ar-117 |
| C-429 | " | " | " | Ar-134 | C-1209 | " | " | " | Ar-134 |
| C-430 | " | " | " | Ar-139 | C-1210 | " | " | " | Ar-139 |
| C-431 | " | " | " | Ar-150 | C-1211 | " | " | " | Ar-150 |
| C-432 | " | " | " | Ar-172 | C-1212 | " | " | " | Ar-172 |
| C-433 | " | " | Ar-74 | Ar-74 | C-1213 | " | " | Ar-74 | Ar-74 |
| C-434 | " | " | " | Ar-78 | C-1214 | " | " | " | Ar-78 |
| C-435 | " | " | " | Ar-82 | C-1215 | " | " | " | Ar-82 |
| C-436 | " | " | " | Ar-117 | C-1216 | " | " | " | Ar-117 |
| C-437 | " | " | " | Ar-134 | C-1217 | " | " | " | Ar-134 |
| C-438 | " | " | " | Ar-139 | C-1218 | " | " | " | Ar-139 |
| C-439 | " | " | " | Ar-150 | C-1219 | " | " | " | Ar-150 |
| C-440 | " | " | " | Ar-172 | C-1220 | " | " | " | Ar-172 |
| C-441 | " | " | Ar-78 | Ar-78 | C-1221 | " | " | Ar-78 | Ar-78 |
| C-442 | " | " | " | Ar-82 | C-1222 | " | " | " | Ar-82 |
| C-443 | " | " | " | Ar-117 | C-1223 | " | " | " | Ar-117 |
| C-444 | " | " | " | Ar-134 | C-1224 | " | " | " | Ar-134 |
| C-445 | " | " | " | Ar-139 | C-1225 | " | " | " | Ar-139 |
| C-446 | " | " | " | Ar-150 | C-1226 | " | " | " | Ar-150 |
| C-447 | " | " | " | Ar-172 | C-1227 | " | " | " | Ar-172 |
| C-448 | " | " | Ar-82 | Ar-82 | C-1228 | " | " | Ar-82 | Ar-82 |
| C-449 | " | " | " | Ar-117 | C-1229 | " | " | " | Ar-117 |
| C-450 | " | " | " | Ar-134 | C-1230 | " | " | " | Ar-134 |
| C-451 | " | " | " | Ar-139 | C-1231 | " | " | " | Ar-139 |
| C-452 | " | " | " | Ar-150 | C-1232 | " | " | " | Ar-150 |
| C-453 | " | " | " | Ar-172 | C-1233 | " | " | " | Ar-172 |
| C-454 | " | " | Ar-117 | Ar-117 | C-1234 | " | " | Ar-117 | Ar-117 |
| C-455 | " | " | " | Ar-134 | C-1235 | " | " | " | Ar-134 |
| C-456 | " | " | " | Ar-139 | C-1236 | " | " | " | Ar-139 |
| C-457 | " | " | " | Ar-150 | C-1237 | " | " | " | Ar-150 |
| C-458 | " | " | " | Ar-172 | C-1238 | " | " | " | Ar-172 |
| C-459 | " | " | Ar-134 | Ar-134 | C-1239 | " | " | Ar-134 | Ar-134 |
| C-460 | " | " | " | Ar-139 | C-1240 | " | " | " | Ar-139 |
| C-461 | " | " | " | Ar-150 | C-1241 | " | " | " | Ar-150 |
| C-462 | " | " | " | Ar-172 | C-1242 | " | " | " | Ar-172 |
| C-463 | " | " | Ar-139 | Ar-139 | C-1243 | " | " | Ar-139 | Ar-139 |
| C-464 | " | " | " | Ar-150 | C-1244 | " | " | " | Ar-150 |
| C-465 | " | " | " | Ar-172 | C-1245 | " | " | " | Ar-172 |
| C-466 | " | " | Ar-150 | Ar-150 | C-1246 | " | " | Ar-150 | Ar-150 |
| C-467 | " | " | " | Ar-172 | C-1247 | " | " | " | Ar-172 |
| C-468 | " | " | Ar-172 | Ar-172 | C-1248 | " | " | Ar-172 | Ar-172 |
| C-469 | " | Methyl | Ar-1 | Ar-1 | C-1249 | R-58 | H | Ar-1 | Ar-1 |
| C-470 | " | " | " | Ar-2 | C-1250 | " | " | " | Ar-2 |
| C-471 | " | " | " | Ar-4 | C-1251 | " | " | " | Ar-4 |
| C-472 | " | " | " | Ar-5 | C-1252 | " | " | " | Ar-5 |
| C-473 | " | " | " | Ar-74 | C-1253 | " | " | " | Ar-74 |
| C-474 | " | " | " | Ar-78 | C-1254 | " | " | " | Ar-78 |
| C-475 | " | " | " | Ar-82 | C-1255 | " | " | " | Ar-82 |
| C-476 | " | " | " | Ar-117 | C-1256 | " | " | " | Ar-117 |
| C-477 | " | " | " | Ar-134 | C-1257 | " | " | " | Ar-134 |
| C-478 | " | " | " | Ar-139 | C-1258 | " | " | " | Ar-139 |
| C-479 | " | " | " | Ar-150 | C-1259 | " | " | " | Ar-150 |
| C-480 | " | " | " | Ar-172 | C-1260 | " | " | " | Ar-172 |
| C-481 | " | " | Ar-2 | Ar-2 | C-1261 | " | " | Ar-2 | Ar-2 |
| C-482 | " | " | " | Ar-4 | C-1262 | " | " | " | Ar-4 |
| C-483 | " | " | " | Ar-5 | C-1263 | " | " | " | Ar-5 |
| C-484 | " | " | " | Ar-74 | C-1264 | " | " | " | Ar-74 |
| C-485 | " | " | " | Ar-78 | C-1265 | " | " | " | Ar-78 |
| C-486 | " | " | " | Ar-82 | C-1266 | " | " | " | Ar-82 |
| C-487 | " | " | " | Ar-117 | C-1267 | " | " | " | Ar-117 |
| C-488 | " | " | " | Ar-134 | C-1268 | " | " | " | Ar-134 |
| C-489 | " | " | " | Ar-139 | C-1269 | " | " | " | Ar-139 |
| C-490 | " | " | " | Ar-150 | C-1270 | " | " | " | Ar-150 |
| C-491 | " | " | " | Ar-172 | C-1271 | " | " | " | Ar-172 |
| C-492 | " | " | Ar-4 | Ar-4 | C-1272 | " | " | Ar-4 | Ar-4 |
| C-493 | " | " | " | Ar-5 | C-1273 | " | " | " | Ar-5 |
| C-494 | " | " | " | Ar-74 | C-1274 | " | " | " | Ar-74 |
| C-495 | " | " | " | Ar-78 | C-1275 | " | " | " | Ar-78 |
| C-496 | " | " | " | Ar-82 | C-1276 | " | " | " | Ar-82 |
| C-497 | " | " | " | Ar-117 | C-1277 | " | " | " | Ar-117 |
| C-498 | " | " | " | Ar-134 | C-1278 | " | " | " | Ar-134 |
| C-499 | " | " | " | Ar-139 | C-1279 | " | " | " | Ar-139 |
| C-500 | " | " | " | Ar-150 | C-1280 | " | " | " | Ar-150 |
| C-501 | " | " | " | Ar-172 | C-1281 | " | " | " | Ar-172 |
| C-502 | " | " | Ar-5 | Ar-5 | C-1282 | " | " | Ar-5 | Ar-5 |
| C-503 | " | " | " | Ar-74 | C-1283 | " | " | " | Ar-74 |
| C-504 | " | " | " | Ar-78 | C-1284 | " | " | " | Ar-78 |
| C-505 | " | " | " | Ar-82 | C-1285 | " | " | " | Ar-82 |
| C-506 | " | " | " | Ar-117 | C-1286 | " | " | " | Ar-117 |
| C-507 | " | " | " | Ar-134 | C-1287 | " | " | " | Ar-134 |
| C-508 | " | " | " | Ar-139 | C-1288 | " | " | " | Ar-139 |
| C-509 | " | " | " | Ar-150 | C-1289 | " | " | " | Ar-150 |
| C-510 | " | " | " | Ar-172 | C-1290 | " | " | " | Ar-172 |
| C-511 | " | " | Ar-74 | Ar-74 | C-1291 | " | " | Ar-74 | Ar-74 |
| C-512 | " | " | " | Ar-78 | C-1292 | " | " | " | Ar-78 |
| C-513 | " | " | " | Ar-82 | C-1293 | " | " | " | Ar-82 |
| C-514 | " | " | " | Ar-117 | C-1294 | " | " | " | Ar-117 |
| C-515 | " | " | " | Ar-134 | C-1295 | " | " | " | Ar-134 |
| C-516 | " | " | " | Ar-139 | C-1296 | " | " | " | Ar-139 |
| C-517 | " | " | " | Ar-150 | C-1297 | " | " | " | Ar-150 |
| C-518 | " | " | " | Ar-172 | C-1298 | " | " | " | Ar-172 |
| C-519 | " | " | Ar-78 | Ar-78 | C-1299 | " | " | Ar-78 | Ar-78 |
| C-520 | " | " | " | Ar-82 | C-1300 | " | " | " | Ar-82 |
| C-521 | " | " | " | Ar-117 | C-1301 | " | " | " | Ar-117 |
| C-522 | " | " | " | Ar-134 | C-1302 | " | " | " | Ar-134 |
| C-523 | " | " | " | Ar-139 | C-1303 | " | " | " | Ar-139 |
| C-524 | " | " | " | Ar-150 | C-1304 | " | " | " | Ar-150 |
| C-525 | " | " | " | Ar-172 | C-1305 | " | " | " | Ar-172 |
| C-526 | " | " | Ar-82 | Ar-82 | C-1306 | " | " | Ar-82 | Ar-82 |
| C-527 | " | " | " | Ar-117 | C-1307 | " | " | " | Ar-117 |
| C-528 | " | " | " | Ar-134 | C-1308 | " | " | " | Ar-134 |
| C-529 | " | " | " | Ar-139 | C-1309 | " | " | " | Ar-139 |
| C-530 | " | " | " | Ar-150 | C-1310 | " | " | " | Ar-150 |
| C-531 | " | " | " | Ar-172 | C-1311 | " | " | " | Ar-172 |
| C-532 | " | " | Ar-117 | Ar-117 | C-1312 | " | " | Ar-117 | Ar-117 |
| C-533 | " | " | " | Ar-134 | C-1313 | " | " | " | Ar-134 |
| C-534 | " | " | " | Ar-139 | C-1314 | " | " | " | Ar-139 |
| C-535 | " | " | " | Ar-150 | C-1315 | " | " | " | Ar-150 |
| C-536 | " | " | " | Ar-172 | C-1316 | " | " | " | Ar-172 |
| C-537 | " | " | Ar-134 | Ar-134 | C-1317 | " | " | Ar-134 | Ar-134 |
| C-538 | " | " | " | Ar-139 | C-1318 | " | " | " | Ar-139 |
| C-539 | " | " | " | Ar-150 | C-1319 | " | " | " | Ar-150 |
| C-540 | " | " | " | Ar-172 | C-1320 | " | " | " | Ar-172 |
| C-541 | " | " | Ar-139 | Ar-139 | C-1321 | " | " | Ar-139 | Ar-139 |
| C-542 | " | " | " | Ar-150 | C-1322 | " | " | " | Ar-150 |
| C-543 | " | " | " | Ar-172 | C-1323 | " | " | " | Ar-172 |
| C-544 | " | " | Ar-150 | Ar-150 | C-1324 | " | " | Ar-150 | Ar-150 |
| C-545 | " | " | " | Ar-172 | C-1325 | " | " | " | Ar-172 |
| C-546 | " | " | Ar-172 | Ar-172 | C-1326 | " | " | Ar-172 | Ar-172 |
| C-547 | " | tert-Butyl | Ar-1 | Ar-1 | C-1327 | " | F | Ar-1 | Ar-1 |
| C-548 | " | " | " | Ar-2 | C-1328 | " | " | " | Ar-2 |
| C-549 | " | " | " | Ar-4 | C-1329 | " | " | " | Ar-4 |
| C-550 | " | " | " | Ar-5 | C-1330 | " | " | " | Ar-5 |
| C-551 | " | " | " | Ar-74 | C-1331 | " | " | " | Ar-74 |
| C-552 | " | " | " | Ar-78 | C-1332 | " | " | " | Ar-78 |
| C-553 | " | " | " | Ar-82 | C-1333 | " | " | " | Ar-82 |
| C-554 | " | " | " | Ar-117 | C-1334 | " | " | " | Ar-117 |
| C-555 | " | " | " | Ar-134 | C-1335 | " | " | " | Ar-134 |
| C-556 | " | " | " | Ar-139 | C-1336 | " | " | " | Ar-139 |
| C-557 | " | " | " | Ar-150 | C-1337 | " | " | " | Ar-150 |
| C-558 | " | " | " | Ar-172 | C-1338 | " | " | " | Ar-172 |
| C-559 | " | " | Ar-2 | Ar-2 | C-1339 | " | " | Ar-2 | Ar-2 |
| C-560 | " | " | " | Ar-4 | C-1340 | " | " | " | Ar-4 |
| C-561 | " | " | " | Ar-5 | C-1341 | " | " | " | Ar-5 |
| C-562 | " | " | " | Ar-74 | C-1342 | " | " | " | Ar-74 |
| C-563 | " | " | " | Ar-78 | C-1343 | " | " | " | Ar-78 |
| C-564 | " | " | " | Ar-82 | C-1344 | " | " | " | Ar-82 |
| C-565 | " | " | " | Ar-117 | C-1345 | " | " | " | Ar-117 |
| C-566 | " | " | " | Ar-134 | C-1346 | " | " | " | Ar-134 |
| C-567 | " | " | " | Ar-139 | C-1347 | " | " | " | Ar-139 |
| C-568 | " | " | " | Ar-150 | C-1348 | " | " | " | Ar-150 |
| C-569 | " | " | " | Ar-172 | C-1349 | " | " | " | Ar-172 |
| C-570 | " | " | Ar-4 | Ar-4 | C-1350 | " | " | Ar-4 | Ar-4 |
| C-571 | " | " | " | Ar-5 | C-1351 | " | " | " | Ar-5 |
| C-572 | " | " | " | Ar-74 | C-1352 | " | " | " | Ar-74 |
| C-573 | " | " | " | Ar-78 | C-1353 | " | " | " | Ar-78 |
| C-574 | " | " | " | Ar-82 | C-1354 | " | " | " | Ar-82 |
| C-575 | " | " | " | Ar-117 | C-1355 | " | " | " | Ar-117 |
| C-576 | " | " | " | Ar-134 | C-1356 | " | " | " | Ar-134 |
| C-577 | " | " | " | Ar-139 | C-1357 | " | " | " | Ar-139 |
| C-578 | " | " | " | Ar-150 | C-1358 | " | " | " | Ar-150 |
| C-579 | " | " | " | Ar-172 | C-1359 | " | " | " | Ar-172 |
| C-580 | " | " | Ar-5 | Ar-5 | C-1360 | " | " | Ar-5 | Ar-5 |
| C-581 | " | " | " | Ar-74 | C-1361 | " | " | " | Ar-74 |
| C-582 | " | " | " | Ar-78 | C-1362 | " | " | " | Ar-78 |
| C-583 | " | " | " | Ar-82 | C-1363 | " | " | " | Ar-82 |
| C-584 | " | " | " | Ar-117 | C-1364 | " | " | " | Ar-117 |
| C-585 | " | " | " | Ar-134 | C-1365 | " | " | " | Ar-134 |
| C-586 | " | " | " | Ar-139 | C-1366 | " | " | " | Ar-139 |
| C-587 | " | " | " | Ar-150 | C-1367 | " | " | " | Ar-150 |
| C-588 | " | " | " | Ar-172 | C-1368 | " | " | " | Ar-172 |
| C-589 | " | " | Ar-74 | Ar-74 | C-1369 | " | " | Ar-74 | Ar-74 |
| C-590 | " | " | " | Ar-78 | C-1370 | " | " | " | Ar-78 |
| C-591 | " | " | " | Ar-82 | C-1371 | " | " | " | Ar-82 |
| C-592 | " | " | " | Ar-117 | C-1372 | " | " | " | Ar-117 |
| C-593 | " | " | " | Ar-134 | C-1373 | " | " | " | Ar-134 |
| C-594 | " | " | " | Ar-139 | C-1374 | " | " | " | Ar-139 |
| C-595 | " | " | " | Ar-150 | C-1375 | " | " | " | Ar-150 |
| C-596 | " | " | " | Ar-172 | C-1376 | " | " | " | Ar-172 |
| C-597 | " | " | Ar-78 | Ar-78 | C-1377 | " | " | Ar-78 | Ar-78 |
| C-598 | " | " | " | Ar-82 | C-1378 | " | " | " | Ar-82 |
| C-599 | " | " | " | Ar-117 | C-1379 | " | " | " | Ar-117 |
| C-600 | " | " | " | Ar-134 | C-1380 | " | " | " | Ar-134 |
| C-601 | " | " | " | Ar-139 | C-1381 | " | " | " | Ar-139 |
| C-602 | " | " | " | Ar-150 | C-1382 | " | " | " | Ar-150 |
| C-603 | " | " | " | Ar-172 | C-1383 | " | " | " | Ar-172 |
| C-604 | " | " | Ar-82 | Ar-82 | C-1384 | " | " | Ar-82 | Ar-82 |
| C-605 | " | " | " | Ar-117 | C-1385 | " | " | " | Ar-117 |
| C-606 | " | " | " | Ar-134 | C-1386 | " | " | " | Ar-134 |
| C-607 | " | " | " | Ar-139 | C-1387 | " | " | " | Ar-139 |
| C-608 | " | " | " | Ar-150 | C-1388 | " | " | " | Ar-150 |
| C-609 | " | " | " | Ar-172 | C-1389 | " | " | " | Ar-172 |
| C-610 | " | " | Ar-117 | Ar-117 | C-1390 | " | " | Ar-117 | Ar-117 |
| C-611 | " | " | " | Ar-134 | C-1391 | " | " | " | Ar-134 |
| C-612 | " | " | " | Ar-139 | C-1392 | " | " | " | Ar-139 |
| C-613 | " | " | " | Ar-150 | C-1393 | " | " | " | Ar-150 |
| C-614 | " | " | " | Ar-172 | C-1394 | " | " | " | Ar-172 |
| C-615 | " | " | Ar-134 | Ar-134 | C-1395 | " | " | Ar-134 | Ar-134 |
| C-616 | " | " | " | Ar-139 | C-1396 | " | " | " | Ar-139 |
| C-617 | " | " | " | Ar-150 | C-1397 | " | " | " | Ar-150 |
| C-618 | " | " | " | Ar-172 | C-1398 | " | " | " | Ar-172 |
| C-619 | " | " | Ar-139 | Ar-139 | C-1399 | " | " | Ar-139 | Ar-139 |
| C-620 | " | " | " | Ar-150 | C-1400 | " | " | " | Ar-150 |
| C-621 | " | " | " | Ar-172 | C-1401 | " | " | " | Ar-172 |
| C-622 | " | " | Ar-150 | Ar-150 | C-1402 | " | " | Ar-150 | Ar-150 |
| C-623 | " | " | " | Ar-172 | C-1403 | " | " | " | Ar-172 |
| C-624 | " | " | Ar-172 | Ar-172 | C-1404 | " | " | Ar-172 | Ar-172 |
| C-625 | R-2 | H | Ar-1 | Ar-1 | C-1405 | " | Methyl | Ar-1 | Ar-1 |
| C-626 | " | " | " | Ar-2 | C-1406 | " | " | " | Ar-2 |
| C-627 | " | " | " | Ar-4 | C-1407 | " | " | " | Ar-4 |
| C-628 | " | " | " | Ar-5 | C-1408 | " | " | " | Ar-5 |
| C-629 | " | " | " | Ar-74 | C-1409 | " | " | " | Ar-74 |
| C-630 | " | " | " | Ar-78 | C-1410 | " | " | " | Ar-78 |
| C-631 | " | " | " | Ar-82 | C-1411 | " | " | " | Ar-82 |
| C-632 | " | " | " | Ar-117 | C-1412 | " | " | " | Ar-117 |
| C-633 | " | " | " | Ar-134 | C-1413 | " | " | " | Ar-134 |
| C-634 | " | " | " | Ar-139 | C-1414 | " | " | " | Ar-139 |
| C-635 | " | " | " | Ar-150 | C-1415 | " | " | " | Ar-150 |
| C-636 | " | " | " | Ar-172 | C-1416 | " | " | " | Ar-172 |
| C-637 | " | " | Ar-2 | Ar-2 | C-1417 | " | " | Ar-2 | Ar-2 |
| C-638 | " | " | " | Ar-4 | C-1418 | " | " | " | Ar-4 |
| C-639 | " | " | " | Ar-5 | C-1419 | " | " | " | Ar-5 |
| C-640 | " | " | " | Ar-74 | C-1420 | " | " | " | Ar-74 |
| C-641 | " | " | " | Ar-78 | C-1421 | " | " | " | Ar-78 |
| C-642 | " | " | " | Ar-82 | C-1422 | " | " | " | Ar-82 |
| C-643 | " | " | " | Ar-117 | C-1423 | " | " | " | Ar-117 |
| C-644 | " | " | " | Ar-134 | C-1424 | " | " | " | Ar-134 |
| C-645 | " | " | " | Ar-139 | C-1425 | " | " | " | Ar-139 |
| C-646 | " | " | " | Ar-150 | C-1426 | " | " | " | Ar-150 |
| C-647 | " | " | " | Ar-172 | C-1427 | " | " | " | Ar-172 |
| C-648 | " | " | Ar-4 | Ar-4 | C-1428 | " | " | Ar-4 | Ar-4 |
| C-649 | " | " | " | Ar-5 | C-1429 | " | " | " | Ar-5 |
| C-650 | " | " | " | Ar-74 | C-1430 | " | " | " | Ar-74 |
| C-651 | " | " | " | Ar-78 | C-1431 | " | " | " | Ar-78 |
| C-652 | " | " | " | Ar-82 | C-1432 | " | " | " | Ar-82 |
| C-653 | " | " | " | Ar-117 | C-1433 | " | " | " | Ar-117 |
| C-654 | " | " | " | Ar-134 | C-1434 | " | " | " | Ar-134 |
| C-655 | " | " | " | Ar-139 | C-1435 | " | " | " | Ar-139 |
| C-656 | " | " | " | Ar-150 | C-1436 | " | " | " | Ar-150 |
| C-657 | " | " | " | Ar-172 | C-1437 | " | " | " | Ar-172 |
| C-658 | " | " | Ar-5 | Ar-5 | C-1438 | " | " | Ar-5 | Ar-5 |
| C-659 | " | " | " | Ar-74 | C-1439 | " | " | " | Ar-74 |
| C-660 | " | " | " | Ar-78 | C-1440 | " | " | " | Ar-78 |
| C-661 | " | " | " | Ar-82 | C-1441 | " | " | " | Ar-82 |
| C-662 | " | " | " | Ar-117 | C-1442 | " | " | " | Ar-117 |
| C-663 | " | " | " | Ar-134 | C-1443 | " | " | " | Ar-134 |
| C-664 | " | " | " | Ar-139 | C-1444 | " | " | " | Ar-139 |
| C-665 | " | " | " | Ar-150 | C-1445 | " | " | " | Ar-150 |
| C-666 | " | " | " | Ar-172 | C-1446 | " | " | " | Ar-172 |
| C-667 | " | " | Ar-74 | Ar-74 | C-1447 | " | " | Ar-74 | Ar-74 |
| C-668 | " | " | " | Ar-78 | C-1448 | " | " | " | Ar-78 |
| C-669 | " | " | " | Ar-82 | C-1449 | " | " | " | Ar-82 |
| C-670 | " | " | " | Ar-117 | C-1450 | " | " | " | Ar-117 |
| C-671 | " | " | " | Ar-134 | C-1451 | " | " | " | Ar-134 |
| C-672 | " | " | " | Ar-139 | C-1452 | " | " | " | Ar-139 |
| C-673 | " | " | " | Ar-150 | C-1453 | " | " | " | Ar-150 |
| C-674 | " | " | " | Ar-172 | C-1454 | " | " | " | Ar-172 |
| C-675 | " | " | Ar-78 | Ar-78 | C-1455 | " | " | Ar-78 | Ar-78 |
| C-676 | " | " | " | Ar-82 | C-1456 | " | " | " | Ar-82 |
| C-677 | " | " | " | Ar-117 | C-1457 | " | " | " | Ar-117 |
| C-678 | " | " | " | Ar-134 | C-1458 | " | " | " | Ar-134 |
| C-679 | " | " | " | Ar-139 | C-1459 | " | " | " | Ar-139 |
| C-680 | " | " | " | Ar-150 | C-1460 | " | " | " | Ar-150 |
| C-681 | " | " | " | Ar-172 | C-1461 | " | " | " | Ar-172 |
| C-682 | " | " | Ar-82 | Ar-82 | C-1462 | " | " | Ar-82 | Ar-82 |
| C-683 | " | " | " | Ar-117 | C-1463 | " | " | " | Ar-117 |
| C-684 | " | " | " | Ar-134 | C-1464 | " | " | " | Ar-134 |
| C-685 | " | " | " | Ar-139 | C-1465 | " | " | " | Ar-139 |
| C-686 | " | " | " | Ar-150 | C-1466 | " | " | " | Ar-150 |
| C-687 | " | " | " | Ar-172 | C-1467 | " | " | " | Ar-172 |
| C-688 | " | " | Ar-117 | Ar-117 | C-1468 | " | " | Ar-117 | Ar-117 |
| C-689 | " | " | " | Ar-134 | C-1469 | " | " | " | Ar-134 |
| C-690 | " | " | " | Ar-139 | C-1470 | " | " | " | Ar-139 |
| C-691 | " | " | " | Ar-150 | C-1471 | " | " | " | Ar-150 |
| C-692 | " | " | " | Ar-172 | C-1472 | " | " | " | Ar-172 |
| C-693 | " | " | Ar-134 | Ar-134 | C-1473 | " | " | Ar-134 | Ar-134 |
| C-694 | " | " | " | Ar-139 | C-1474 | " | " | " | Ar-139 |
| C-695 | " | " | " | Ar-150 | C-1475 | " | " | " | Ar-150 |
| C-696 | " | " | " | Ar-172 | C-1476 | " | " | " | Ar-172 |
| C-697 | " | " | Ar-139 | Ar-139 | C-1477 | " | " | Ar-139 | Ar-139 |
| C-698 | " | " | " | Ar-150 | C-1478 | " | " | " | Ar-150 |
| C-699 | " | " | " | Ar-172 | C-1479 | " | " | " | Ar-172 |
| C-700 | " | " | Ar-150 | Ar-150 | C-1480 | " | " | Ar-150 | Ar-150 |
| C-701 | " | " | " | Ar-172 | C-1481 | " | " | " | Ar-172 |
| C-702 | " | " | Ar-172 | Ar-172 | C-1482 | " | " | Ar-172 | Ar-172 |
| C-703 | " | F | Ar-1 | Ar-1 | C-1483 | " | tert-Butyl | Ar-1 | Ar-1 |
| C-704 | " | " | " | Ar-2 | C-1484 | " | " | " | Ar-2 |
| C-705 | " | " | " | Ar-4 | C-1485 | " | " | " | Ar-4 |
| C-706 | " | " | " | Ar-5 | C-1486 | " | " | " | Ar-5 |
| C-707 | " | " | " | Ar-74 | C-1487 | " | " | " | Ar-74 |
| C-708 | " | " | " | Ar-78 | C-1488 | " | " | " | Ar-78 |
| C-709 | " | " | " | Ar-82 | C-1489 | " | " | " | Ar-82 |
| C-710 | " | " | " | Ar-117 | C-1490 | " | " | " | Ar-117 |
| C-711 | " | " | " | Ar-134 | C-1491 | " | " | " | Ar-134 |
| C-712 | " | " | " | Ar-139 | C-1492 | " | " | " | Ar-139 |
| C-713 | " | " | " | Ar-150 | C-1493 | " | " | " | Ar-150 |
| C-714 | " | " | " | Ar-172 | C-1494 | " | " | " | Ar-172 |
| C-715 | " | " | Ar-2 | Ar-2 | C-1495 | " | " | Ar-2 | Ar-2 |
| C-716 | " | " | " | Ar-4 | C-1496 | " | " | " | Ar-4 |
| C-717 | " | " | " | Ar-5 | C-1497 | " | " | " | Ar-5 |
| C-718 | " | " | " | Ar-74 | C-1498 | " | " | " | Ar-74 |
| C-719 | " | " | " | Ar-78 | C-1499 | " | " | " | Ar-78 |
| C-720 | " | " | " | Ar-82 | C-1500 | " | " | " | Ar-82 |
| C-721 | " | " | " | Ar-117 | C-1501 | " | " | " | Ar-117 |
| C-722 | " | " | " | Ar-134 | C-1502 | " | " | " | Ar-134 |
| C-723 | " | " | " | Ar-139 | C-1503 | " | " | " | Ar-139 |
| C-724 | " | " | " | Ar-150 | C-1504 | " | " | " | Ar-150 |
| C-725 | " | " | " | Ar-172 | C-1505 | " | " | " | Ar-172 |
| C-726 | " | " | Ar-4 | Ar-4 | C-1506 | " | " | Ar-4 | Ar-4 |
| C-727 | " | " | " | Ar-5 | C-1507 | " | " | " | Ar-5 |
| C-728 | " | " | " | Ar-74 | C-1508 | " | " | " | Ar-74 |
| C-729 | " | " | " | Ar-78 | C-1509 | " | " | " | Ar-78 |
| C-730 | " | " | " | Ar-82 | C-1510 | " | " | " | Ar-82 |
| C-731 | " | " | " | Ar-117 | C-1511 | " | " | " | Ar-117 |
| C-732 | " | " | " | Ar-134 | C-1512 | " | " | " | Ar-134 |
| C-733 | " | " | " | Ar-139 | C-1513 | " | " | " | Ar-139 |
| C-734 | " | " | " | Ar-150 | C-1514 | " | " | " | Ar-150 |
| C-735 | " | " | " | Ar-172 | C-1515 | " | " | " | Ar-172 |
| C-736 | " | " | Ar-5 | Ar-5 | C-1516 | " | " | Ar-5 | Ar-5 |
| C-737 | " | " | " | Ar-74 | C-1517 | " | " | " | Ar-74 |
| C-738 | " | " | " | Ar-78 | C-1518 | " | " | " | Ar-78 |
| C-739 | " | " | " | Ar-82 | C-1519 | " | " | " | Ar-82 |
| C-740 | " | " | " | Ar-117 | C-1520 | " | " | " | Ar-117 |
| C-741 | " | " | " | Ar-134 | C-1521 | " | " | " | Ar-134 |
| C-742 | " | " | " | Ar-139 | C-1522 | " | " | " | Ar-139 |
| C-743 | " | " | " | Ar-150 | C-1523 | " | " | " | Ar-150 |
| C-744 | " | " | " | Ar-172 | C-1524 | " | " | " | Ar-172 |
| C-745 | " | " | Ar-74 | Ar-74 | C-1525 | " | " | Ar-74 | Ar-74 |
| C-746 | " | " | " | Ar-78 | C-1526 | " | " | " | Ar-78 |
| C-747 | " | " | " | Ar-82 | C-1527 | " | " | " | Ar-82 |
| C-748 | " | " | " | Ar-117 | C-1528 | " | " | " | Ar-117 |
| C-749 | " | " | " | Ar-134 | C-1529 | " | " | " | Ar-134 |
| C-750 | " | " | " | Ar-139 | C-1530 | " | " | " | Ar-139 |
| C-751 | " | " | " | Ar-150 | C-1531 | " | " | " | Ar-150 |
| C-752 | " | " | " | Ar-172 | C-1532 | " | " | " | Ar-172 |
| C-753 | " | " | Ar-78 | Ar-78 | C-1533 | " | " | Ar-78 | Ar-78 |
| C-754 | " | " | " | Ar-82 | C-1534 | " | " | " | Ar-82 |
| C-755 | " | " | " | Ar-117 | C-1535 | " | " | " | Ar-117 |
| C-756 | " | " | " | Ar-134 | C-1536 | " | " | " | Ar-134 |
| C-757 | " | " | " | Ar-139 | C-1537 | " | " | " | Ar-139 |
| C-758 | " | " | " | Ar-150 | C-1538 | " | " | " | Ar-150 |
| C-759 | " | " | " | Ar-172 | C-1539 | " | " | " | Ar-172 |
| C-760 | " | " | Ar-82 | Ar-82 | C-1540 | " | " | Ar-82 | Ar-82 |
| C-761 | " | " | " | Ar-117 | C-1541 | " | " | " | Ar-117 |
| C-762 | " | " | " | Ar-134 | C-1542 | " | " | " | Ar-134 |
| C-763 | " | " | " | Ar-139 | C-1543 | " | " | " | Ar-139 |
| C-764 | " | " | " | Ar-150 | C-1544 | " | " | " | Ar-150 |
| C-765 | " | " | " | Ar-172 | C-1545 | " | " | " | Ar-172 |
| C-766 | " | " | Ar-117 | Ar-117 | C-1546 | " | " | Ar-117 | Ar-117 |
| C-767 | " | " | " | Ar-134 | C-1547 | " | " | " | Ar-134 |
| C-768 | " | " | " | Ar-139 | C-1548 | " | " | " | Ar-139 |
| C-769 | " | " | " | Ar-150 | C-1549 | " | " | " | Ar-150 |
| C-770 | " | " | " | Ar-172 | C-1550 | " | " | " | Ar-172 |
| C-771 | " | " | Ar-134 | Ar-134 | C-1551 | " | " | Ar-134 | Ar-134 |
| C-772 | " | " | " | Ar-139 | C-1552 | " | " | " | Ar-139 |
| C-773 | " | " | " | Ar-150 | C-1553 | " | " | " | Ar-150 |
| C-774 | " | " | " | Ar-172 | C-1554 | " | " | " | Ar-172 |
| C-775 | " | " | Ar-139 | Ar-139 | C-1555 | " | " | Ar-139 | Ar-139 |
| C-776 | " | " | " | Ar-150 | C-1556 | " | " | " | Ar-150 |
| C-777 | " | " | " | Ar-172 | C-1557 | " | " | " | Ar-172 |
| C-778 | " | " | Ar-150 | Ar-150 | C-1558 | " | " | Ar-150 | Ar-150 |
| C-779 | " | " | " | Ar-172 | C-1559 | " | " | " | Ar-172 |
| C-780 | " | " | Ar-172 | Ar-172 | C-1560 | " | " | Ar-172 | Ar-172 |
| C-781 | " | Methyl | Ar-1 | Ar-1 | C-1561 | R-66 | H | Ar-1 | Ar-1 |
| C-782 | " | " | " | Ar-2 | C-1562 | " | " | " | Ar-2 |
| C-783 | " | " | " | Ar-4 | C-1563 | " | " | " | Ar-4 |
| C-784 | " | " | " | Ar-5 | C-1564 | " | " | " | Ar-5 |
| C-785 | " | " | " | Ar-74 | C-1565 | " | " | " | Ar-74 |
| C-786 | " | " | " | Ar-78 | C-1566 | " | " | " | Ar-78 |
| C-787 | " | " | " | Ar-82 | C-1567 | " | " | " | Ar-82 |
| C-788 | " | " | " | Ar-117 | C-1568 | " | " | " | Ar-117 |
| C-789 | " | " | " | Ar-134 | C-1569 | " | " | " | Ar-134 |
| C-790 | " | " | " | Ar-139 | C-1570 | " | " | " | Ar-139 |
| C-791 | " | " | " | Ar-150 | C-1571 | " | " | " | Ar-150 |
| C-792 | " | " | " | Ar-172 | C-1572 | " | " | " | Ar-172 |
| C-793 | " | " | Ar-2 | Ar-2 | C-1573 | " | " | Ar-2 | Ar-2 |
| C-794 | " | " | " | Ar-4 | C-1574 | " | " | " | Ar-4 |
| C-795 | " | " | " | Ar-5 | C-1575 | " | " | " | Ar-5 |
| C-796 | " | " | " | Ar-74 | C-1576 | " | " | " | Ar-74 |
| C-797 | " | " | " | Ar-78 | C-1577 | " | " | " | Ar-78 |
| C-798 | " | " | " | Ar-82 | C-1578 | " | " | " | Ar-82 |
| C-799 | " | " | " | Ar-117 | C-1579 | " | " | " | Ar-117 |
| C-800 | " | " | " | Ar-134 | C-1580 | " | " | " | Ar-134 |
| C-801 | " | " | " | Ar-139 | C-1581 | " | " | " | Ar-139 |
| C-802 | " | " | " | Ar-150 | C-1582 | " | " | " | Ar-150 |
| C-803 | " | " | " | Ar-172 | C-1583 | " | " | " | Ar-172 |
| C-804 | " | " | Ar-4 | Ar-4 | C-1584 | " | " | Ar-4 | Ar-4 |
| C-805 | " | " | " | Ar-5 | C-1585 | " | " | " | Ar-5 |
| C-806 | " | " | " | Ar-74 | C-1586 | " | " | " | Ar-74 |
| C-807 | " | " | " | Ar-78 | C-1587 | " | " | " | Ar-78 |
| C-808 | " | " | " | Ar-82 | C-1588 | " | " | " | Ar-82 |
| C-809 | " | " | " | Ar-117 | C-1589 | " | " | " | Ar-117 |
| C-810 | " | " | " | Ar-134 | C-1590 | " | " | " | Ar-134 |
| C-811 | " | " | " | Ar-139 | C-1591 | " | " | " | Ar-139 |
| C-812 | " | " | " | Ar-150 | C-1592 | " | " | " | Ar-150 |
| C-813 | " | " | " | Ar-172 | C-1593 | " | " | " | Ar-172 |
| C-814 | " | " | Ar-5 | Ar-5 | C-1594 | " | " | Ar-5 | Ar-5 |
| C-815 | " | " | " | Ar-74 | C-1595 | " | " | " | Ar-74 |
| C-816 | " | " | " | Ar-78 | C-1596 | " | " | " | Ar-78 |
| C-817 | " | " | " | Ar-82 | C-1597 | " | " | " | Ar-82 |
| C-818 | " | " | " | Ar-117 | C-1598 | " | " | " | Ar-117 |
| C-819 | " | " | " | Ar-134 | C-1599 | " | " | " | Ar-134 |
| C-820 | " | " | " | Ar-139 | C-1600 | " | " | " | Ar-139 |
| C-821 | " | " | " | Ar-150 | C-1601 | " | " | " | Ar-150 |
| C-822 | " | " | " | Ar-172 | C-1602 | " | " | " | Ar-172 |
| C-823 | " | " | Ar-74 | Ar-74 | C-1603 | " | " | Ar-74 | Ar-74 |
| C-824 | " | " | " | Ar-78 | C-1604 | " | " | " | Ar-78 |
| C-825 | " | " | " | Ar-82 | C-1605 | " | " | " | Ar-82 |
| C-826 | " | " | " | Ar-117 | C-1606 | " | " | " | Ar-117 |
| C-827 | " | " | " | Ar-134 | C-1607 | " | " | " | Ar-134 |
| C-828 | " | " | " | Ar-139 | C-1608 | " | " | " | Ar-139 |
| C-829 | " | " | " | Ar-150 | C-1609 | " | " | " | Ar-150 |
| C-830 | " | " | " | Ar-172 | C-1610 | " | " | " | Ar-172 |
| C-831 | " | " | Ar-78 | Ar-78 | C-1611 | " | " | Ar-78 | Ar-78 |
| C-832 | " | " | " | Ar-82 | C-1612 | " | " | " | Ar-82 |
| C-833 | " | " | " | Ar-117 | C-1613 | " | " | " | Ar-117 |
| C-834 | " | " | " | Ar-134 | C-1614 | " | " | " | Ar-134 |
| C-835 | " | " | " | Ar-139 | C-1615 | " | " | " | Ar-139 |
| C-836 | " | " | " | Ar-150 | C-1616 | " | " | " | Ar-150 |
| C-837 | " | " | " | Ar-172 | C-1617 | " | " | " | Ar-172 |
| C-838 | " | " | Ar-82 | Ar-82 | C-1618 | " | " | Ar-82 | Ar-82 |
| C-839 | " | " | " | Ar-117 | C-1619 | " | " | " | Ar-117 |
| C-840 | " | " | " | Ar-134 | C-1620 | " | " | " | Ar-134 |
| C-841 | " | " | " | Ar-139 | C-1621 | " | " | " | Ar-139 |
| C-842 | " | " | " | Ar-150 | C-1622 | " | " | " | Ar-150 |
| C-843 | " | " | " | Ar-172 | C-1623 | " | " | " | Ar-172 |
| C-844 | " | " | Ar-117 | Ar-117 | C-1624 | " | " | Ar-117 | Ar-117 |
| C-845 | " | " | " | Ar-134 | C-1625 | " | " | " | Ar-134 |
| C-846 | " | " | " | Ar-139 | C-1626 | " | " | " | Ar-139 |
| C-847 | " | " | " | Ar-150 | C-1627 | " | " | " | Ar-150 |
| C-848 | " | " | " | Ar-172 | C-1628 | " | " | " | Ar-172 |
| C-849 | " | " | Ar-134 | Ar-134 | C-1629 | " | " | Ar-134 | Ar-134 |
| C-850 | " | " | " | Ar-139 | C-1630 | " | " | " | Ar-139 |
| C-851 | " | " | " | Ar-150 | C-1631 | " | " | " | Ar-150 |
| C-852 | " | " | " | Ar-172 | C-1632 | " | " | " | Ar-172 |
| C-853 | " | " | Ar-139 | Ar-139 | C-1633 | " | " | Ar-139 | Ar-139 |
| C-854 | " | " | " | Ar-150 | C-1634 | " | " | " | Ar-150 |
| C-855 | " | " | " | Ar-172 | C-1635 | " | " | " | Ar-172 |
| C-856 | " | " | Ar-150 | Ar-150 | C-1636 | " | " | Ar-150 | Ar-150 |
| C-857 | " | " | " | Ar-172 | C-1637 | " | " | " | Ar-172 |
| C-858 | " | " | Ar-172 | Ar-172 | C-1638 | " | " | Ar-172 | Ar-172 |
| C-859 | " | tert-Butyl | Ar-1 | Ar-1 | C-1639 | " | F | Ar-1 | Ar-1 |
| C-860 | " | " | " | Ar-2 | C-1640 | " | " | " | Ar-2 |
| C-861 | " | " | " | Ar-4 | C-1641 | " | " | " | Ar-4 |
| C-862 | " | " | " | Ar-5 | C-1642 | " | " | " | Ar-5 |
| C-863 | " | " | " | Ar-74 | C-1643 | " | " | " | Ar-74 |
| C-864 | " | " | " | Ar-78 | C-1644 | " | " | " | Ar-78 |
| C-865 | " | " | " | Ar-82 | C-1645 | " | " | " | Ar-82 |
| C-866 | " | " | " | Ar-117 | C-1646 | " | " | " | Ar-117 |
| C-867 | " | " | " | Ar-134 | C-1647 | " | " | " | Ar-134 |
| C-868 | " | " | " | Ar-139 | C-1648 | " | " | " | Ar-139 |
| C-869 | " | " | " | Ar-150 | C-1649 | " | " | " | Ar-150 |
| C-870 | " | " | " | Ar-172 | C-1650 | " | " | " | Ar-172 |
| C-871 | " | " | Ar-2 | Ar-2 | C-1651 | " | " | Ar-2 | Ar-2 |
| C-872 | " | " | " | Ar-4 | C-1652 | " | " | " | Ar-4 |
| C-873 | " | " | " | Ar-5 | C-1653 | " | " | " | Ar-5 |
| C-874 | " | " | " | Ar-74 | C-1654 | " | " | " | Ar-74 |
| C-875 | " | " | " | Ar-78 | C-1655 | " | " | " | Ar-78 |
| C-876 | " | " | " | Ar-82 | C-1656 | " | " | " | Ar-82 |
| C-877 | " | " | " | Ar-117 | C-1657 | " | " | " | Ar-117 |
| C-878 | " | " | " | Ar-134 | C-1658 | " | " | " | Ar-134 |
| C-879 | " | " | " | Ar-139 | C-1659 | " | " | " | Ar-139 |
| C-880 | " | " | " | Ar-150 | C-1660 | " | " | " | Ar-150 |
| C-881 | " | " | " | Ar-172 | C-1661 | " | " | " | Ar-172 |
| C-882 | " | " | Ar-4 | Ar-4 | C-1662 | " | " | Ar-4 | Ar-4 |
| C-883 | " | " | " | Ar-5 | C-1663 | " | " | " | Ar-5 |
| C-884 | " | " | " | Ar-74 | C-1664 | " | " | " | Ar-74 |
| C-885 | " | " | " | Ar-78 | C-1665 | " | " | " | Ar-78 |
| C-886 | " | " | " | Ar-82 | C-1666 | " | " | " | Ar-82 |
| C-887 | " | " | " | Ar-117 | C-1667 | " | " | " | Ar-117 |
| C-888 | " | " | " | Ar-134 | C-1668 | " | " | " | Ar-134 |
| C-889 | " | " | " | Ar-139 | C-1669 | " | " | " | Ar-139 |
| C-890 | " | " | " | Ar-150 | C-1670 | " | " | " | Ar-150 |
| C-891 | " | " | " | Ar-172 | C-1671 | " | " | " | Ar-172 |
| C-892 | " | " | Ar-5 | Ar-5 | C-1672 | " | " | Ar-5 | Ar-5 |
| C-893 | " | " | " | Ar-74 | C-1673 | " | " | " | Ar-74 |
| C-894 | " | " | " | Ar-78 | C-1674 | " | " | " | Ar-78 |
| C-895 | " | " | " | Ar-82 | C-1675 | " | " | " | Ar-82 |
| C-896 | " | " | " | Ar-117 | C-1676 | " | " | " | Ar-117 |
| C-897 | " | " | " | Ar-134 | C-1677 | " | " | " | Ar-134 |
| C-898 | " | " | " | Ar-139 | C-1678 | " | " | " | Ar-139 |
| C-899 | " | " | " | Ar-150 | C-1679 | " | " | " | Ar-150 |
| C-900 | " | " | " | Ar-172 | C-1680 | " | " | " | Ar-172 |
| C-901 | " | " | Ar-74 | Ar-74 | C-1681 | " | " | Ar-74 | Ar-74 |
| C-902 | " | " | " | Ar-78 | C-1682 | " | " | " | Ar-78 |
| C-903 | " | " | " | Ar-82 | C-1683 | " | " | " | Ar-82 |
| C-904 | " | " | " | Ar-117 | C-1684 | " | " | " | Ar-117 |
| C-905 | " | " | " | Ar-134 | C-1685 | " | " | " | Ar-134 |
| C-906 | " | " | " | Ar-139 | C-1686 | " | " | " | Ar-139 |
| C-907 | " | " | " | Ar-150 | C-1687 | " | " | " | Ar-150 |
| C-908 | " | " | " | Ar-172 | C-1688 | " | " | " | Ar-172 |
| C-909 | " | " | Ar-78 | Ar-78 | C-1689 | " | " | Ar-78 | Ar-78 |
| C-910 | " | " | " | Ar-82 | C-1690 | " | " | " | Ar-82 |
| C-911 | " | " | " | Ar-117 | C-1691 | " | " | " | Ar-117 |
| C-912 | " | " | " | Ar-134 | C-1692 | " | " | " | Ar-134 |
| C-913 | " | " | " | Ar-139 | C-1693 | " | " | " | Ar-139 |
| C-914 | " | " | " | Ar-150 | C-1694 | " | " | " | Ar-150 |
| C-915 | " | " | " | Ar-172 | C-1695 | " | " | " | Ar-172 |
| C-916 | " | " | Ar-82 | Ar-82 | C-1696 | " | " | Ar-82 | Ar-82 |
| C-917 | " | " | " | Ar-117 | C-1697 | " | " | " | Ar-117 |
| C-918 | " | " | " | Ar-134 | C-1698 | " | " | " | Ar-134 |
| C-919 | " | " | " | Ar-139 | C-1699 | " | " | " | Ar-139 |
| C-920 | " | " | " | Ar-150 | C-1700 | " | " | " | Ar-150 |
| C-921 | " | " | " | Ar-172 | C-1701 | " | " | " | Ar-172 |
| C-922 | " | " | Ar-117 | Ar-117 | C-1702 | " | " | Ar-117 | Ar-117 |
| C-923 | " | " | " | Ar-134 | C-1703 | " | " | " | Ar-134 |
| C-924 | " | " | " | Ar-139 | C-1704 | " | " | " | Ar-139 |
| C-925 | " | " | " | Ar-150 | C-1705 | " | " | " | Ar-150 |
| C-926 | " | " | " | Ar-172 | C-1706 | " | " | " | Ar-172 |
| C-927 | " | " | Ar-134 | Ar-134 | C-1707 | " | " | Ar-134 | Ar-134 |
| C-928 | " | " | " | Ar-139 | C-1708 | " | " | " | Ar-139 |
| C-929 | " | " | " | Ar-150 | C-1709 | " | " | " | Ar-150 |
| C-930 | " | " | " | Ar-172 | C-1710 | " | " | " | Ar-172 |
| C-931 | " | " | Ar-139 | Ar-139 | C-1711 | " | " | Ar-139 | Ar-139 |
| C-932 | " | " | " | Ar-150 | C-1712 | " | " | " | Ar-150 |
| C-933 | " | " | " | Ar-172 | C-1713 | " | " | " | Ar-172 |
| C-934 | " | " | Ar-150 | Ar-150 | C-1714 | " | " | Ar-150 | Ar-150 |
| C-935 | " | " | " | Ar-172 | C-1715 | " | " | " | Ar-172 |
| C-936 | " | " | Ar-172 | Ar-172 | C-1716 | " | " | Ar-172 | Ar-172 |
| C-937 | R-21 | H | Ar-1 | Ar-1 | C-1717 | " | Methyl | Ar-1 | Ar-1 |
| C-938 | " | " | " | Ar-2 | C-1718 | " | " | " | Ar-2 |
| C-939 | " | " | " | Ar-4 | C-1719 | " | " | " | Ar-4 |
| C-940 | " | " | " | Ar-5 | C-1720 | " | " | " | Ar-5 |
| C-941 | " | " | " | Ar-74 | C-1721 | " | " | " | Ar-74 |
| C-942 | " | " | " | Ar-78 | C-1722 | " | " | " | Ar-78 |
| C-943 | " | " | " | Ar-82 | C-1723 | " | " | " | Ar-82 |
| C-944 | " | " | " | Ar-117 | C-1724 | " | " | " | Ar-117 |
| C-945 | " | " | " | Ar-134 | C-1725 | " | " | " | Ar-134 |
| C-946 | " | " | " | Ar-139 | C-1726 | " | " | " | Ar-139 |
| C-947 | " | " | " | Ar-150 | C-1727 | " | " | " | Ar-150 |
| C-948 | " | " | " | Ar-172 | C-1728 | " | " | " | Ar-172 |
| C-949 | " | " | Ar-2 | Ar-2 | C-1729 | " | " | Ar-2 | Ar-2 |
| C-950 | " | " | " | Ar-4 | C-1730 | " | " | " | Ar-4 |
| C-951 | " | " | " | Ar-5 | C-1731 | " | " | " | Ar-5 |
| C-952 | " | " | " | Ar-74 | C-1732 | " | " | " | Ar-74 |
| C-953 | " | " | " | Ar-78 | C-1733 | " | " | " | Ar-78 |
| C-954 | " | " | " | Ar-82 | C-1734 | " | " | " | Ar-82 |
| C-955 | " | " | " | Ar-117 | C-1735 | " | " | " | Ar-117 |
| C-956 | " | " | " | Ar-134 | C-1736 | " | " | " | Ar-134 |
| C-957 | " | " | " | Ar-139 | C-1737 | " | " | " | Ar-139 |
| C-958 | " | " | " | Ar-150 | C-1738 | " | " | " | Ar-150 |
| C-959 | " | " | " | Ar-172 | C-1739 | " | " | " | Ar-172 |
| C-960 | " | " | Ar-4 | Ar-4 | C-1740 | " | " | Ar-4 | Ar-4 |
| C-961 | " | " | " | Ar-5 | C-1741 | " | " | " | Ar-5 |
| C-962 | " | " | " | Ar-74 | C-1742 | " | " | " | Ar-74 |
| C-963 | " | " | " | Ar-78 | C-1743 | " | " | " | Ar-78 |
| C-964 | " | " | " | Ar-82 | C-1744 | " | " | " | Ar-82 |
| C-965 | " | " | " | Ar-117 | C-1745 | " | " | " | Ar-117 |
| C-966 | " | " | " | Ar-134 | C-1746 | " | " | " | Ar-134 |
| C-967 | " | " | " | Ar-139 | C-1747 | " | " | " | Ar-139 |
| C-968 | " | " | " | Ar-150 | C-1748 | " | " | " | Ar-150 |
| C-969 | " | " | " | Ar-172 | C-1749 | " | " | " | Ar-172 |
| C-970 | " | " | Ar-5 | Ar-5 | C-1750 | " | " | Ar-5 | Ar-5 |
| C-971 | " | " | " | Ar-74 | C-1 751 | " | " | " | Ar-74 |
| C-972 | " | " | " | Ar-78 | C-1752 | " | " | " | Ar-78 |
| C-973 | " | " | " | Ar-82 | C-1753 | " | " | " | Ar-82 |
| C-974 | " | " | " | Ar-117 | C-1754 | " | " | " | Ar-117 |
| C-975 | " | " | " | Ar-134 | C-1755 | " | " | " | Ar-134 |
| C-976 | " | " | " | Ar-139 | C-1756 | " | " | " | Ar-139 |
| C-977 | " | " | " | Ar-150 | C-1757 | " | " | " | Ar-150 |
| C-978 | " | " | " | Ar-172 | C-1758 | " | " | " | Ar-172 |
| C-979 | " | " | Ar-74 | Ar-74 | C-1759 | " | " | Ar-74 | Ar-74 |
| C-980 | " | " | " | Ar-78 | C-1760 | " | " | " | Ar-78 |
| C-981 | " | " | " | Ar-82 | C-1761 | " | " | " | Ar-82 |
| C-982 | " | " | " | Ar-117 | C-1762 | " | " | " | Ar-117 |
| C-983 | " | " | " | Ar-134 | C-1763 | " | " | " | Ar-134 |
| C-984 | " | " | " | Ar-139 | C-1764 | " | " | " | Ar-139 |
| C-985 | " | " | " | Ar-150 | C-1765 | " | " | " | Ar-150 |
| C-986 | " | " | " | Ar-172 | C-1766 | " | " | " | Ar-172 |
| C-987 | " | " | Ar-78 | Ar-78 | C-1767 | " | " | Ar-78 | Ar-78 |
| C-988 | " | " | " | Ar-82 | C-1768 | " | " | " | Ar-82 |
| C-989 | " | " | " | Ar-117 | C-1769 | " | " | " | Ar-117 |
| C-990 | " | " | " | Ar-134 | C-1770 | " | " | " | Ar-134 |
| C-991 | " | " | " | Ar-139 | C-1771 | " | " | " | Ar-139 |
| C-992 | " | " | " | Ar-150 | C-1772 | " | " | " | Ar-150 |
| C-993 | " | " | " | Ar-172 | C-1773 | " | " | " | Ar-172 |
| C-994 | " | " | Ar-82 | Ar-82 | C-1774 | " | " | Ar-82 | Ar-82 |
| C-995 | " | " | " | Ar-117 | C-1775 | " | " | " | Ar-117 |
| C-996 | " | " | " | Ar-134 | C-1776 | " | " | " | Ar-134 |
| C-997 | " | " | " | Ar-139 | C-1777 | " | " | " | Ar-139 |
| C-998 | " | " | " | Ar-150 | C-1778 | " | " | " | Ar-150 |
| C-999 | " | " | " | Ar-172 | C-1779 | " | " | " | Ar-172 |
| C-1000 | " | " | Ar-117 | Ar-117 | C-1780 | " | " | Ar-117 | Ar-117 |
| C-1001 | " | " | " | Ar-134 | C-1781 | " | " | " | Ar-134 |
| C-1 002 | " | " | " | Ar-139 | C-1782 | " | " | " | Ar-139 |
| C-1003 | " | " | " | Ar-150 | C-1783 | " | " | " | Ar-150 |
| C-1004 | " | " | " | Ar-172 | C-1784 | " | " | " | Ar-172 |
| C-1005 | " | " | Ar-134 | Ar-134 | C-1785 | " | " | Ar-134 | Ar-134 |
| C-1006 | " | " | " | Ar-139 | C-1786 | " | " | " | Ar-139 |
| C-1007 | " | " | " | Ar-150 | C-1787 | " | " | " | Ar-150 |
| C-1008 | " | " | " | Ar-172 | C-1788 | " | " | " | Ar-172 |
| C-1009 | " | " | Ar-139 | Ar-139 | C-1789 | " | " | Ar-139 | Ar-139 |
| C-1010 | " | " | " | Ar-150 | C-1790 | " | " | " | Ar-150 |
| C-1011 | " | " | " | Ar-172 | C-1791 | " | " | " | Ar-172 |
| C-1012 | " | " | Ar-150 | Ar-150 | C-1792 | " | " | Ar-150 | Ar-150 |
| C-1013 | " | " | " | Ar-172 | C-1793 | " | " | " | Ar-172 |
| C-1014 | " | " | Ar-172 | Ar-172 | C-1794 | " | " | Ar-172 | Ar-172 |
| C-1015 | " | F | Ar-1 | Ar-1 | C-1795 | " | tert-Butyl | Ar-1 | Ar-1 |
| C-1016 | " | " | " | Ar-2 | C-1796 | " | " | " | Ar-2 |
| C-1017 | " | " | " | Ar-4 | C-1797 | " | " | " | Ar-4 |
| C-1018 | " | " | " | Ar-5 | C-1798 | " | " | " | Ar-5 |
| C-1019 | " | " | " | Ar-74 | C-1799 | " | " | " | Ar-74 |
| C-1 020 | " | " | " | Ar-78 | C-1800 | " | " | " | Ar-78 |
| C-1021 | " | " | " | Ar-82 | C-1801 | " | " | " | Ar-82 |
| C-1022 | " | " | " | Ar-117 | C-1802 | " | " | " | Ar-117 |
| C-1023 | " | " | " | Ar-134 | C-1803 | " | " | " | Ar-134 |
| C-1024 | " | " | " | Ar-139 | C-1804 | " | " | " | Ar-139 |
| C-1025 | " | " | " | Ar-150 | C-1805 | " | " | " | Ar-150 |
| C-1026 | " | " | " | Ar-172 | C-1806 | " | " | " | Ar-172 |
| C-1027 | " | " | Ar-2 | Ar-2 | C-1807 | " | " | Ar-2 | Ar-2 |
| C-1028 | " | " | " | Ar-4 | C-1808 | " | " | " | Ar-4 |
| C-1029 | " | " | " | Ar-5 | C-1809 | " | " | " | Ar-5 |
| C-1 030 | " | " | " | Ar-74 | C-1810 | " | " | " | Ar-74 |
| C-1031 | " | " | " | Ar-78 | C-1811 | " | " | " | Ar-78 |
| C-1032 | " | " | " | Ar-82 | C-1812 | " | " | " | Ar-82 |
| C-1033 | " | " | " | Ar-117 | C-1813 | " | " | " | Ar-117 |
| C-1034 | " | " | " | Ar-134 | C-1814 | " | " | " | Ar-134 |
| C-1035 | " | " | " | Ar-139 | C-1815 | " | " | " | Ar-139 |
| C-1036 | " | " | " | Ar-150 | C-1816 | " | " | " | Ar-150 |
| C-1037 | " | " | " | Ar-172 | C-1817 | " | " | " | Ar-172 |
| C-1038 | " | " | Ar-4 | Ar-4 | C-1818 | " | " | Ar-4 | Ar-4 |
| C-1039 | " | " | " | Ar-5 | C-1819 | " | " | " | Ar-5 |
| C-1040 | " | " | " | Ar-74 | C-1820 | " | " | " | Ar-74 |
| C-1041 | " | " | " | Ar-78 | C-1821 | " | " | " | Ar-78 |
| C-1042 | " | " | " | Ar-82 | C-1822 | " | " | " | Ar-82 |
| C-1043 | " | " | " | Ar-117 | C-1823 | " | " | " | Ar-117 |
| C-1044 | " | " | " | Ar-134 | C-1824 | " | " | " | Ar-134 |
| C-1045 | " | " | " | Ar-139 | C-1825 | " | " | " | Ar-139 |
| C-1046 | " | " | " | Ar-150 | C-1826 | " | " | " | Ar-150 |
| C-1047 | " | " | " | Ar-172 | C-1827 | " | " | " | Ar-172 |
| C-1048 | " | " | Ar-5 | Ar-5 | C-1828 | " | " | Ar-5 | Ar-5 |
| C-1049 | " | " | " | Ar-74 | C-1829 | " | " | " | Ar-74 |
| C-1050 | " | " | " | Ar-78 | C-1830 | " | " | " | Ar-78 |
| C-1051 | " | " | " | Ar-82 | C-1831 | " | " | " | Ar-82 |
| C-1052 | " | " | " | Ar-117 | C-1832 | " | " | " | Ar-117 |
| C-1053 | " | " | " | Ar-134 | C-1833 | " | " | " | Ar-134 |
| C-1054 | " | " | " | Ar-139 | C-1834 | " | " | " | Ar-139 |
| C-1055 | " | " | " | Ar-150 | C-1835 | " | " | " | Ar-150 |
| C-1056 | " | " | " | Ar-172 | C-1836 | " | " | " | Ar-172 |
| C-1057 | " | " | Ar-74 | Ar-74 | C-1837 | " | " | Ar-74 | Ar-74 |
| C-1058 | " | " | " | Ar-78 | C-1838 | " | " | " | Ar-78 |
| C-1059 | " | " | " | Ar-82 | C-1839 | " | " | " | Ar-82 |
| C-1060 | " | " | " | Ar-117 | C-1840 | " | " | " | Ar-117 |
| C-1061 | " | " | " | Ar-134 | C-1841 | " | " | " | Ar-134 |
| C-1062 | " | " | " | Ar-139 | C-1842 | " | " | " | Ar-139 |
| C-1063 | " | " | " | Ar-150 | C-1843 | " | " | " | Ar-150 |
| C-1064 | " | " | " | Ar-172 | C-1844 | " | " | " | Ar-172 |
| C-1065 | " | " | Ar-78 | Ar-78 | C-1845 | " | " | Ar-78 | Ar-78 |
| C-1066 | " | " | " | Ar-82 | C-1846 | " | " | " | Ar-82 |
| C-1067 | " | " | " | Ar-117 | C-1847 | " | " | " | Ar-117 |
| C-1068 | " | " | " | Ar-134 | C-1848 | " | " | " | Ar-134 |
| C-1069 | " | " | " | Ar-139 | C-1849 | " | " | " | Ar-139 |
| C-1070 | " | " | " | Ar-150 | C-1850 | " | " | " | Ar-150 |
| C-1071 | " | " | " | Ar-172 | C-1851 | " | " | " | Ar-172 |
| C-1072 | " | " | Ar-82 | Ar-82 | C-1852 | " | " | Ar-82 | Ar-82 |
| C-1073 | " | " | " | Ar-117 | C-1853 | " | " | " | Ar-117 |
| C-1074 | " | " | " | Ar-134 | C-1854 | " | " | " | Ar-134 |
| C-1075 | " | " | " | Ar-139 | C-1855 | " | " | " | Ar-139 |
| C-1076 | " | " | " | Ar-150 | C-1856 | " | " | " | Ar-150 |
| C-1077 | " | " | " | Ar-172 | C-1857 | " | " | " | Ar-172 |
| C-1078 | " | " | Ar-117 | Ar-117 | C-1858 | " | " | Ar-117 | Ar-117 |
| C-1079 | " | " | " | Ar-134 | C-1859 | " | " | " | Ar-134 |
| C-1080 | " | " | " | Ar-139 | C-1860 | " | " | " | Ar-139 |
| C-1081 | " | " | " | Ar-150 | C-1861 | " | " | " | Ar-150 |
| C-1082 | " | " | " | Ar-172 | C-1862 | " | " | " | Ar-172 |
| C-1083 | " | " | Ar-134 | Ar-134 | C-1863 | " | " | Ar-134 | Ar-134 |
| C-1084 | " | " | " | Ar-139 | C-1864 | " | " | " | Ar-139 |
| C-1085 | " | " | " | Ar-150 | C-1865 | " | " | " | Ar-150 |
| C-1086 | " | " | " | Ar-172 | C-1866 | " | " | " | Ar-172 |
| C-1087 | " | " | Ar-139 | Ar-139 | C-1867 | " | " | Ar-139 | Ar-139 |
| C-1088 | " | " | " | Ar-150 | C-1868 | " | " | " | Ar-150 |
| C-1089 | " | " | " | Ar-172 | C-1869 | " | " | " | Ar-172 |
| C-1090 | " | " | Ar-150 | Ar-150 | C-1870 | " | " | Ar-150 | Ar-150 |
| C-1091 | " | " | " | Ar-172 | C-1871 | " | " | " | Ar-172 |
| C-1092 | " | " | Ar-172 | Ar-172 | C-1872 | " | " | Ar-172 | Ar-172 |

Furthermore preferred are compounds which correspond to the compounds C-313 to C-1872 above, with the exception that they are derived from the following formula

| |
|---|
| 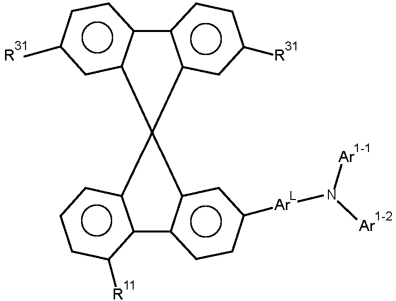 |
| Formula (I-A-1-2-2) |

where Ar^{L} is phenylene, preferably 1,4-phenylene, and where R¹¹, R³¹, Ar¹⁻¹ and Ar¹⁻² are specified as shown for the corresponding compounds C-313 to C-1872.

Preferred compounds according to formula (I) are shown in the following table:

| | | |
|---|---|---|
| 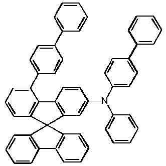 | 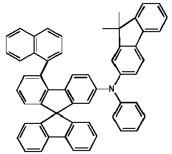 | 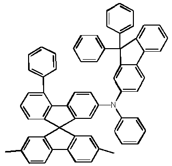 |
| (1) | (2) | (3) |
| 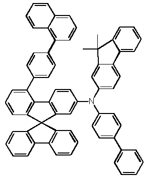 | 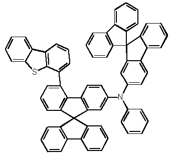 | 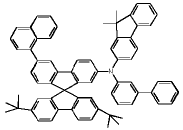 |
| (4) | (5) | (6) |
| 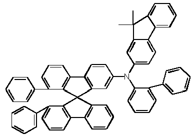 | 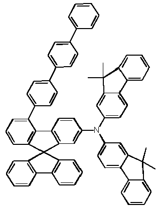 | 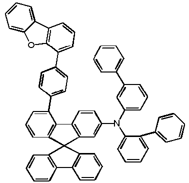 |
| (7) | (8) | (9) |
| 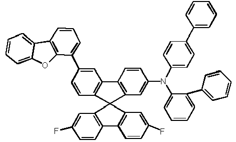 | 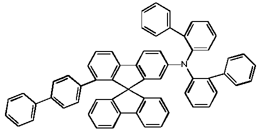 | 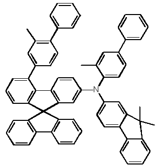 |
| (10) | (11) | (12) |
| 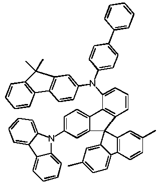 | 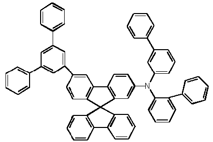 | 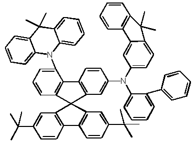 |
| (13) | (14) | (15) |
| 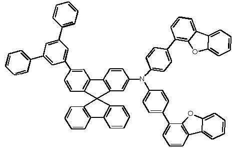 | 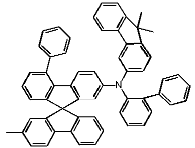 | 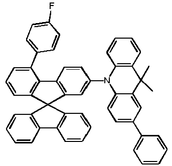 |
| (16) | (17) | (18) |
| 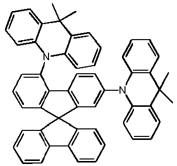 | 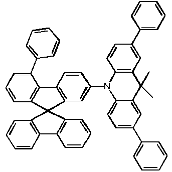 | 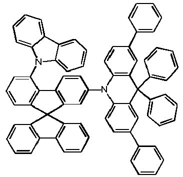 |
| (19) | (20) | (21) |
| 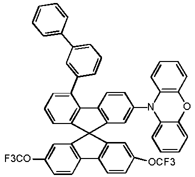 | 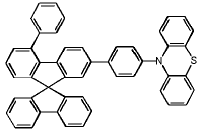 | 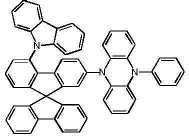 |
| (22) | (23) | (24) |
| 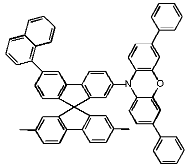 | 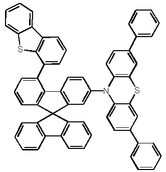 | 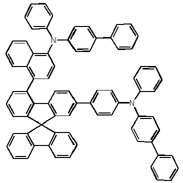 |
| (25) | (26) | (27) |
| 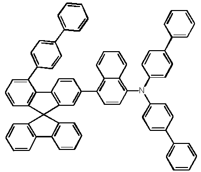 | 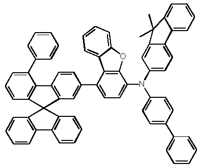 | 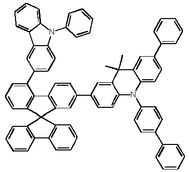 |
| (28) | (29) | (30) |
| 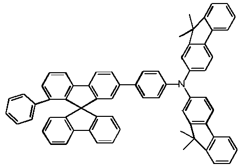 | 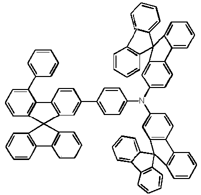 | 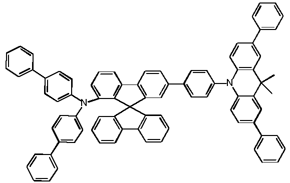 |
| (31) | (32) | (33) |
| 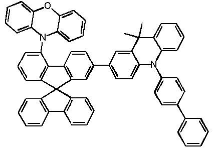 | 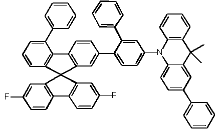 | 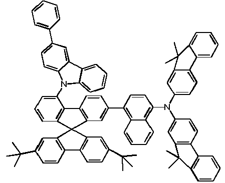 |
| (34) | (35) | (36) |
| 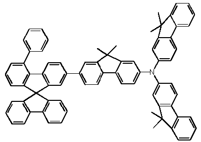 | 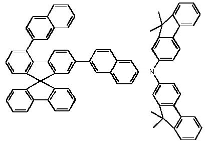 | 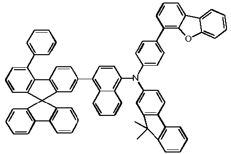 |
| (37) | (38) | (39) |
| 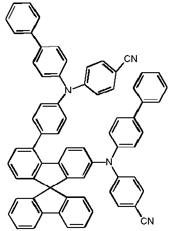 | 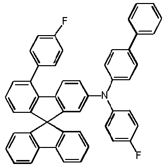 | 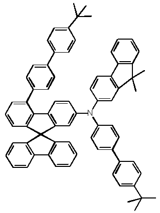 |
| (40) | (41) | (42) |
| 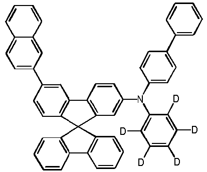 | 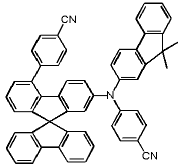 | 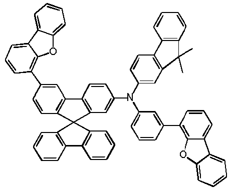 |
| (43) | (44) | (45) |
| 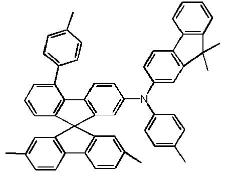 | 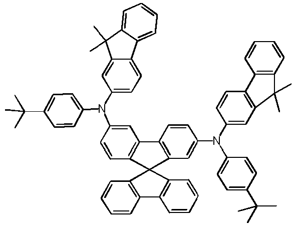 | 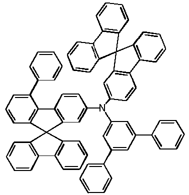 |
| (46) | (47) | (48) |
| 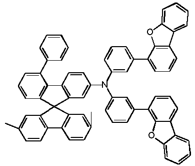 | 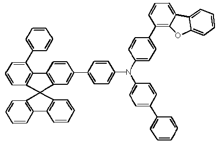 | 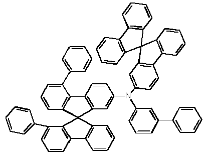 |
| (49) | (50) | (51) |
| 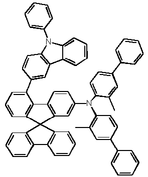 | 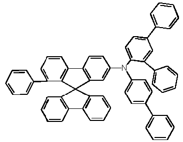 | 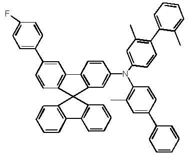 |
| (52) | (53) | (54) |
| 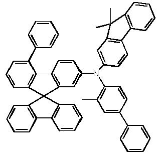 | 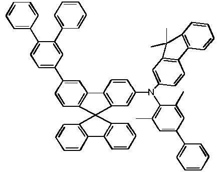 | 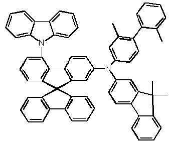 |
| (55) | (56) | (57) |
| 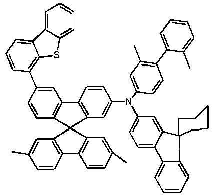 | 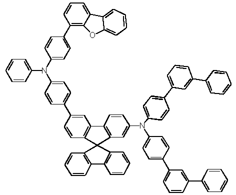 | 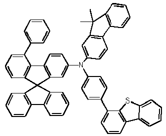 |
| (58) | (59) | (60) |
| 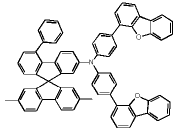 | 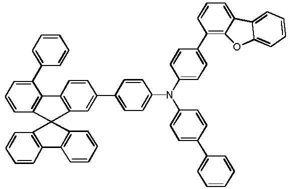 | 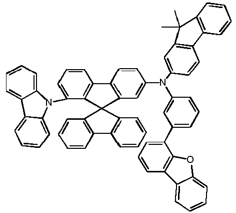 |
| (61) | (62) | (63) |
| 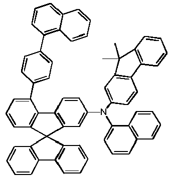 | 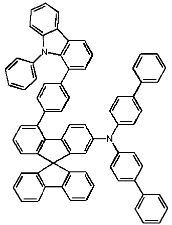 | 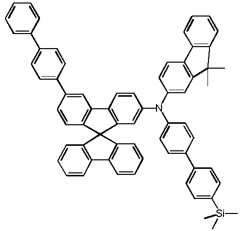 |
| (64) | (65) | (66) |
| 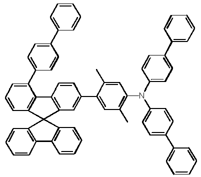 | 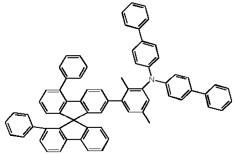 | 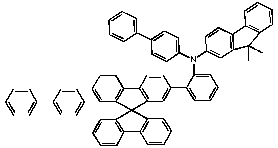 |
| (67) | (68) | (69) |
| 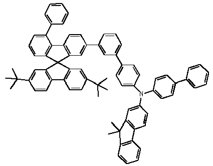 | 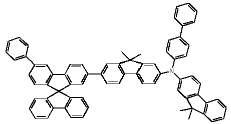 | 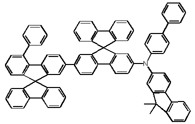 |
| (70) | (71) | (72) |
| 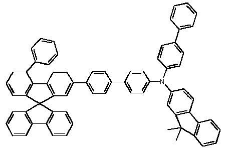 | 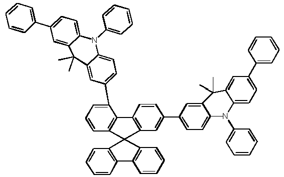 | 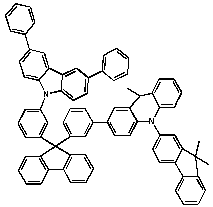 |
| (73) | (74) | (75) |
| 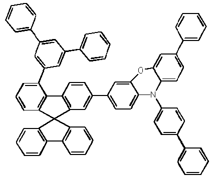 | 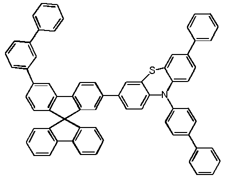 | 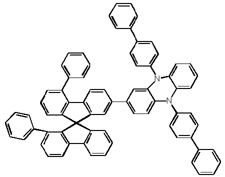 |
| (76) | (77) | (78) |
| 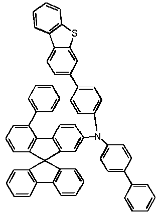 | 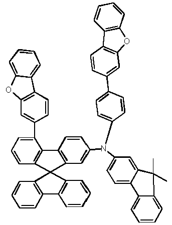 | 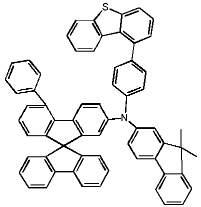 |
| (79) | (80) | (81) |
| 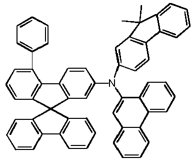 | 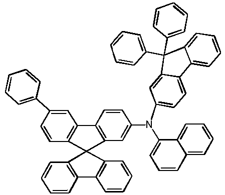 | 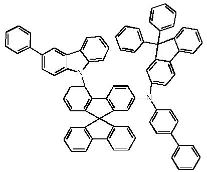 |
| (82) | (83) | (84) |
| 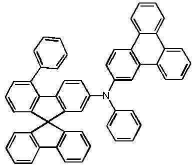 | 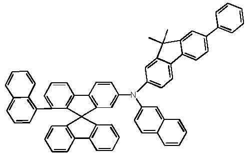 | 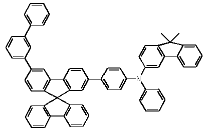 |
| (85) | (86) | (87) |
| 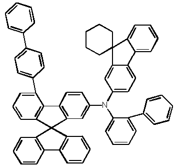 | 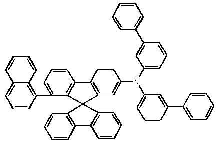 | 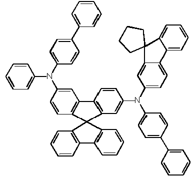 |
| (88) | (89) | (90) |
| 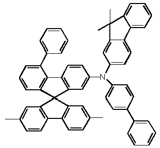 | 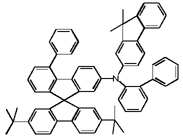 | 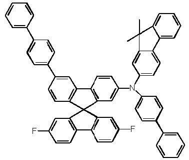 |
| (91) | (92) | (93) |
| 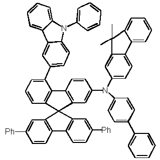 | 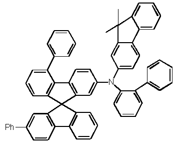 | 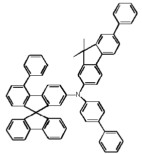 |
| (94) | (95) | (96) |
| 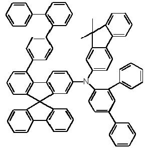 | 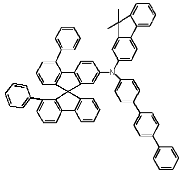 | 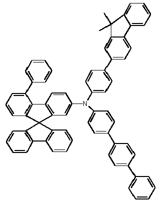 |
| (97) | (98) | (99) |
| 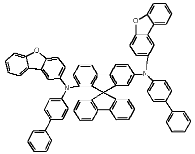 | 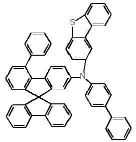 | 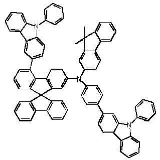 |
| (100) | (101) | (102) |
| 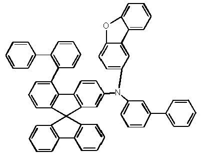 | 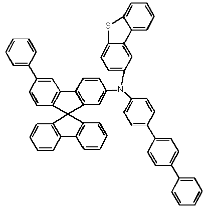 | 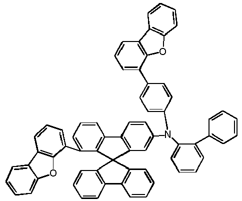 |
| (103) | (104) | (105) |

The compounds according to the present application are prepared by using standard methods known in the art of organic synthesis, such as metal catalysed coupling reactions, in particular Suzuki reactions and Buchwald reactions, nucleophilic addition reactions of metallated aryl derivatives to carbonyl groups, and acid-catalysed cyclisation reactions.

According to a preferred synthesis process, a biphenyl derivative which is substituted with the three reactive groups X¹ to X³, where X¹ is present in the position ortho to the phenyl-phenyl bond, is selectively metallated, preferably with Li or Mg, in the position of X¹. In a second step, the metallated biphenyl derivative is reacted with a fluorenone derivative in a nucleophilic addition reaction. The formed intermediate having the tertiary hydroxyl group is cyclized under acidic conditions to the spirobifluorene, which bears the two reactive groups X² and X³.

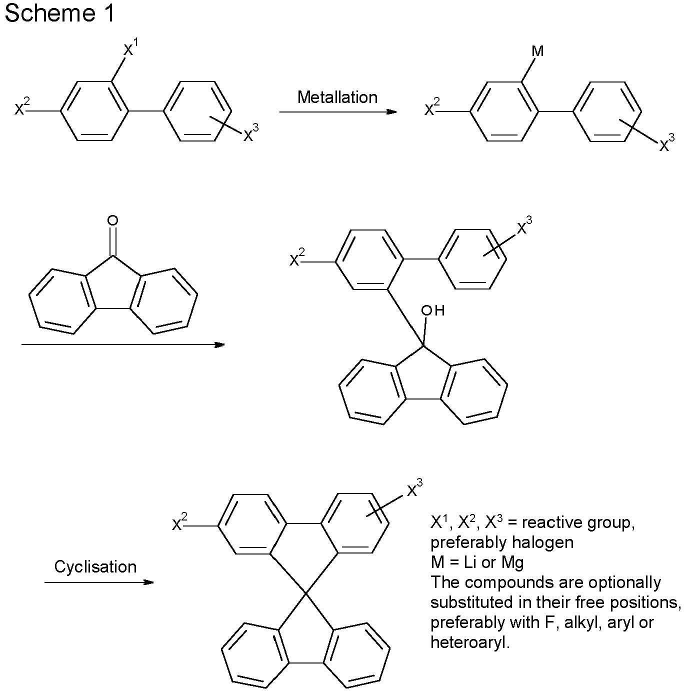

The obtained compounds are then further reacted in the positions of their groups X² and X³, so that an optionally bridged diarylamine group, optionally linked via a spacer group, is present in the position of the group X², and an optionally bridged diarylamine group, optionally liked via a spacer group, or an aryl group, or a heteroaryl group, is present in the position of the group X³. This is achieved with coupling reactions, carried out in sequential or in parallel manner, which are selected from Suzuki reactions with aryl or heteroaryl derivatives and Buchwald reactions with optionally bridged diaryl amine derivatives. In the case of parallel coupling reactions, two optionally bridged diarylamine groups can be introduced in the positions of the groups X² and X³ by Buchwald reaction. In the case where sequential coupling reactions selected from Buchwald reactions and Suzuki reactions are carried out, one after the other, the first reaction takes place at the position of the group X² and X³ which has the higher reactivity, and the second reaction takes place at the position of the group X² and X³ which has the lower reactivity.

As a result of the above-mentioned reactions, compounds according to formula (I) of the present application are obtained.

A further embodiment of the present invention is therefore a process for preparation of a compound according to formula (I), characterized in that it comprises the reactions steps
1) metallation of a biphenyl derivative which has one reactive group in a position which is ortho to the phenyl-phenyl bond, and which bears two additional reactive groups in other positions, where the metallation takes place in the position which is ortho to the phenyl-phenyl bond;
2) addition of the metallated biphenyl derivative to a fluorenone derivative;
3) cyclisation of the resulting addition product to a spirobifluorene derivative, where the cyclisation takes place under acidic conditions or with a Lewis acid, and where the spirobifluorene derivative bears two reactive groups; and
4) coupling of the spirobifluorene derivative with groups selected from aromatic ring systems, heteroaromatic ring systems and amine groups, in the positions of the two reactive groups.

The metallation of step 1) is preferably a lithiation or a Grignard reaction. The reactive group is preferably a halogen group, more preferably Cl or Br. The coupling reaction of step 4) is preferably selected from a Buchwald reaction in the case of coupling with an amine group, and the coupling reaction is preferably selected from a Suzuki reaction in the case of coupling with an aromatic ring system or heteroaromatic ring system. Steps 1) to 4) are preferably carried out in their numeric sequence. Furthermore, preferably, step 2) is carried out directly after step 1), and step 3) is carried out directly after step 2), and step 4) is carried out directly after step 3). "Directly" means in this regard that no chemical reactions are carried out in between the reaction steps.

The above-described compounds, especially compounds substituted by reactive leaving groups, such as bromine, iodine, chlorine, boronic acid or boronic ester, may find use as monomers for production of corresponding oligomers, dendrimers or polymers. Suitable reactive leaving groups are, for example, bromine, iodine, chlorine, boronic acids, boronic esters, amines, alkenyl or alkynyl groups having a terminal C-C double bond or C-C triple bond, oxiranes, oxetanes, groups which enter into a cycloaddition, for example a 1,3-dipolar cycloaddition, for example dienes or azides, carboxylic acid derivatives, alcohols and silanes.

The invention therefore further provides oligomers, polymers or dendrimers containing one or more compounds of formula (I), wherein the bond(s) to the polymer, oligomer or dendrimer may be localized at any desired positions substituted by R¹, R², R³ or R⁴ in formula (I). According to the linkage of the compound of formula (I), the compound is part of a side chain of the oligomer or polymer or part of the main chain. An oligomer in the context of this invention is understood to mean a compound formed from at least three monomer units. A polymer in the context of the invention is understood to mean a compound formed from at least ten monomer units. The polymers, oligomers or dendrimers of the invention may be conjugated, partly conjugated or nonconjugated. The oligomers or polymers of the invention may be linear, branched or dendritic. In the structures having linear linkage, the units of formula (I) may be joined directly to one another, or they may be joined to one another via a bivalent group, for example via a substituted or unsubstituted alkylene group, via a heteroatom or via a bivalent aromatic or heteroaromatic group. In branched and dendritic structures, it is possible, for example, for three or more units of formula (I) to be joined via a trivalent or higher-valency group, for example via a trivalent or higher-valency aromatic or heteroaromatic group, to give a branched or dendritic oligomer or polymer.

For the repeat units of formula (I) in oligomers, dendrimers and polymers, the same preferences apply as described above for compounds of formula (I).

For preparation of the oligomers or polymers, the monomers of the invention are homopolymerized or copolymerized with further monomers. Suitable and preferred comonomers are chosen from fluorenes (for example according to EP 842208 or WO 2000/22026), spirobifluorenes (for example according to EP 707020, EP 894107 or WO 2006/061181), paraphenylenes (for example according to WO 1992/18552), carbazoles (for example according to WO 2004/070772 or WO 2004/113468), thiophenes (for example according to EP 1028136), dihydrophenanthrenes (for example according to WO 2005/014689 or WO 2007/006383), cis- and trans-indenofluorenes (for example according to WO 2004/041901 or WO 2004/113412), ketones (for example according to WO 2005/040302), phenanthrenes (for example according to WO 2005/104264 or WO 2007/017066) or else a plurality of these units. The polymers, oligomers and dendrimers typically contain still further units, for example emitting (fluorescent or phosphorescent) units, for example vinyltriarylamines (for example according to WO 2007/068325) or phosphorescent metal complexes (for example according to WO 2006/003000), and/or charge transport units, especially those based on triarylamines.

The polymers and oligomers of the invention are generally prepared by polymerization of one or more monomer types, of which at least one monomer leads to repeat units of the formula (I) in the polymer. Suitable polymerization reactions are known to those skilled in the art and are described in the literature. Particularly suitable and preferred polymerization reactions which lead to formation of C-C or C-N bonds are the Suzuki polymerization, the Yamamoto polymerization, the Stille polymerization and the Hartwig-Buchwald polymerization.

For the processing of the compounds of the invention from a liquid phase, for example by spin-coating or by printing methods, formulations of the compounds of the invention are required. These formulations may, for example, be solutions, dispersions or emulsions. For this purpose, it may be preferable to use mixtures of two or more solvents. Suitable and preferred solvents are, for example, toluene, anisole, o-, m- or p-xylene, methyl benzoate, mesitylene, tetralin, veratrole, THF, methyl-THF, THP, chlorobenzene, dioxane, phenoxytoluene, especially 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The invention therefore further provides a formulation, especially a solution, dispersion or emulsion, comprising at least one compound of formula (I) and at least one solvent, preferably an organic solvent. The way in which such solutions can be prepared is known to those skilled in the art and is described, for example, in WO 2002/072714, WO 2003/019694 and the literature cited therein.

The compounds of the invention are suitable for use in electronic devices, especially in organic electroluminescent devices (OLEDs). Depending on the substitution, the compounds are used in different functions and layers.

The invention therefore further provides for the use of the compound of formula (I) in an electronic device. This electronic device is preferably selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and more preferably organic electroluminescent devices (OLEDs).

The invention further provides, as already set out above, an electronic device comprising at least one compound of formula (1). This electronic device is preferably selected from the abovementioned devices.

It is more preferably an organic electroluminescent device (OLED) comprising anode, cathode and at least one emitting layer, characterized in that at least one organic layer, which may be an emitting layer, a hole transport layer or another layer, preferably an emitting layer or a hole transport layer, particularly preferably a hole transport layer, comprises at least one compound of formula (1).

Apart from the cathode, anode and emitting layer, the organic electroluminescent device may also comprise further layers. These are selected, for example, from in each case one or more hole injection layers, hole transport layers, hole blocking layers, electron transport layers, electron injection layers, electron blocking layers, exciton blocking layers, interlayers, charge generation layers (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) and/or organic or inorganic p/n junctions.

The sequence of the layers of the organic electroluminescent device comprising the compound of the formula (I) is preferably as follows: anode-hole injection layer-hole transport layer-optionally further hole transport layer(s)-optionally electron blocking layer-emitting layer-optionally hole blocking layer-electron transport layer-electron injection layer-cathode. It is additionally possible for further layers to be present in the OLED.

The organic electroluminescent device of the invention may contain two or more emitting layers. More preferably, these emission layers in this case have several emission maxima between 380 nm and 750 nm overall, such that the overall result is white emission; in other words, various emitting compounds which may fluoresce or phosphoresce and which emit blue, green, yellow, orange or red light are used in the emitting layers. Especially preferred are three-layer systems, i.e. systems having three emitting layers, where the three layers show blue, green and orange or red emission (for the basic construction see, for example, WO 2005/011013). The compounds of the invention are preferably present in the hole transport layer, hole injection layer or electron blocking layer, most preferably in the electron blocking layer.

It is preferable in accordance with the invention when the compound of formula (I) is used in an electronic device comprising one or more phosphorescent emitting compounds. In this case, the compound may be present in different layers, preferably in a hole transport layer, an electron blocking layer, a hole injection layer or in an emitting layer.

The term "phosphorescent emitting compounds" typically encompasses compounds where the emission of light is effected through a spin-forbidden transition, for example a transition from an excited triplet state or a state having a higher spin quantum number, for example a quintet state.

Suitable phosphorescent emitting compounds (= triplet emitters) are especially compounds which, when suitably excited, emit light, preferably in the visible region, and also contain at least one atom of atomic number greater than 20, preferably greater than 38, and less than 84, more preferably greater than 56 and less than 80. Preference is given to using, as phosphorescent emitting compounds, compounds containing copper, molybdenum, tungsten, rhenium, ruthenium, osmium, rhodium, iridium, palladium, platinum, silver, gold or europium, especially compounds containing iridium, platinum or copper. In the context of the present invention, all luminescent iridium, platinum or copper complexes are considered to be phosphorescent emitting compounds.

Examples of the above-described emitting compounds can be found in applications WO 00/70655, WO 01/41512, WO 02/02714, WO 02/15645, EP 1191613, EP 1191612, EP 1191614, WO 05/033244, WO 05/019373 and US 2005/0258742. In general, all phosphorescent complexes as used for phosphorescent OLEDs according to the prior art and as known to those skilled in the art in the field of organic electroluminescent devices are suitable. It is also possible for the person skilled in the art, without exercising inventive skill, to use further phosphorescent complexes in combination with the compounds of formula (I) in organic electroluminescent devices. Further examples are listed in a table which follows.

It is also possible in accordance with the invention to use the compound of formula (I) in an electronic device comprising one or more fluorescent emitting compounds.

In a preferred embodiment of the invention, the compounds of formula (I) are used as hole-transporting material. In that case, the compounds are preferably present in a hole transport layer, an electron blocking layer or a hole injection layer. Particular preference is given to use in an electron blocking layer.

A hole transport layer according to the present application is a layer having a hole-transporting function between the anode and emitting layer.

Hole injection layers and electron blocking layers are understood in the context of the present application to be specific embodiments of hole transport layers. A hole injection layer, in the case of a plurality of hole transport layers between the anode and emitting layer, is a hole transport layer which directly adjoins the anode or is separated therefrom only by a single coating of the anode. An electron blocking layer, in the case of a plurality of hole transport layers between the anode and emitting layer, is that hole transport layer which directly adjoins the emitting layer on the anode side. Preferably, the OLED of the invention comprises two, three or four hole-transporting layers between the anode and emitting layer, at least one of which preferably contains a compound of formula (I), and more preferably exactly one or two contain a compound of formula (I).

If the compound of formula (I) is used as hole transport material in a hole transport layer, a hole injection layer or an electron blocking layer, the compound can be used as pure material, i.e. in a proportion of 100%, in the hole transport layer, or it can be used in combination with one or more further compounds. In a preferred embodiment, the organic layer comprising the compound of the formula (I) then additionally contains one or more p-dopants. p-Dopants used according to the present invention are preferably those organic electron acceptor compounds capable of oxidizing one or more of the other compounds in the mixture.

Particularly preferred embodiments of p-dopants are the compounds disclosed in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 and DE 102012209523.

Particularly preferred p-dopants are quinodimethane compounds, azaindenofluorenediones, azaphenalenes, azatriphenylenes, I₂, metal halides, preferably transition metal halides, metal oxides, preferably metal oxides containing at least one transition metal or a metal of main group 3, and transition metal complexes, preferably complexes of Cu, Co, Ni, Pd and Pt with ligands containing at least one oxygen atom as bonding site. Preference is further given to transition metal oxides as dopants, preferably oxides of rhenium, molybdenum and tungsten, more preferably Re₂O₇, MoO₃, WO₃ and ReO₃.

The p-dopants are preferably in substantially homogeneous distribution in the p-doped layers. This can be achieved, for example, by coevaporation of the p-dopant and the hole transport material matrix.

Preferred p-dopants are especially the following compounds:

| | | |
|---|---|---|
| 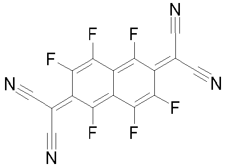 | 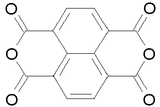 | 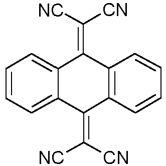 |
| (D-1) | (D-2) | (D-3) |
| 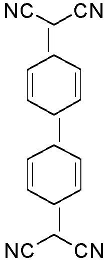 | 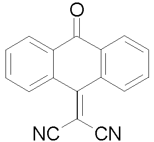 | 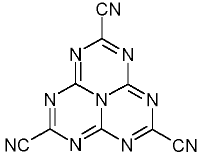 |
| (D-4) | (D-5) | (D-6) |
| 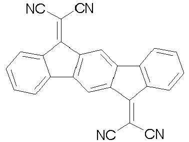 | 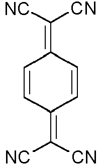 | 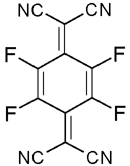 |
| (D-7) | (D-8) | (D-9) |
| 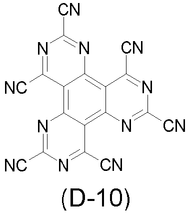 | 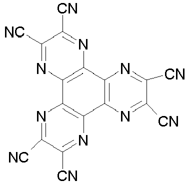 | 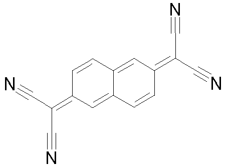 |
| | (D-11) | (D-12) |
| 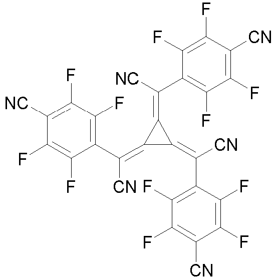 | | |
| (D-13) | | |

In a further preferred embodiment of the invention, the compound of formula (I) is used as hole transport material in combination with a hexaazatriphenylene derivative as described in US 2007/0092755. Particular preference is given here to using the hexaazatriphenylene derivative in a separate layer.

In a further embodiment of the present invention, the compound of the formula (I) is used in an emitting layer as matrix material in combination with one or more emitting compounds, preferably phosphorescent emitting compounds.

The proportion of the matrix material in the emitting layer in this case is between 50.0% and 99.9% by volume, preferably between 80.0% and 99.5% by volume, and more preferably between 92.0% and 99.5% by volume for fluorescent emitting layers and between 85.0% and 97.0% by volume for phosphorescent emitting layers.

Correspondingly, the proportion of the emitting compound is between 0.1% and 50.0% by volume, preferably between 0.5% and 20.0% by volume, and more preferably between 0.5% and 8.0% by volume for fluorescent emitting layers and between 3.0% and 15.0% by volume for phosphorescent emitting layers.

An emitting layer of an organic electroluminescent device may also comprise systems comprising a plurality of matrix materials (mixed matrix systems) and/or a plurality of emitting compounds. In this case too, the emitting compounds are generally those compounds having the smaller proportion in the system and the matrix materials are those compounds having the greater proportion in the system. In individual cases, however, the proportion of a single matrix material in the system may be less than the proportion of a single emitting compound.

It is preferable that the compounds of formula (I) are used as a component of mixed matrix systems. The mixed matrix systems preferably comprise two or three different matrix materials, more preferably two different matrix materials. Preferably, in this case, one of the two materials is a material having hole-transporting properties and the other material is a material having electron-transporting properties. The compound of the formula (I) is preferably the matrix material having hole-transporting properties. The desired electron-transporting and hole-transporting properties of the mixed matrix components may, however, also be combined mainly or entirely in a single mixed matrix component, in which case the further mixed matrix component(s) fulfill(s) other functions. The two different matrix materials may be present in a ratio of 1:50 to 1:1, preferably 1:20 to 1:1, more preferably 1:10 to 1:1 and most preferably 1:4 to 1:1. Preference is given to using mixed matrix systems in phosphorescent organic electroluminescent devices. One source of more detailed information about mixed matrix systems is the application WO 2010/108579.

The mixed matrix systems may comprise one or more emitting compounds, preferably one or more phosphorescent emitting compounds. In general, mixed matrix systems are preferably used in phosphorescent organic electroluminescent devices.

Particularly suitable matrix materials which can be used in combination with the compounds of the invention as matrix components of a mixed matrix system are selected from the preferred matrix materials specified below for phosphorescent emitting compounds or the preferred matrix materials for fluorescent emitting compounds, according to what type of emitting compound is used in the mixed matrix system.

Preferred phosphorescent emitting compounds for use in mixed matrix systems are the same as detailed further up as generally preferred phosphorescent emitter materials.

Preferred embodiments of the different functional materials in the electronic device are listed hereinafter.

Preferred phosphorescent emitting compounds are the following ones:

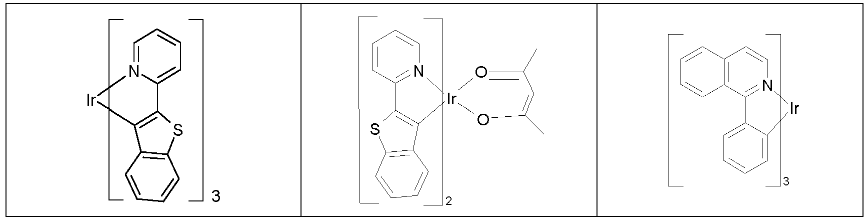

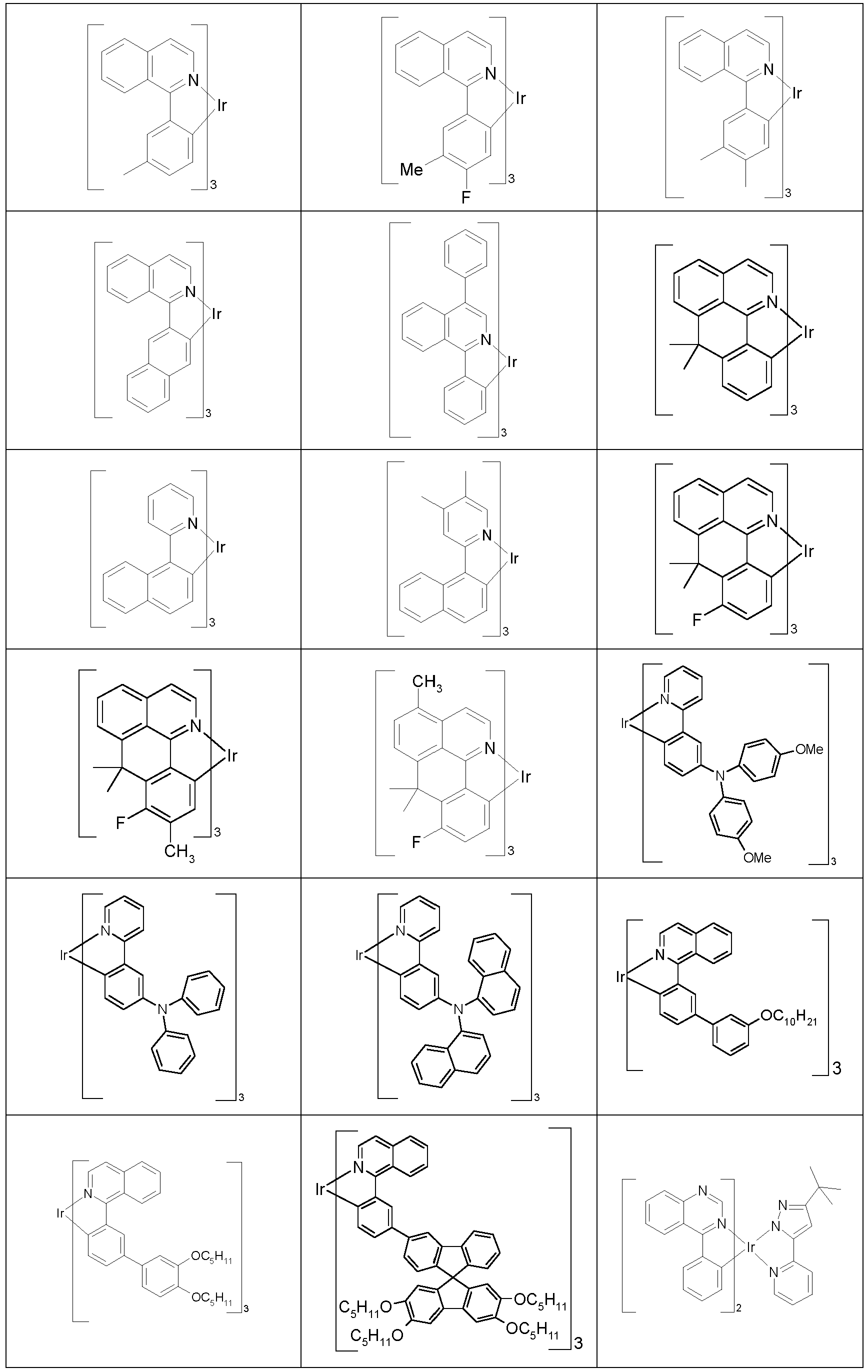

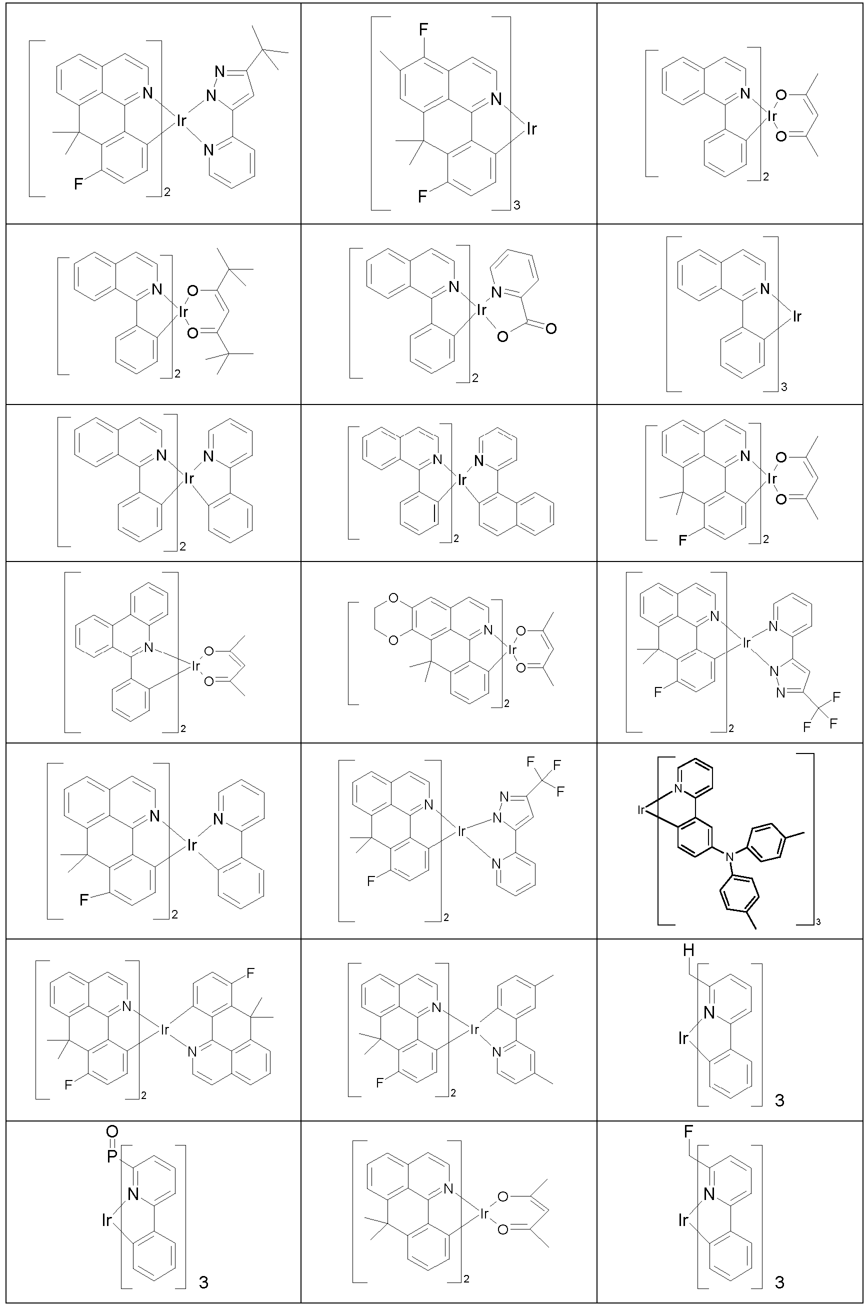

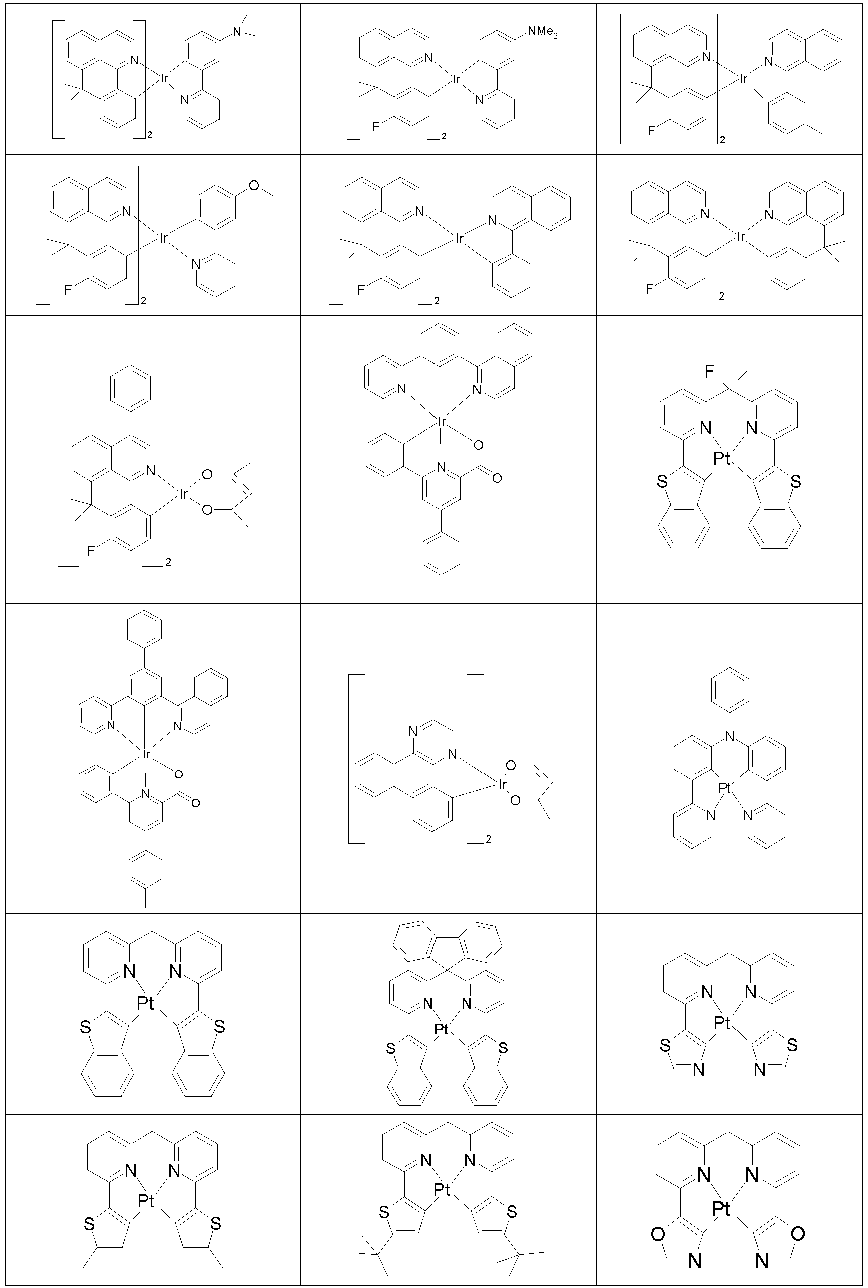

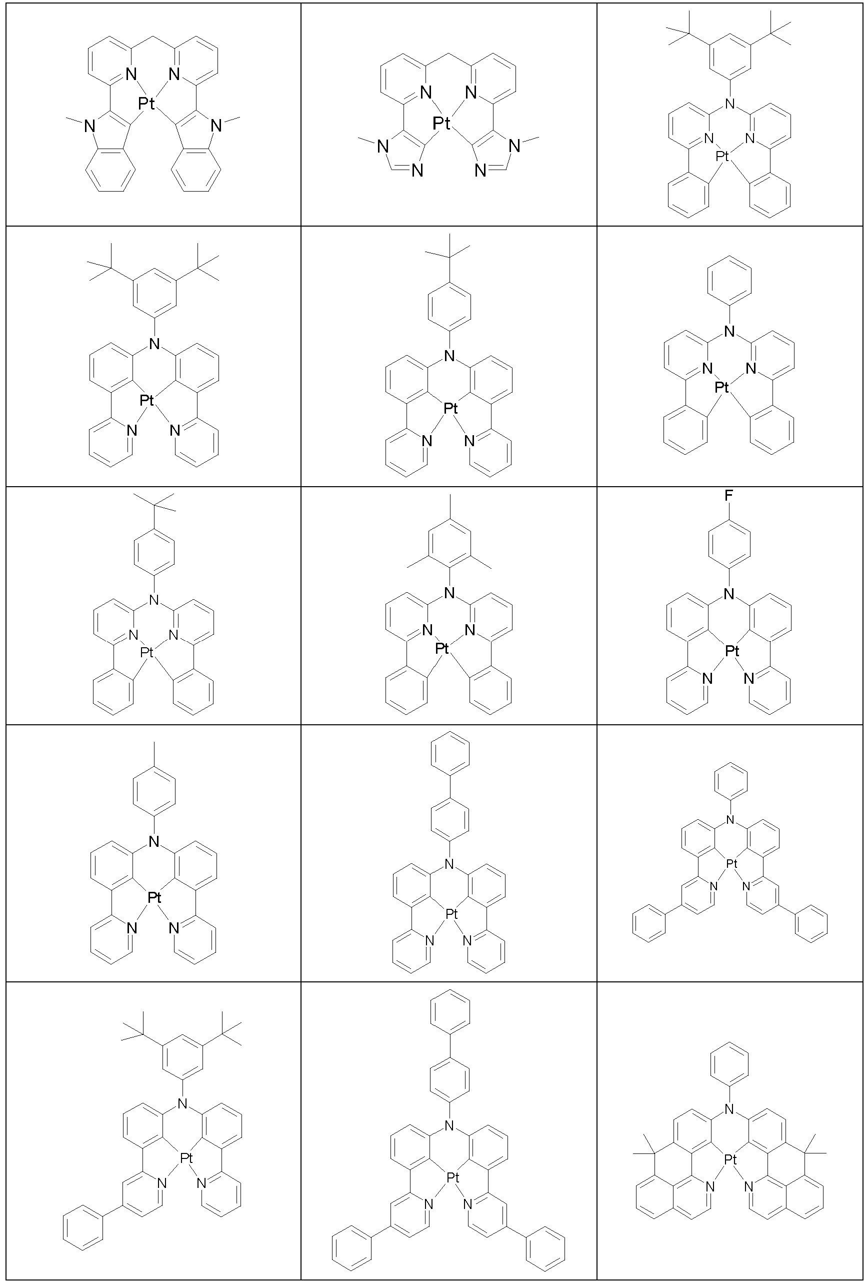

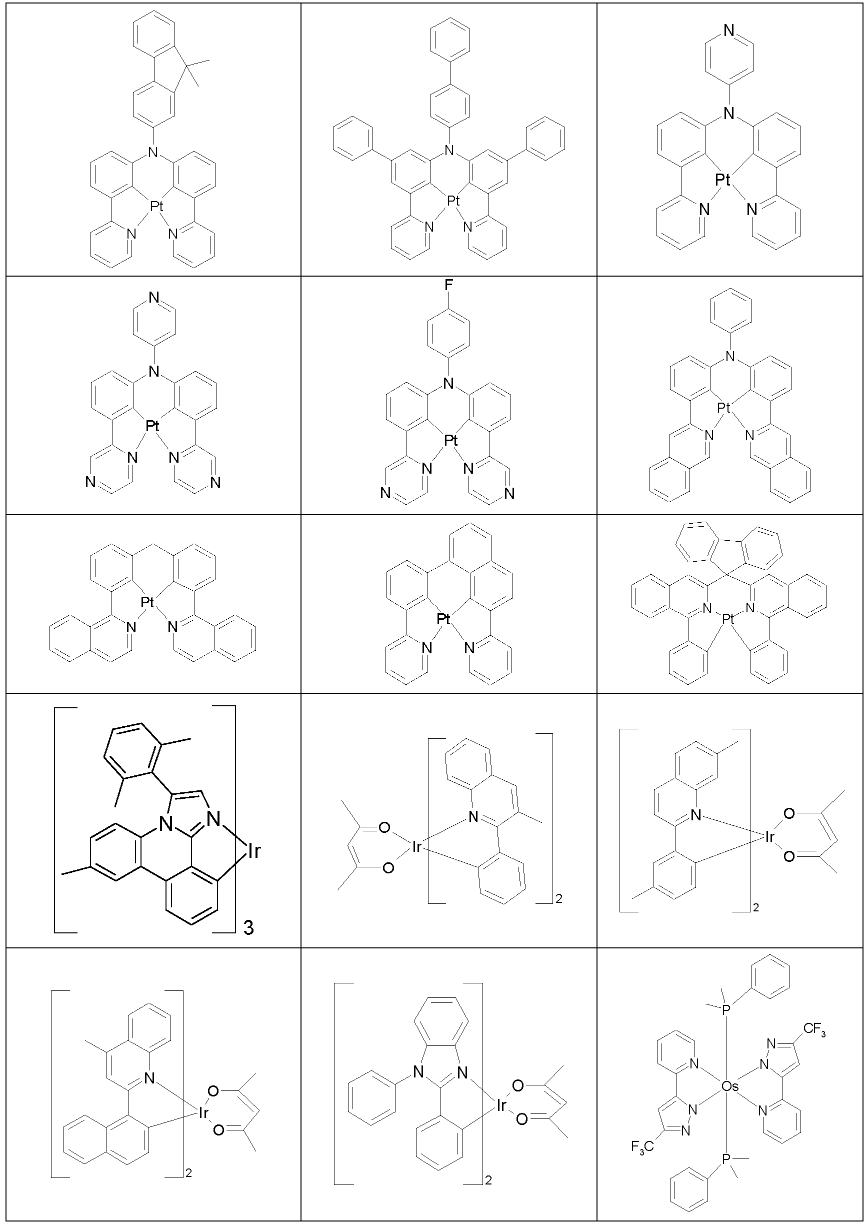

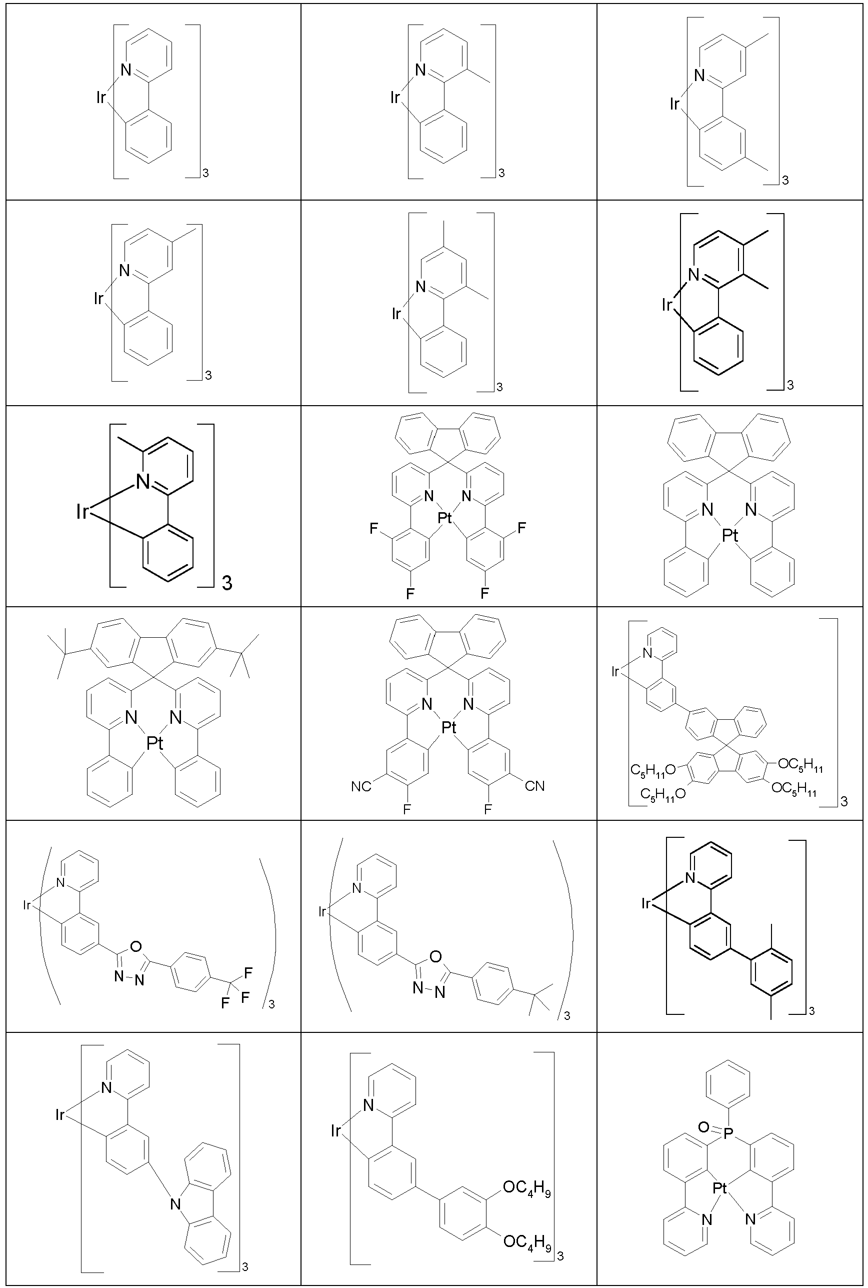

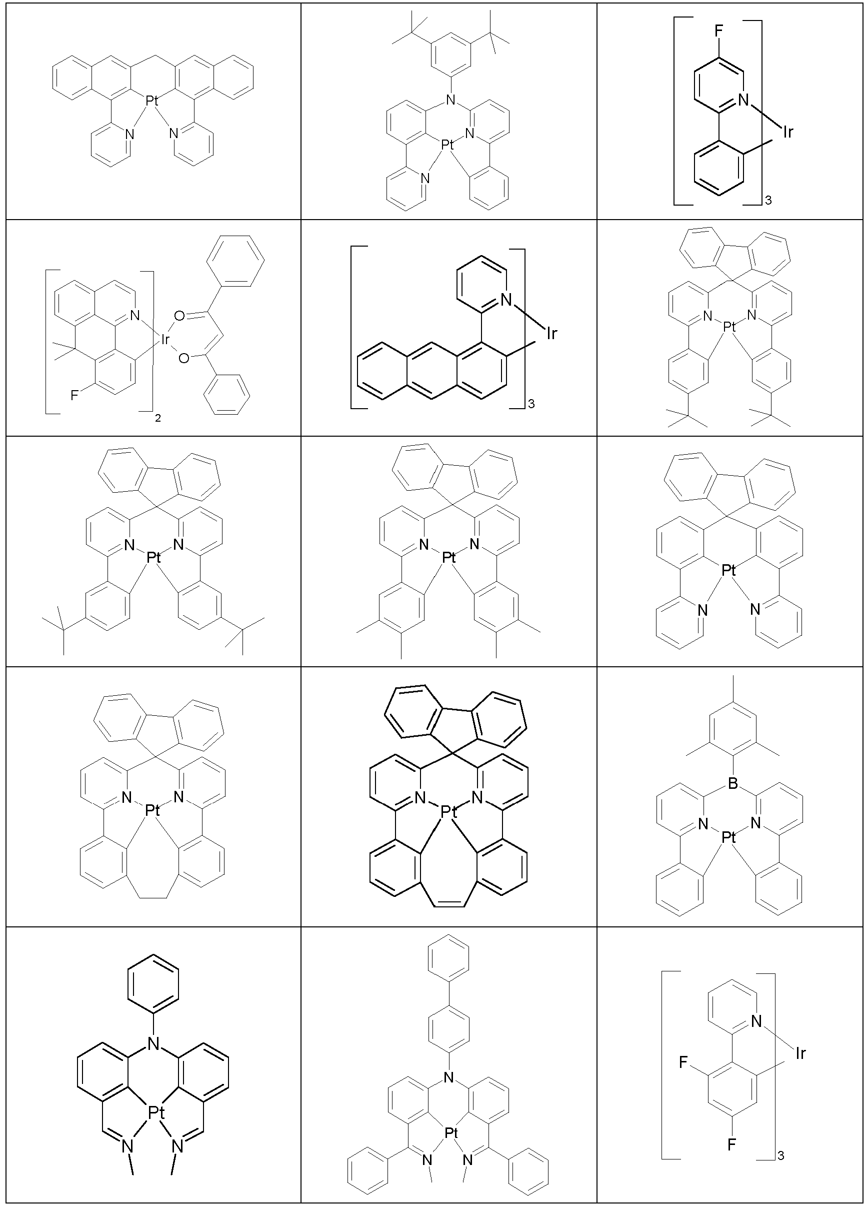

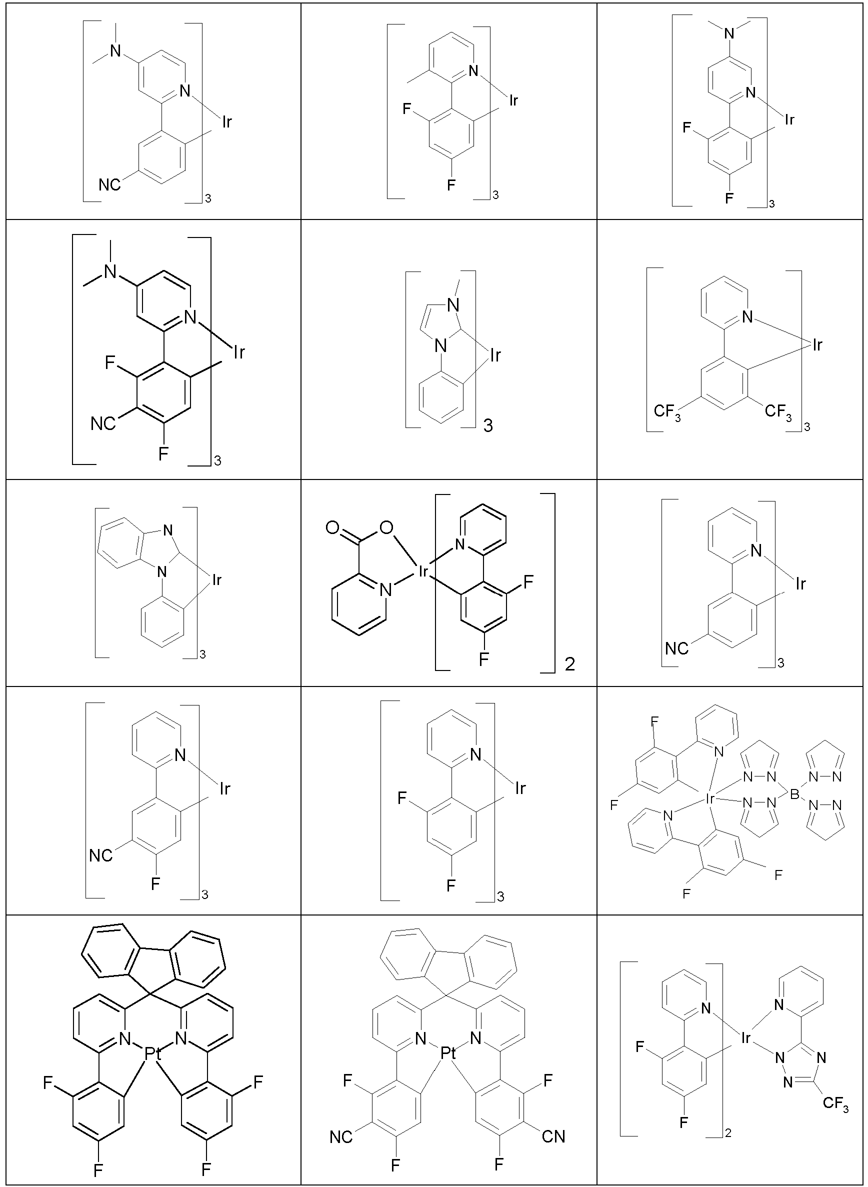

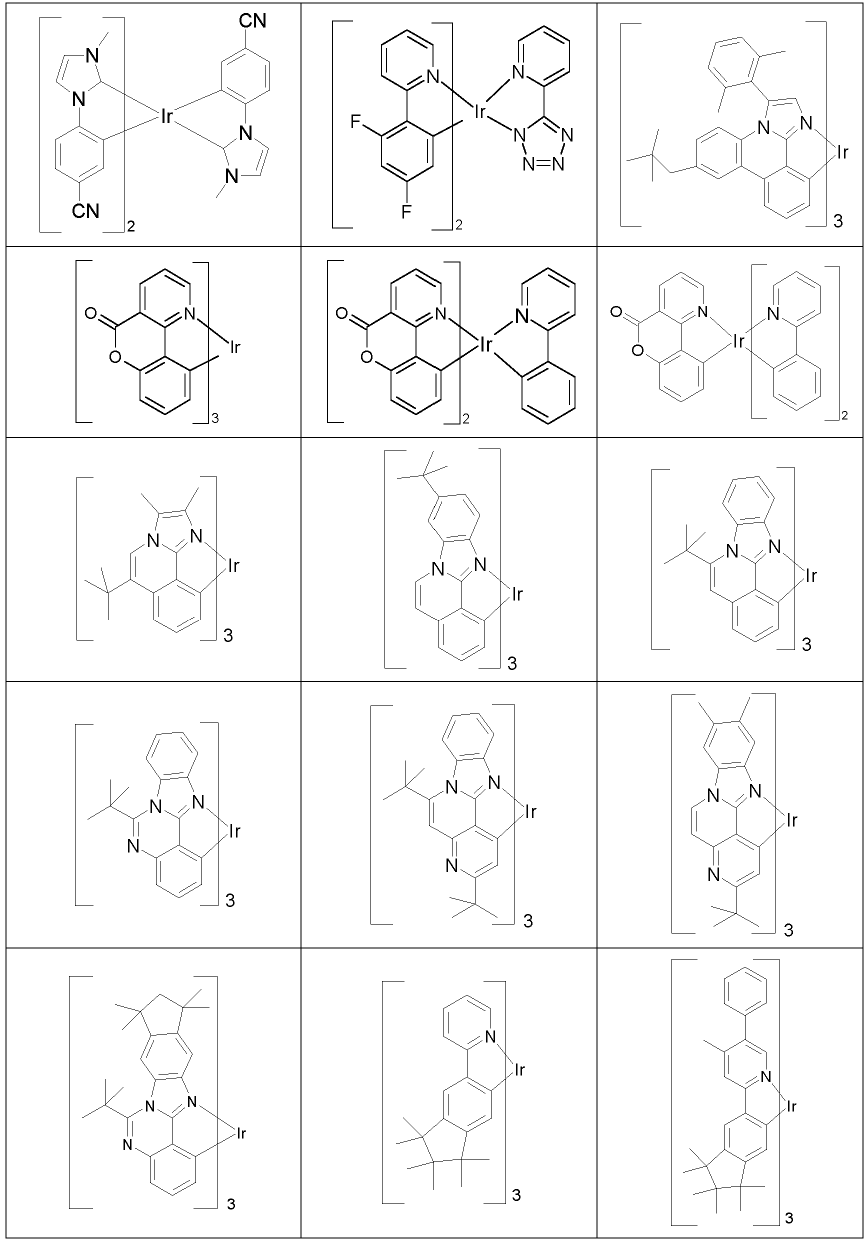

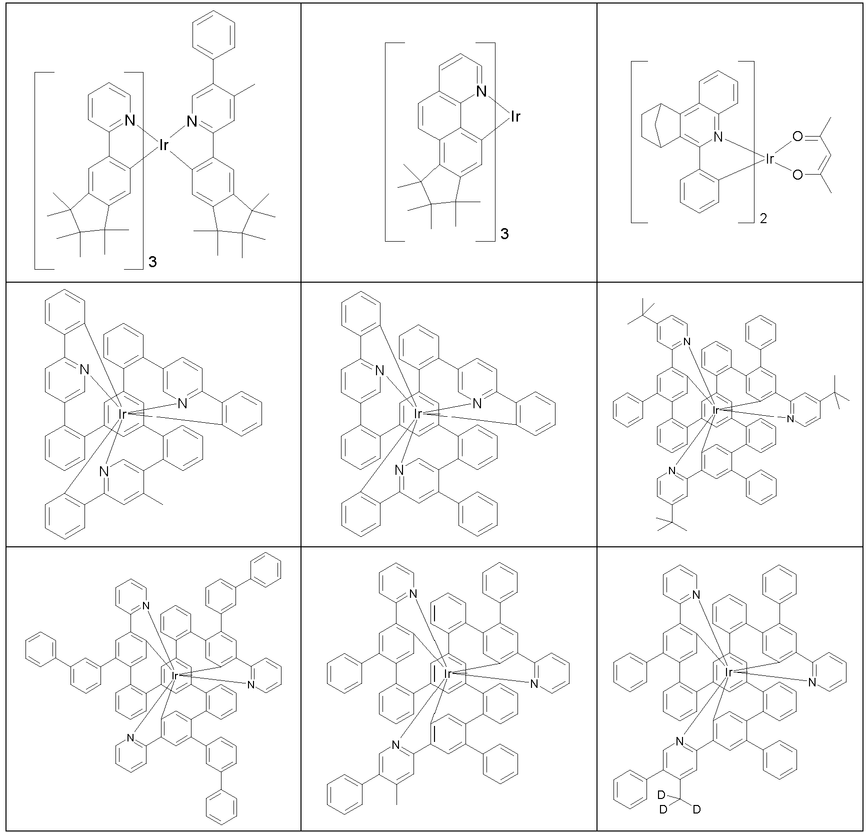

Preferred fluorescent emitting compounds are selected from the class of the arylamines. An arylamine or an aromatic amine in the context of this invention is understood to mean a compound containing three substituted or unsubstituted aromatic or heteroaromatic ring systems bonded directly to the nitrogen. Preferably, at least one of these aromatic or heteroaromatic ring systems is a fused ring system, more preferably having at least 14 aromatic ring atoms. Preferred examples of these are aromatic anthracenamines, aromatic anthracenediamines, aromatic pyrenamines, aromatic pyrenediamines, aromatic chrysenamines or aromatic chrysenediamines. An aromatic anthracenamine is understood to mean a compound in which a diarylamino group is bonded directly to an anthracene group, preferably in the 9 position. An aromatic anthracenediamine is understood to mean a compound in which two diarylamino groups are bonded directly to an anthracene group, preferably in the 9,10 positions. Aromatic pyrenamines, pyrenediamines, chrysenamines and chrysenediamines are defined analogously, where the diarylamino groups are bonded to the pyrene preferably in the 1 position or 1,6 positions. Further preferred emitting compounds are indenofluorenamines or -fluorenediamines, for example according to WO 2006/108497 or WO 2006/122630, benzoindenofluorenamines or - fluorenediamines, for example according to WO 2008/006449, and dibenzoindenofluoreneamines or -diamines, for example according to WO 2007/140847, and the indenofluorene derivatives having fused aryl groups disclosed in WO 2010/012328. Likewise preferred are the pyrenearylamines disclosed in WO 2012/048780 and in WO 2013/185871. Likewise preferred are the benzoindenofluorenamines disclosed in WO 2014/037077, the benzofluorenamines disclosed in WO 2014/106522, the extended benzoindenofluorenes disclosed in WO 2014/111269 and in WO 2017/036574, the phenoxazines disclosed in WO 2017/028940 and in WO 2017/028941, and the fluorene derivatives bonded to furan units or to thiophene units that are disclosed in WO 2016/150544.

Useful matrix materials, preferably for fluorescent emitting compounds, include materials of various substance classes. Preferred matrix materials are selected from the classes of the oligoarylenes (e.g. 2,2',7,7'-tetraphenylspirobifluorene according to EP 676461 or dinaphthylanthracene), especially of the oligoarylenes containing fused aromatic groups, the oligoarylenevinylenes (e.g. DPVBi or spiro-DPVBi according to EP 676461), the polypodal metal complexes (for example according to WO 2004/081017), the hole-conducting compounds (for example according to WO 2004/058911), the electron-conducting compounds, especially ketones, phosphine oxides, sulphoxides, etc. (for example according to WO 2005/084081 and WO 2005/084082), the atropisomers (for example according to WO 2006/048268), the boronic acid derivatives (for example according to WO 2006/117052) or the benzanthracenes (for example according to WO 2008/145239). Particularly preferred matrix materials are selected from the classes of the oligoarylenes comprising naphthalene, anthracene, benzanthracene and/or pyrene or atropisomers of these compounds, the oligoarylenevinylenes, the ketones, the phosphine oxides and the sulphoxides. Very particularly preferred matrix materials are selected from the classes of the oligoarylenes comprising anthracene, benzanthracene, benzophenanthrene and/or pyrene or atropisomers of these compounds. An oligoarylene in the context of this invention shall be understood to mean a compound in which at least three aryl or arylene groups are bonded to one another. Preference is further given to the anthracene derivatives disclosed in WO 2006/097208, WO 2006/131192, WO 2007/065550, WO 2007/110129, WO 2007/065678, WO 2008/145239, WO 2009/100925, WO 2011/054442 and EP 1553154, the pyrene compounds disclosed in EP 1749809, EP 1905754 and US 2012/0187826, the benzanthracenylanthracene compounds disclosed in WO 2015/158409, the indenobenzofurans disclosed in WO 2017/025165, and the phenanthrylanthracenes disclosed in WO 2017/036573.

Preferred matrix materials for phosphorescent emitting compounds are, as well as the compounds of the formula (I), aromatic ketones, aromatic phosphine oxides or aromatic sulphoxides or sulphones, for example according to WO 2004/013080, WO 2004/093207, WO 2006/005627 or WO 2010/006680, triarylamines, carbazole derivatives, e.g. CBP (N,N-biscarbazolylbiphenyl) or the carbazole derivatives disclosed in WO 2005/039246, US 2005/0069729, JP 2004/288381, EP 1205527 or WO 2008/086851, indolocarbazole derivatives, for example according to WO 2007/063754 or WO 2008/056746, indenocarbazole derivatives, for example according to WO 2010/136109, WO 2011/000455 or WO 2013/041176, azacarbazole derivatives, for example according to EP 1617710, EP 1617711, EP 1731584, JP 2005/347160, bipolar matrix materials, for example according to WO 2007/137725, silanes, for example according to WO 2005/111172, azaboroles or boronic esters, for example according to WO 2006/117052, triazine derivatives, for example according to WO 2010/015306, WO 2007/063754 or WO 2008/056746, zinc complexes, for example according to EP 652273 or WO 2009/062578, diazasilole or tetraazasilole derivatives, for example according to WO 2010/054729, diazaphosphole derivatives, for example according to WO 2010/054730, bridged carbazole derivatives, for example according to US 2009/0136779, WO 2010/050778, WO 2011/042107, WO 2011/088877 or WO 2012/143080, triphenylene derivatives, for example according to WO 2012/048781, or lactams, for example according to WO 2011/116865 or WO 2011/137951.

Suitable charge transport materials as usable in the hole injection or hole transport layer or electron blocking layer or in the electron transport layer of the electronic device of the invention are, as well as the compounds of the formula (I), for example, the compounds disclosed in Y. Shirota et al., Chem. Rev. 2007, 107(4), 953-1010, or other materials as used in these layers according to the prior art.

Preferably, the inventive OLED comprises two or more different hole-transporting layers. The compound of the formula (I) may be used here in one or more of or in all the hole-transporting layers. In a preferred embodiment, the compound of the formula (I) is used in exactly one or exactly two hole-transporting layers, and other compounds, preferably aromatic amine compounds, are used in the further hole-transporting layers present. Further compounds which are used alongside the compounds of the formula (I), preferably in hole-transporting layers of the OLEDs of the invention, are especially indenofluorenamine derivatives (for example according to WO 06/122630 or WO 06/100896), the amine derivatives disclosed in EP 1661888, hexaazatriphenylene derivatives (for example according to WO 01/049806), amine derivatives with fused aromatics (for example according to US 5,061,569), the amine derivatives disclosed in WO 95/09147, monobenzoindenofluorenamines (for example according to WO 08/006449), dibenzoindenofluorenamines (for example according to WO 07/140847), spirobifluorenamines (for example according to WO 2012/034627 or WO 2013/120577), fluorenamines (for example according to WO 2014/015937, WO 2014/015938, WO 2014/015935 and WO 2015/082056), spirodibenzopyranamines (for example according to WO 2013/083216), dihydroacridine derivatives (for example according to WO 2012/150001), spirodibenzofurans and spirodibenzothiophenes, for example according to WO 2015/022051, WO 2016/102048 and WO 2016/131521, phenanthrenediarylamines, for example according to WO 2015/131976, spirotribenzotropolones, for example according to WO 2016/087017, spirobifluorenes with meta-phenyldiamine groups, for example according to WO 2016/078738, spirobisacridines, for example according to WO 2015/158411, xanthenediarylamines, for example according to WO 2014/072017, and 9,10-dihydroanthracene spiro compounds with diarylamino groups according to WO 2015/086108.

Very particular preference is given to the use of spirobifluorenes substituted by diarylamino groups in the 4 position as hole-transporting compounds, especially to the use of those compounds that are claimed and disclosed in WO 2013/120577, and to the use of spirobifluorenes substituted by diarylamino groups in the 2 position as hole-transporting compounds, especially to the use of those compounds that are claimed and disclosed in WO 2012/034627.

Materials used for the electron transport layer may be any materials as used according to the prior art as electron transport materials in the electron transport layer. Especially suitable are aluminum complexes, for example Alq₃, zirconium complexes, for example Zrq₄, lithium complexes, for example Liq, benzimidazole derivatives, triazine derivatives, pyrimidine derivatives, pyridine derivatives, pyrazine derivatives, quinoxaline derivatives, quinoline derivatives, oxadiazole derivatives, aromatic ketones, lactams, boranes, diazaphosphole derivatives and phosphine oxide derivatives. Further suitable materials are derivatives of the abovementioned compounds as disclosed in JP 2000/053957, WO 2003/060956, WO 2004/028217, WO 2004/080975 and WO 2010/072300.

Preferred cathodes of the electronic device are metals having a low work function, metal alloys or multilayer structures composed of various metals, for example alkaline earth metals, alkali metals, main group metals or lanthanoids (e.g. Ca, Ba, Mg, Al, In, Mg, Yb, Sm, etc.). Additionally suitable are alloys composed of an alkali metal or alkaline earth metal and silver, for example an alloy composed of magnesium and silver. In the case of multilayer structures, in addition to the metals mentioned, it is also possible to use further metals having a relatively high work function, for example Ag or Al, in which case combinations of the metals such as Ca/Ag, Mg/Ag or Ba/Ag, for example, are generally used. It may also be preferable to introduce a thin interlayer of a material having a high dielectric constant between a metallic cathode and the organic semiconductor. Examples of useful materials for this purpose are alkali metal or alkaline earth metal fluorides, but also the corresponding oxides or carbonates (e.g. LiF, Li₂O, BaF₂, MgO, NaF, CsF, Cs₂CO₃, etc.). It is also possible to use lithium quinolinate (LiQ) for this purpose. The layer thickness of this layer is preferably between 0.5 and 5 nm.

Preferred anodes are materials having a high work function. Preferably, the anode has a work function of greater than 4.5 eV versus vacuum. Firstly, metals having a high redox potential are suitable for this purpose, for example Ag, Pt or Au. Secondly, metal/metal oxide electrodes (e.g. Al/Ni/NiOₓ, Al/PtOₓ) may also be preferred. For some applications, at least one of the electrodes has to be transparent or partly transparent in order to enable the irradiation of the organic material (organic solar cell) or the emission of light (OLED, O-laser). Preferred anode materials here are conductive mixed metal oxides. Particular preference is given to indium tin oxide (ITO) or indium zinc oxide (IZO). Preference is further given to conductive doped organic materials, especially conductive doped polymers. In addition, the anode may also consist of two or more layers, for example of an inner layer of ITO and an outer layer of a metal oxide, preferably tungsten oxide, molybdenum oxide or vanadium oxide.

The device is structured appropriately (according to the application), contact-connected and finally sealed, in order to rule out damaging effects by water and air.

In a preferred embodiment, the electronic device is characterized in that one or more layers are coated by a sublimation process. In this case, the materials are applied by vapour deposition in vacuum sublimation systems at an initial pressure of less than 10⁻⁵ mbar, preferably less than 10⁻⁶ mbar. In this case, however, it is also possible that the initial pressure is even lower, for example less than 10⁻⁷ mbar.

Preference is likewise given to an electronic device, characterized in that one or more layers are coated by the OVPD (organic vapour phase deposition) method or with the aid of a carrier gas sublimation. In this case, the materials are applied at a pressure between 10⁻⁵ mbar and 1 bar. A special case of this method is the OVJP (organic vapour jet printing) method, in which the materials are applied directly by a nozzle and thus structured (for example M. S. Arnold et al., Appl. Phys. Lett. 2008, 92, 053301).

Preference is additionally given to an electronic device, characterized in that one or more layers are produced from solution, for example by spin-coating, or by any printing method, for example screen printing, flexographic printing, nozzle printing or offset printing, but more preferably LITI (light-induced thermal imaging, thermal transfer printing) or inkjet printing. For this purpose, soluble compounds of formula (I) are needed. High solubility can be achieved by suitable substitution of the compounds.

It is further preferable that an electronic device of the invention is produced by applying one or more layers from solution and one or more layers by a sublimation method.

According to the invention, the electronic devices comprising one or more compounds of formula (I) can be used in displays, as light sources in lighting applications and as light sources in medical and/or cosmetic applications (e.g. light therapy).

### Examples

### A) Synthesis examples

The following syntheses are carried out under a protective-gas atmosphere, unless indicated otherwise. The starting materials can be purchased from ALDRICH or ABCR. The numbers in square brackets in the case of the starting materials known from the literature are the corresponding CAS numbers.

### Example 1

### Synthesis of 5-bromo-2-chloro-9,9'-spirobifluorene 1a

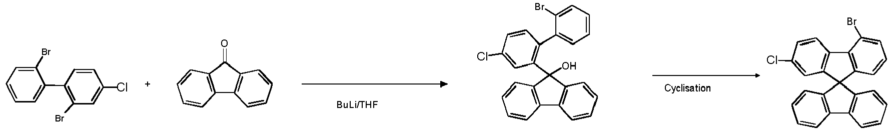

A solution of 2, 2'-dibromo-4-chloro-biphenyl (84 g, 239 mmol) in THF (200ml) is treated with 109 mL of n-BuLi (2,2 M in hexane, 239 mmol) under argon at -78 °C. The mixture is stirred for 30 minutes. A solution of fluoren-9-one (44 g, 239 mmol) in 150 mL THF is added dropwise. The reaction proceeds at -78 °C for 30 minutes and then is stirred at room temperature overnight. The reaction is quenched with water and the solid is filtered. Without further purification, a solution of the alcohol in 966 mL toluene and 2,9 g p-toluenesulfonic acid is refluxed overnight . After cooling, the organic phase is washed with water and the solvent is removed under vacuum. The product is isolated in the form of a white solid (60 g, 91% of theory).

The synthesis of further halogenated spirobifluorene derivatives is carried out analogously:

| | Bromo-biphenyl | Aryl-fluorenone | Product | Yield |
|---|---|---|---|---|
| **1b** | 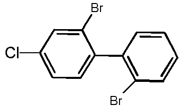 [1801701-07-4] | 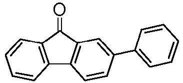 [3096-49-9] | 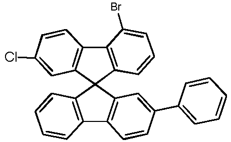 | 86% |
| **1c** | 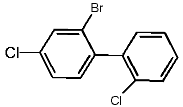 [1801752-89-5] | 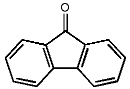 | 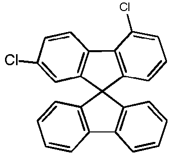 | 84% |
| **1d** | 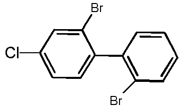 [1801701-07-4] | 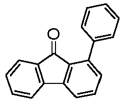 [486-25-9] | 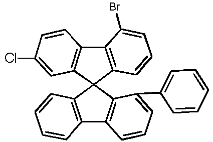 | 90% |
| **1f** | 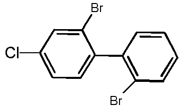 [1801701-07-4] | 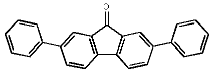 [115033-91-5] | 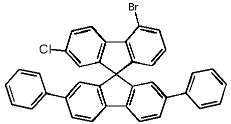 | 90% |
| **1g** | 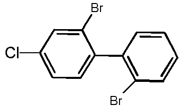 [1801701-07-4] | 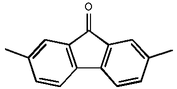 [2840-49-5] | 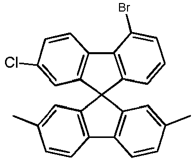 | 66% |
| **1h** | 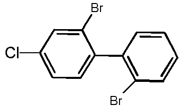 [1801701-07-4] | 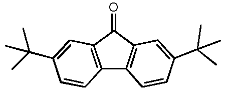 [58775-13-6] | 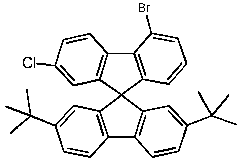 | 81% |
| **1i** | 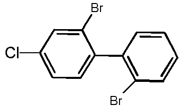 [1801701-07-4] | 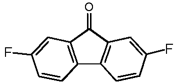 [24313-53-9] | 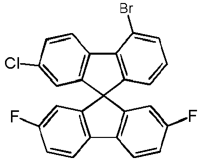 | 77% |
| **1j** | 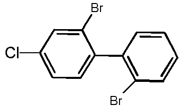 [1801701-07-4] | 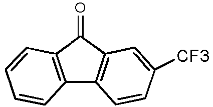 [22052-25-1] | 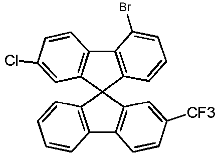 | 85% |
| **1k** | 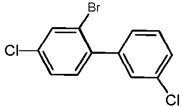 | 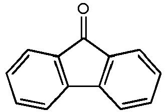 | 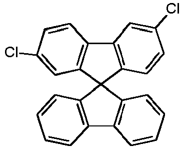 | 81% |
| | [1801752-88-4] | | 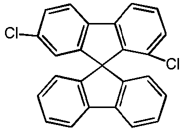 Each of the two isomers will be isolated | |
| **1l** | 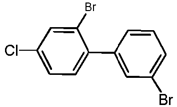 | 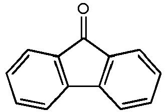 | 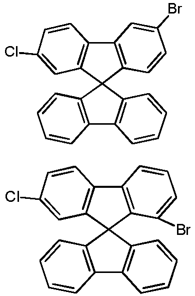 Each of the two isomers will be isolated | 80% |

### Synthesis of 2-chloro-5-phenyl-9,9'-spirobifluorene 2a

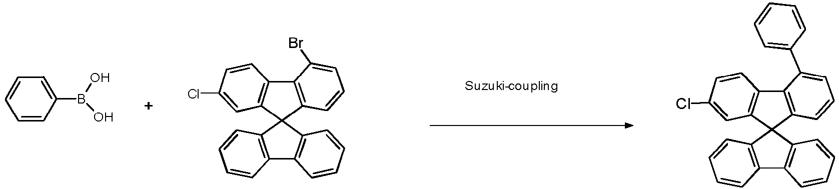

31,5 g (251 mmol) of of phenyl-boronic acid, 110 g (251 mmol) of 5-bromo-2-chloro-9,9'-spirobifluorene, 9,9 g (8,5 mmol) of Pd(P(Ph₃))₄, and 66,8 g (627 mmol) of Na₂CO₃ are dissolved in 903 mL of water, 278 mL of ethanol and 1,9 L of toluene. The reaction mixture is refluxed and agitated under an argon atmosphere for 12 hours and after cooling to room temperature, the mixture is filtered through Celite. The filtrate is evaporated *in vacuo,* and the residue is crystallised from heptane. The product is isolated in the form of an off-white solid (100 g 94% of theory).

The following compounds are synthesized analogously:

| Ex. | Halogenated spiro | Boronic acid | Product | Yield |
|---|---|---|---|---|
| **2b** | 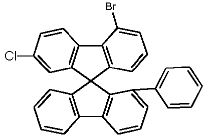 | 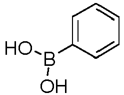 | 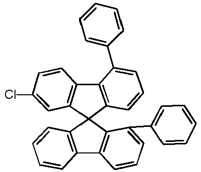 | 86% |
| **2c** | 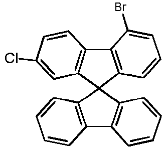 | 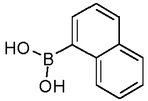 | 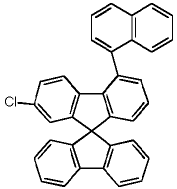 | 82% |
| **2d** | 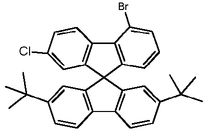 | 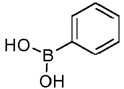 | 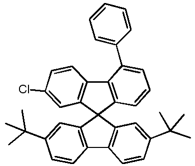 | 43% |
| **2e** | 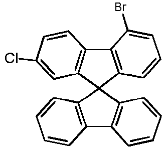 | 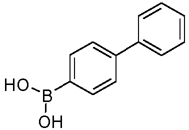 | 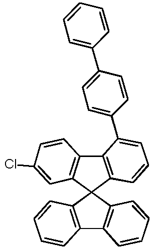 | 92% |
| **2f** | 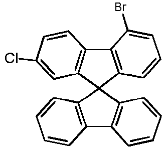 | 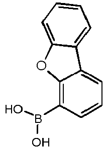 | 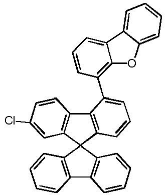 | 90% |
| **2g** | 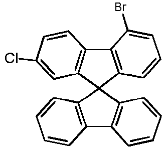 | 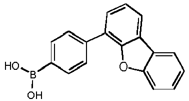 | 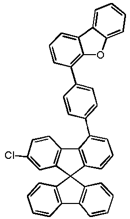 | 85% |
| **2h** | 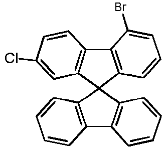 | 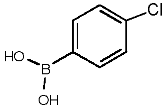 | 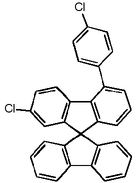 | 55% |
| **2i** | 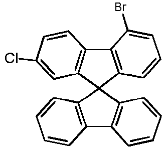 | 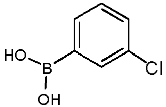 | 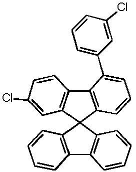 | 54% |
| **2j** | 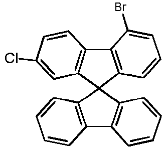 | 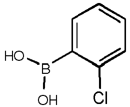 | 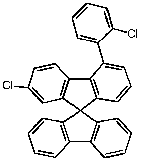 | 40% |
| **2k** | 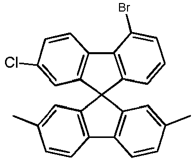 | 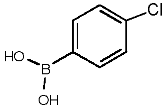 | 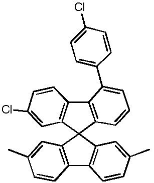 | 50% |
| **2l** | 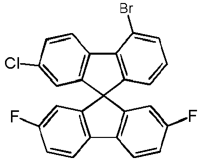 | 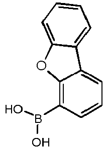 | 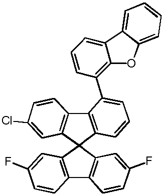 | 80% |
| **2m** | 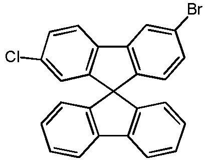 | 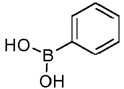 | 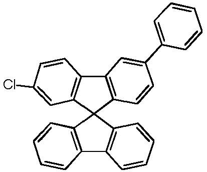 | 85% |
| **2n** | 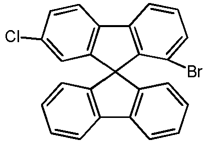 | 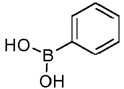 | 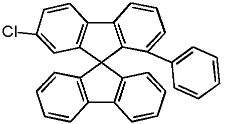 | 60% |

### Synthesis of N-{[1,1'-biphenyl]-4-yl}-N-(9,9-dimethyl-9H-fluoren-2-yl)-5-phenyl-9,9'-spirobi[fluorene]-2-amine 3a

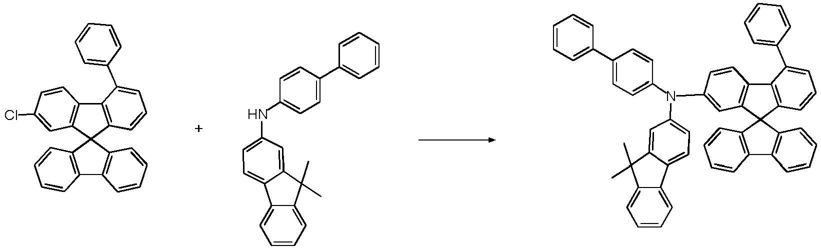

Tri-tert-butylphosphine (1,32 mL of a 1.0 M solution in toluene, 1,32 mmol), Pd₂(dba)₃ (607 mg, 0,66 mmol) and sodium tert-butoxide (4,8 g, 49,7 mmol) are added to a solution of biphenyl-4-yl-(9,9-dimethyl-9H-fluoren-2-yl)-amine (11,9 g, 33,1 mmol) and 2'-chloro-5'-phenyl-9,9'-spirobifluorene (14,7 g, 33,1 mmol) in degassed toluene (500 ml), and the mixture is heated under reflux for 6 h. The reaction mixture is cooled to room temperature, extended with toluene and filtered through Celite. The filtrate is evaporated *in vacuo,* and the residue is crystallised from toluene/heptane. The crude product is extracted in a Soxhlet extractor (toluene) and purified by zone sublimation *in vacuo* twice. The product is isolated in the form of an off-white solid (9,5 g, 38% of theory).

The following compounds are obtained analogously:

| Ex. | Halogenated spiro | Amine | Product | Yield |
|---|---|---|---|---|
| **3b** | 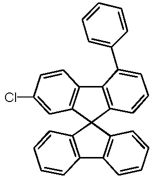 | 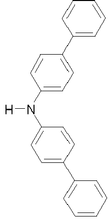 [102113-98-4] | 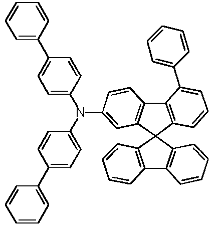 | 43% |
| **3c** | 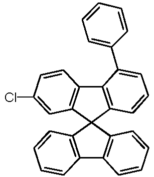 | 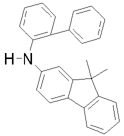 [1198395-24-2] | 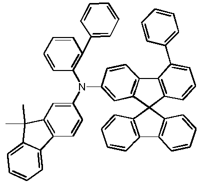 | 85% |
| **3d** | 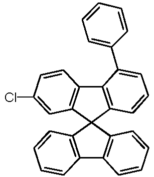 | 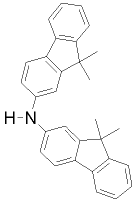 [500717-23-7] | 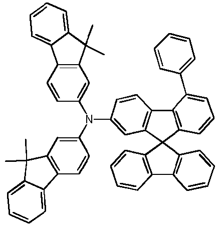 | 55% |
| **3e** | 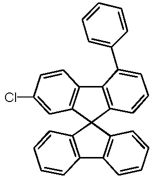 | 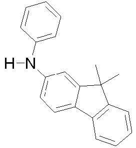 [355832-04-1] | 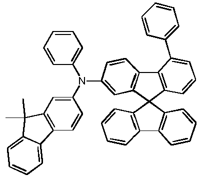 | 64% |
| **3f** | 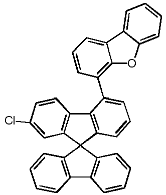 | 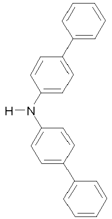 [102113-98-4] | 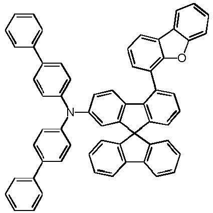 | 56% |
| **3g** | 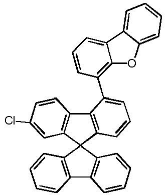 | 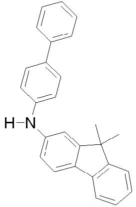 [897671-69-1] | 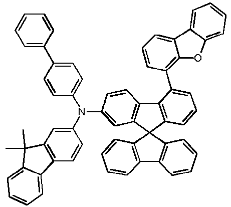 | 73% |
| **3h** | 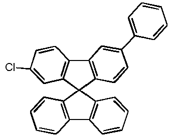 | 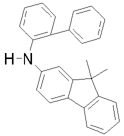 [1198395-24-2] | 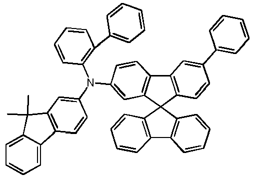 | 65% |
| **3i** | 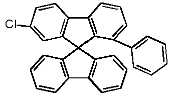 | 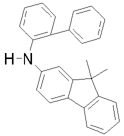 [1198395-24-2] | 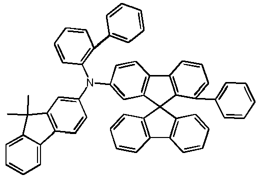 | 67% |
| **3j** | 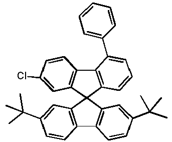 | 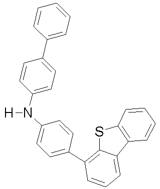 [1431879-08-1] | 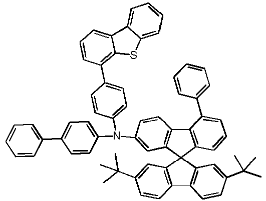 | 66% |
| **3k** | 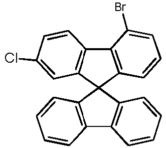 | 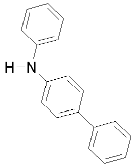 [32228-99-2] | 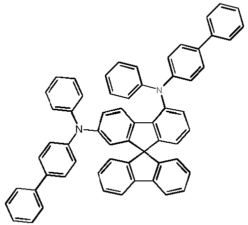 | 50% |
| **3l** | 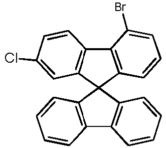 | 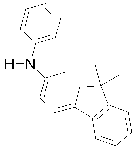 [355832-04-1] | 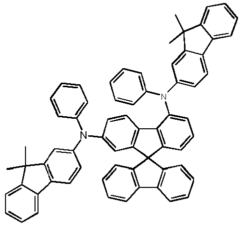 | 62% |
| **3m** | 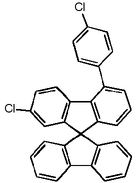 | 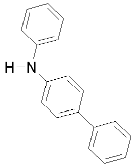 [32228-99-2] | 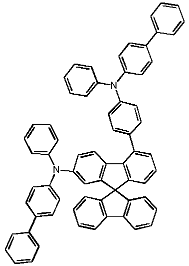 | 73% |
| **3n** | 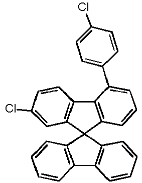 | 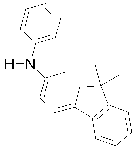 [355832-04-1] | 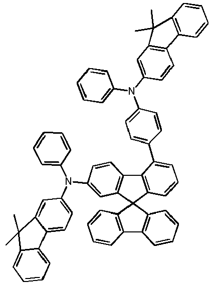 | 69% |
| **3o** | 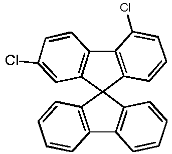 | 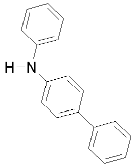 [32228-99-2] | 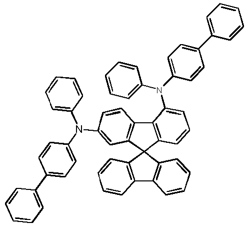 | 57% |
| **3p** | 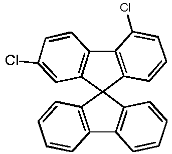 | 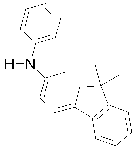 [355832-04-1] | 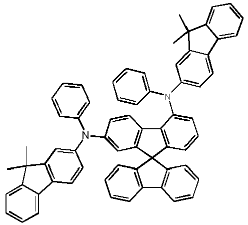 | 65% |
| **3q** | 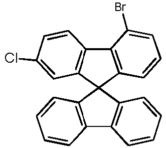 | 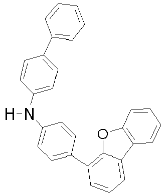 [955959-89-4] | 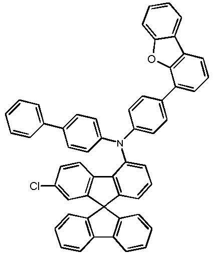 | 71% |
| **3r** | 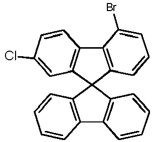 | 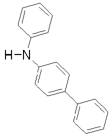 | 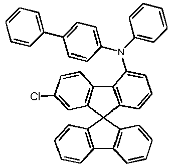 | 71% |
| **3s** | 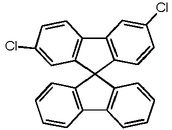 | 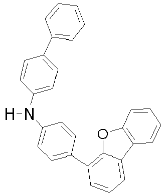 [955959-89-4] | 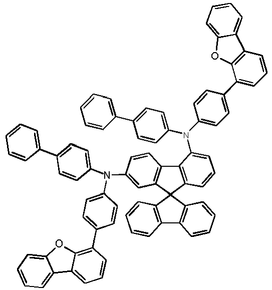 | 72% |
| **3t** | 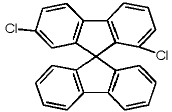 | 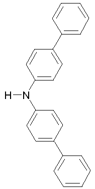 [102113-98-4] | 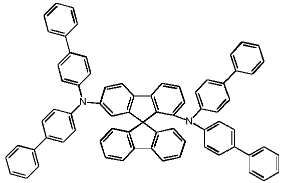 | 62% |
| **3u** | 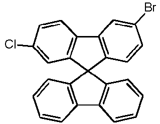 | 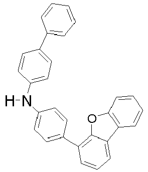 [955959-89-4] | 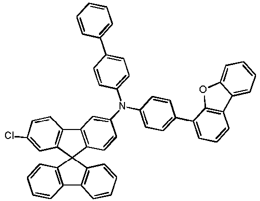 | 45% |
| **3v** | 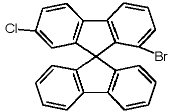 | 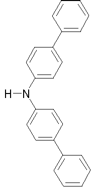 [102113-98-4] | 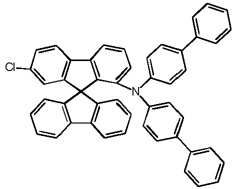 | 66% |
| **3w** | 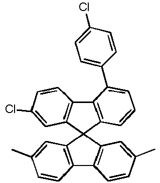 | 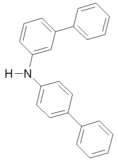 [570391-47-8] | 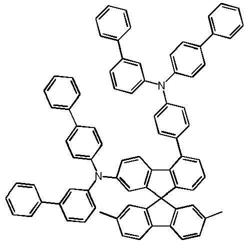 | 66% |
| **3x** | 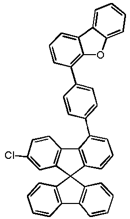 | 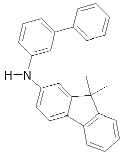 [1372778-66-9] | 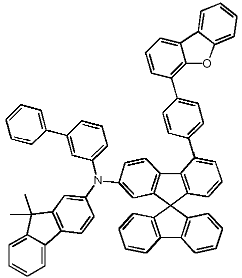 | 71% |
| **3y** | 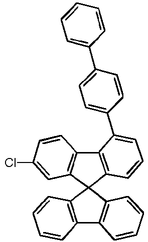 | 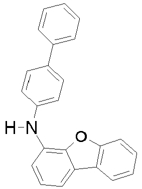 [1318338-47-4] | 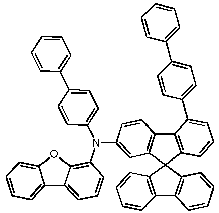 | 49% |
| **3z** | 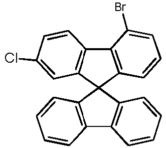 | 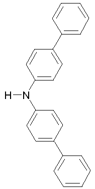 [102113-98-4] | 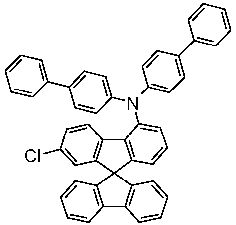 | 65% |
| **3aa** | 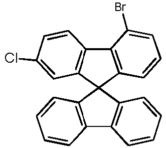 | 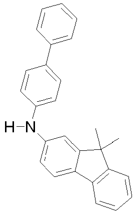 [897671-69-1] | 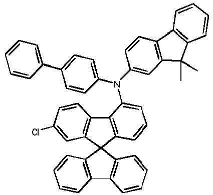 | 73% |
| **3ab** | 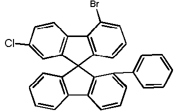 | 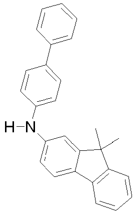 [897671-69-1] | 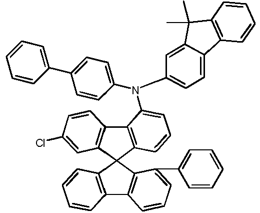 | 70% |
| **3ac** | 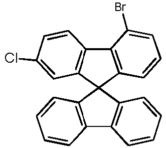 | 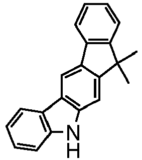 1257220-47-5 | 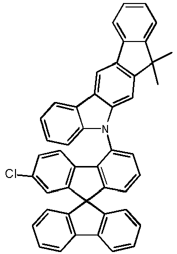 | 80% |
| **3ad** | 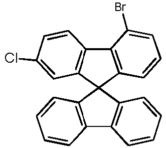 | 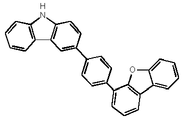 | 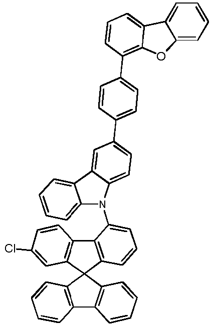 | 75% |
| **3ae** | 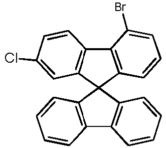 | 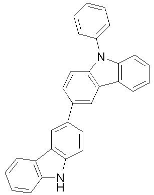 1060735-14-9 | 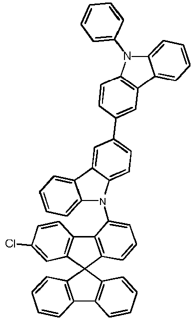 | 60% |
| **3af** | 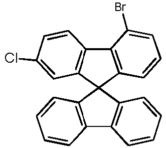 | 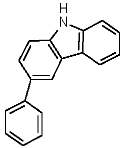 103012-26-6 | 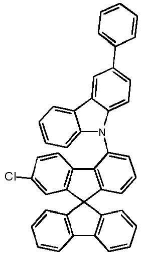 | 85% |
| **3ag** | 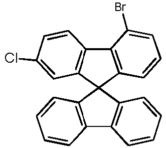 | 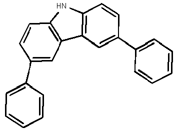 | 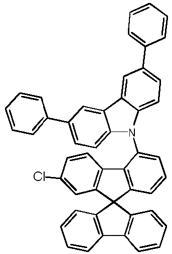 | 80% |
| **3ah** | 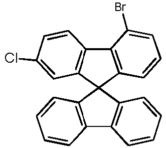 | 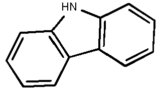 | 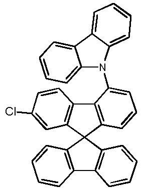 | 76% |
| **3ai** | 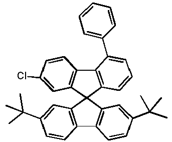 | 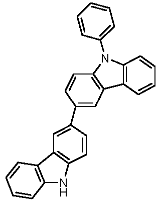 | 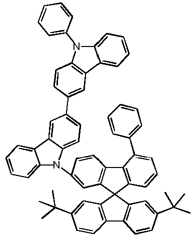 | 67% |
| **3aj** | 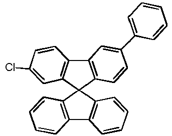 | 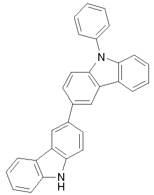 | 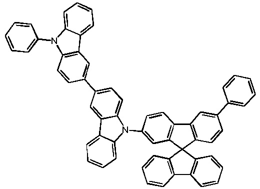 | 50% |
| **3ak** | 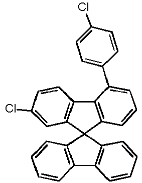 | 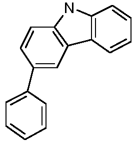 | 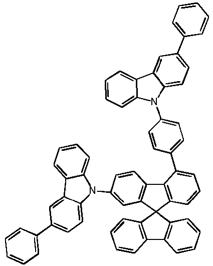 | 40% |
| **3al** | 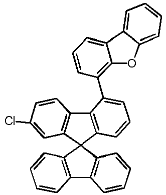 | 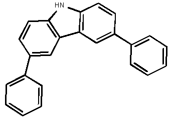 | 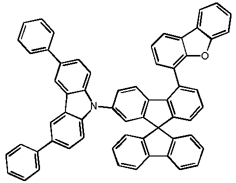 | 50% |
| **3am** | 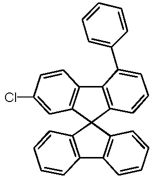 | 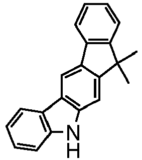 | 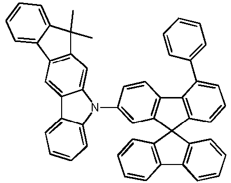 | 38% |
| **3an** | 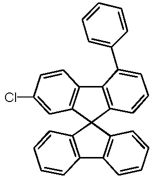 | 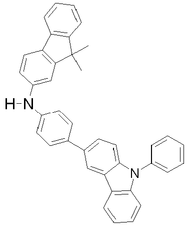 [1354653-33-0] | 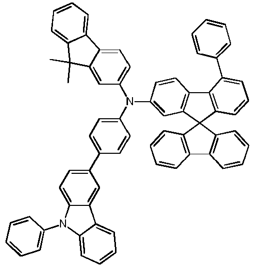 | 35% |
| **3ao** | 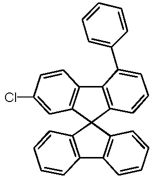 | 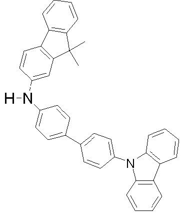 [1267248-54-3] | 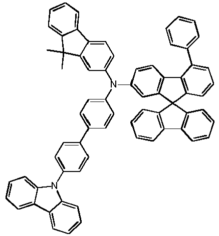 | 45% |
| **3ap** | 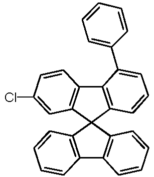 | 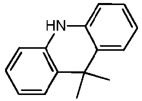 | 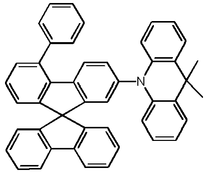 | 50% |
| **3aq** | 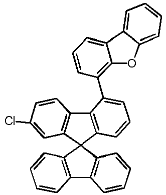 | 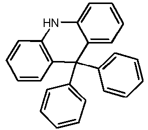 | 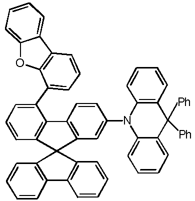 | 55% |
| **3ar** | 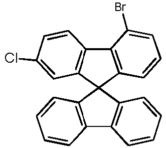 | 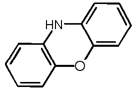 | 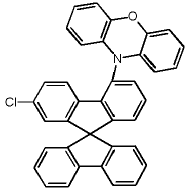 | 60% |
| **3as** | 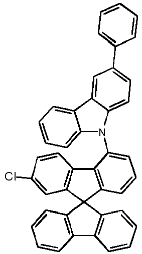 | 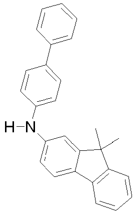 [897671-69-1] | 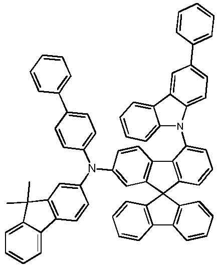 | 55% |
| **3at** | 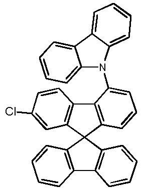 | 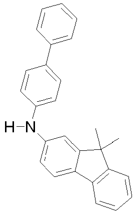 [897671-69-1] | 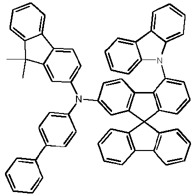 | 45% |
| **3au** | 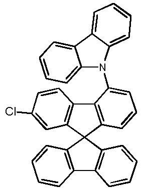 | 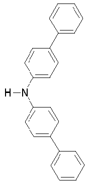 [102113-98-4] | 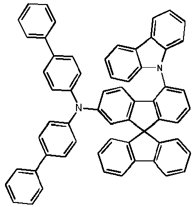 | 52% |
| **3av** | 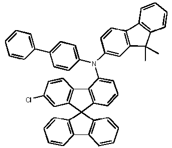 | 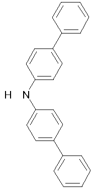 [102113-98-4] | 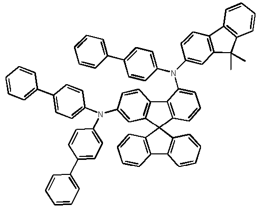 | 47% |
| **3aw** | 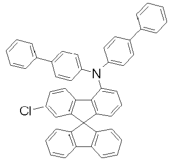 | 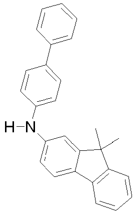 [897671-69-1] | 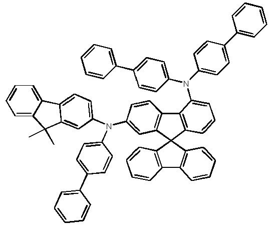 | 51% |
| **3ax** | 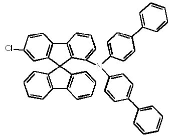 | 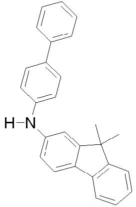 [897671-69-1] | 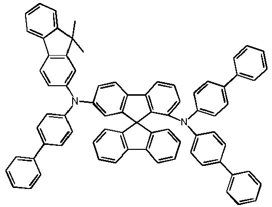 | 48% |
| **3ay** | 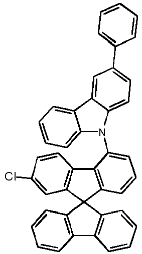 | 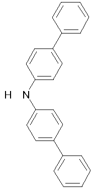 [102113-98-4] | 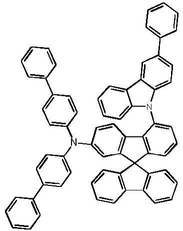 | 46% |
| **3az** | 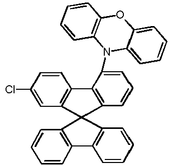 | 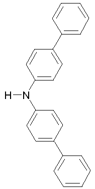 [102113-98-4] | 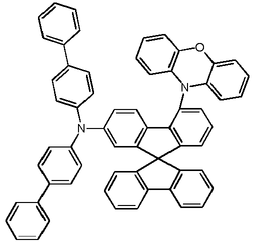 | 38% |
| **3ba** | 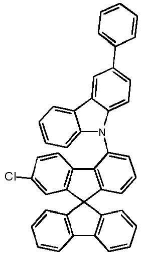 | 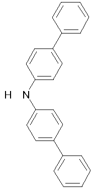 [102113-98-4] | 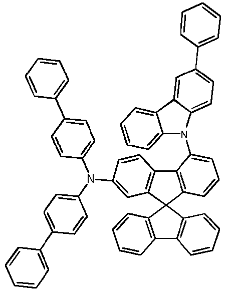 | 55% |

### Synthesis of N-{[1,1'-biphenyl]-4-yl}-9,9-dimethyl-N-(4-{4-phenyl-9,9'-spirobi[fluorene]-7-yl}phenyl)-9H-fluoren-2-amine 4a

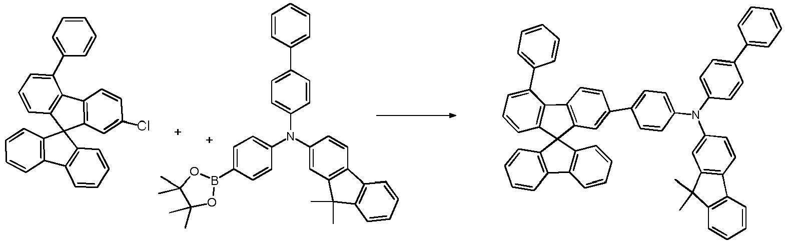

59.1 g (101.8 mmol) of Biphenyl-4-yl-(9,9-dimethyl-9H-fluoren-2-yl (4,4,5,5-tetramethyl-[1,3,2]dioxaborolan-2-yl)-phenyl]-amine, 43,5 g (101.8 mmol) of 2-chloro-5-phenyl-9,9'-spirobifluorene, 3.88 g (5.14 mmol) of PdCl₂(Cy)₃, 31.2 g (205.6 mmol) of cesium fluoride are dissolved in 800 mL of toluene. The reaction mixture is refluxed and agitated under an argon atmosphere for 12 hours and after cooling to room temperature, the mixture is filtered through Celite. The filtrate is evaporated *in vacuo,* and the residue is crystallised from heptane. The crude product is extracted in a Soxhlet extractor (toluene) and purified by zone sublimation *in vacuo* twice. The product is isolated in the form of a white solid (42 g, 51% of theory).

The following compounds are synthesized analogously:

| Ex. | Halogenated spiro | Amine | Product | Yield |
|---|---|---|---|---|
| **4b** | 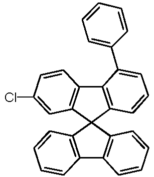 | 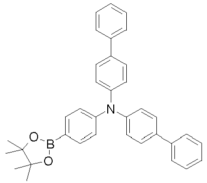 | 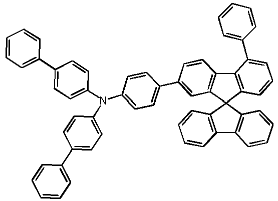 | 50% |
| **4c** | 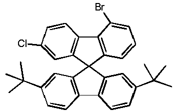 | 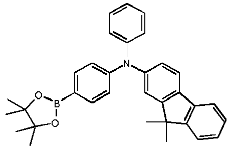 | 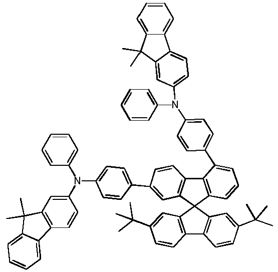 | 55% |
| **4d** | 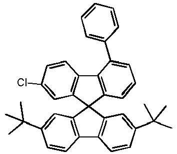 | 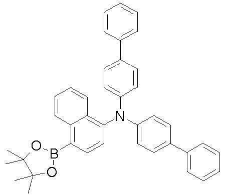 | 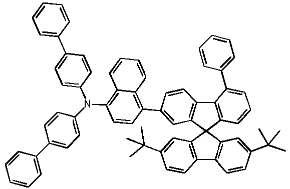 | 60% |
| **4e** | 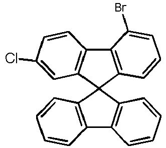 | 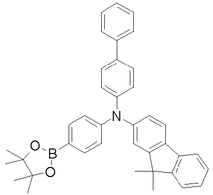 | 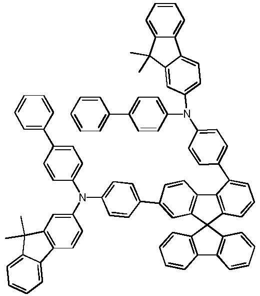 | 63% |
| **4f** | 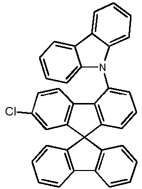 | 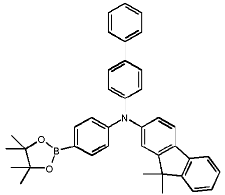 | 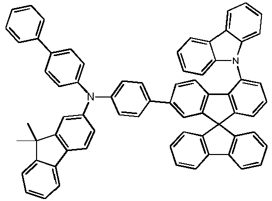 | 70% |
| **4g** | 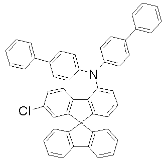 | 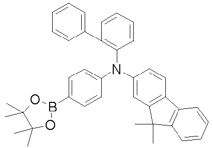 | 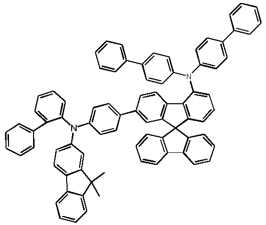 | 75% |
| **4h** | 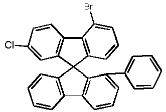 | 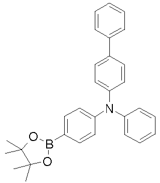 | 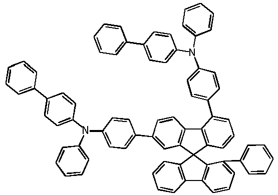 | 55% |
| **4i** | 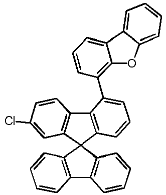 | 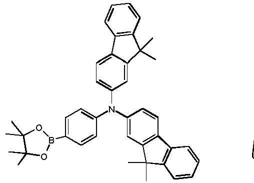 | 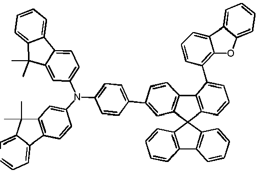 | 64% |
| **4j** | 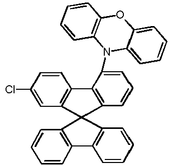 | 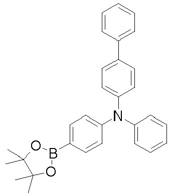 | 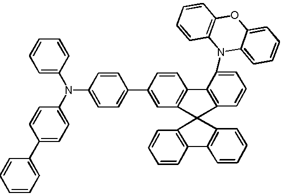 | 60% |
| **4k** | 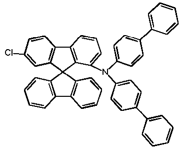 | 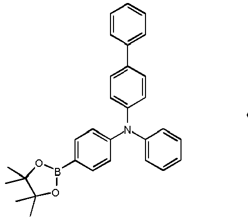 | 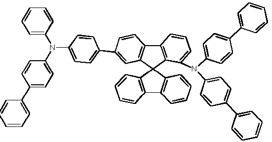 | 59% |
| **4l** | 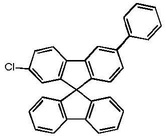 | 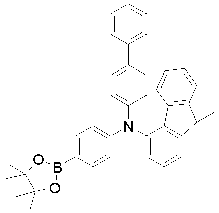 | 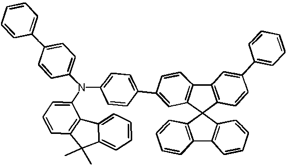 | 75% |
| **4m** | 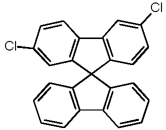 | 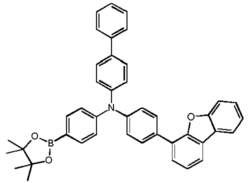 | 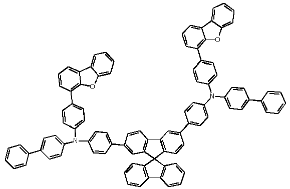 | 81% |
| **4n** | 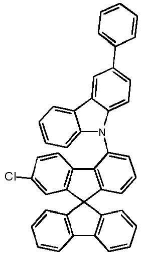 | 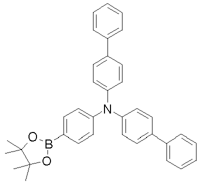 | 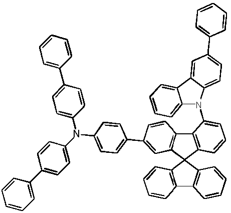 | 85% |
| **4o** | 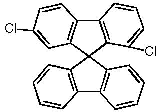 | 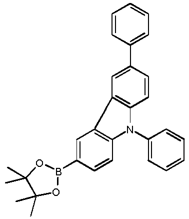 | 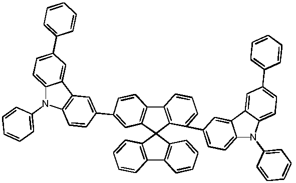 | 77% |
| **4p** | 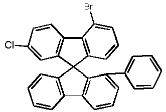 | 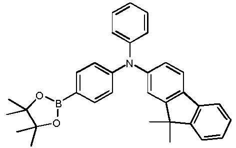 | 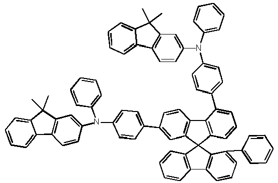 | 62% |
| **4q** | 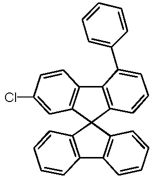 | 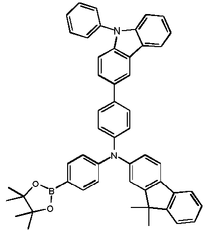 | 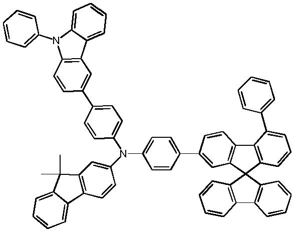 | 60% |
| **4r** | 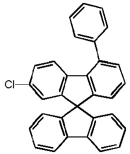 | 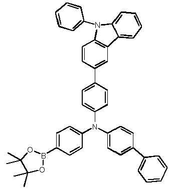 | 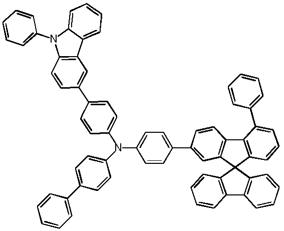 | 72% |
| **4s** | 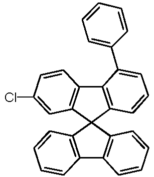 | 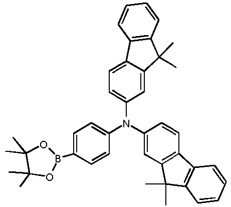 | 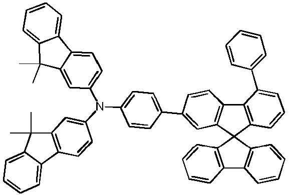 | 45% |

### B) Device Examples

### 1) General Procedure

OLEDs comprising compounds according to the present application, and OLEDs comprising reference compounds are prepared by the following general process: The substrates used are glass plates coated with structured ITO (indium tin oxide) in a thickness of 50nm. The OLEDs basically have the following layer structure: substrate / hole-injection layer (HIL) / hole-transport layer (HTL) / electron-blocking layer (EBL) / emission layer (EML) / electron-transport layer (ETL) / electron-injection layer (EIL) and finally a cathode. The cathode is formed by an aluminium layer with a thickness of 100nm. The specific device setup of the OLEDs is shown in Table 1, and the materials required for the production of the OLEDs are shown in Table 3.

All materials are applied by thermal vapour deposition in a vacuum chamber. The emission layer here always consists of at least one matrix material (host material) and an emitting dopant (emitter), which is admixed with the matrix material or matrix materials in a certain proportion by volume by coevaporation. An expression such as H1:SEB (5%) here means that material H1 is present in the layer in a proportion by volume of 95% and SEB is present in the layer in a proportion by volume of 5%. Analogously, other layers may also consist of a mixture of two or more materials.

The OLEDs are characterised by standard methods. For this purpose, the electroluminescence spectra and the external quantum efficiency (EQE, measured in per cent) as a function of the luminous density, calculated from current/voltage/luminous density characteristic lines (IUL characteristic lines) assuming Lambert emission characteristics, and the lifetime are determined. The expression EQE @ 10mA/cm² denotes the external quantum efficiency at an operating current density of 10mA/cm². LT80 @ 60mA/cm2 is the lifetime until the OLED has dropped from its initial luminance of i.e. 5000cd/m² to 80% of the initial intensity, i.e. to 4000cd/m² without using any acceleration factor. The data for the various OLEDs containing inventive and comparative materials are summarised in Table 2.

In particular, compounds according to the invention are suitable as HIL, HTL, or EBL materials, or as matrix materials in the EML in OLEDs. They are suitable for use as a single material in a layer, but also for use as a mixed component in HIL, HTL, EBL or within the EML.

### 2) Examples for use of compounds according to the application in HIL, HTL and EBL of OLEDs

Table 2 shows the performance data which is obtained with the specific OLED examples shown in Table 1. OLEDs C1 and C2 are reference examples, which comprise the prior art compounds HTM-b and EBM. OLEDs E1, E2 and E3 are OLEDs according to the present application, which comprise the inventive compounds HTM-1, HTM-2 and HTM-3. Compared with the OLEDs according to the prior art (C1 to C2), the samples comprising the compounds according to the invention (E1 to E3) exhibit better performance both in singlet blue devices (C1 compared to E1 and E3) and also in triplet green devices (C2 compared to E2).

It can be shown, that lifetime of device E1 is better than the reference example C1. This shows the improved performance of the compound HTM-1, compared to the reference material HTM-b. Similarly, lifetime of device E3 is better than the one of the device C1. This shows the improved performance of the compound HTM-3, compared to the reference material HTM-b.

Finally, device E2 shows better lifetime than the reference example C2. This shows the improved performance of the compound HTM-2, compared to the reference compound EBM.

| **Table 1: Device Setup** | | | | | | |
|---|---|---|---|---|---|---|
| ***Ex.*** | ***HIL*** | ***HTL*** | ***EBL*** | ***EML*** | ***ETL*** | ***EIL*** |
| | Thickness / nm | Thickness / nm | Thickness / nm | Thickness / nm | Thickness / nm | Thickness / nm |
| C1 | HTM-b: p-doped(5%) 20 nm | HTM-b 180 nm | EBM 10 nm | H:SEB(5%) 20 nm | ETM: LiQ(50%) 30 nm | LiQ 1 nm |
| C2 | HTM-a: p-doped(5%) 20 nm | HTM-a 220 nm | EBM 10 nm | TMM-1: TMM-2(28%):TEG(12%) 30 nm | ETM: LiQ(50%) 30 nm | LiQ 1 nm |
| E1 | HTM-1: p-doped(5%) 20 nm | HTM-1 180 nm | EBM 10 nm | H:SEB(5%) 20 nm | ETM: LiQ(50%) 30 nm | LiQ 1 nm |
| E2 | HTM-a: p-doped(5%) 20 nm | HTM-a 220 nm | HTM-2 10 nm | TMM-1: TMM-2(28%):TEG(12%) 30 nm | ETM: LiQ(50%) 30 nm | LiQ 1 nm |
| E3 | HTM-3: p-doped(5%) 20 nm | HTM-3 180 nm | EBM 10 nm | H:SEB(5%) 20 nm | ETM: LiQ(50%) 30 nm | LiQ 1 nm |

| **Table 2: Data for the OLEDs** | | | |
|---|---|---|---|
| | U | EQE | LT80 @ 60/40* mA/cm² |
| | [V] | [%] | [h] |
| C1 | 4.3 | 8.5 | 130 |
| C2 | 3.8 | 17.7 | 270* |
| E1 | 3.9 | 8.6 | 180 |
| E2 | 3.8 | 16.0 | 320* |
| E3 | 4.1 | 9.0 | 170 |

### 3) Comparison between an OLED comprising the compound HTM-1 according to the invention, and an OLED comprising the compound HTM-c, in the HIL and HTL of a singlet blue device

The two OLEDs are prepared according to the general process described above under 1).

The stack structures are shown in Table 1b below:

| **Table 1b: Device Setup** | | | | | | |
|---|---|---|---|---|---|---|
| ***Ex.*** | ***HIL*** | ***HTL*** | ***EBL*** | ***EML*** | ***ETL*** | ***EIL*** |
| | Thickness / nm | Thickness / nm | Thickness / nm | Thickness / nm | Thickness / nm | Thickness / nm |
| C3 | HTM-c: p-doped(5%) 20 nm | HTM-c 180 nm | EBM 10 nm | H:SEB(5%) 20 nm | ETM: LiQ(50%) 30 nm | LiQ 1 nm |
| E4 | HTM-1: p-doped(5%) 20 nm | HTM-1 180 nm | EBM 10 nm | H:SEB(5%) 20 nm | ETM: LiQ(50%) 30 nm | LiQ 1 nm |

While the operating voltage and the lifetime remain similar, a strong increase in EQE is found for the OLED E4 comprising the compound according to the invention HTM-1, compared to the OLED C3 comprising the comparative compound HTM-c. OLED E4 has an EQE of 9.1%, whereas OLED C3 has an EQE of 7.9 %.

### 4) Comparison between an OLED comprising the compound HTM-1 according to the invention, and an OLED comprising the compound HTM-c, in the EBL of a triplet green device

The two OLEDs are prepared according to the general process described above under 1).

The stack structures are shown in Table 1c below:

| **Table 1c: Device Setup** | | | | | | | |
|---|---|---|---|---|---|---|---|
| ***Ex.*** | ***HIL*** | ***HTL*** | ***EBL*** | ***EML*** | ***ETL1*** | ***ETL2*** | ***EIL*** |
| | Thickness / nm | Thickness / nm | Thickness / nm | Thickness / nm | Thickness / nm | Thickness / nm | Thickness / nm |
| C4 | HTM-a: p-doped(5%) 20 nm | HTM-a 220 nm | HTM-c 10 nm | TMM-1: TMM-2(29%):TEG(12%) 30 nm | ETM 10 nm | ETM: LiQ(50%) 30 nm | LiQ 1 nm |
| E5 | HTM-a: p-doped(5%) 20 nm | HTM-a 220 nm | HTM-1 10 nm | TMM-1: TMM-2(29%):TEG(12%) 30 nm | ETM 10 nm | ETM: LiQ(50%) 30 nm | LiQ 1 nm |

While the operating voltage and the lifetime remain similar, a strong increase in EQE is found for the OLED E5 comprising the compound according to the invention HTM-1, compared to the OLED C4 comprising the comparative compound HTM-c. OLED E5 shows an EQE of 15.9%, whereas OLED C4 shows an EQE of 14.9%.

### 5) Further device examples with compounds HTM-4 to HTM-7

OLEDs E6, E7, E8 and E9 are OLEDs according to the present application, which comprise the inventive compounds HTM-4, HTM-5, HTM-6 and HTM-7.

E6 shows the performance of the inventive compound HTM-4 as a HIL and HTL material in a singlet blue device (for detailed stack see below). Here, a lifetime LT80@60 mA/cm² of 290 h is found, along with good efficiency and voltage.

E7, E8 and E9 show the performance of the inventive compounds HTM-5, HTM-6 and HTM-7 as EBL materials in a triplet green device (for detailed stack see below). Here, lifetimes LT80@40 mA/cm² of 390 h (E7), 280 h (E8), and 310 h (E9) are found, along with good efficiency and voltage.

| **Table 1d: Device Setup** | | | | | | |
|---|---|---|---|---|---|---|
| ***Ex.*** | ***HIL*** | ***HTL*** | ***EBL*** | ***EML*** | ***ETL*** | ***EIL*** |
| | Thickness / nm | Thickness / nm | Thickness / nm | Thickness / nm | Thickness / nm | Thickness / nm |
| E6 | HTM-4: p-doped(5%) 20 nm | HTM-4 180 nm | EBM 10 nm | H:SEB(5%) 20 nm | ETM: LiQ(50%) 30 nm | LiQ 1 nm |
| E7 | HTM-a: p-doped(5%) 20 nm | HTM-a 220 nm | HTM-5 10 nm | TMM-1: TMM-2(28%):TEG(12%) 30 nm | ETM: LiQ(50%) 30 nm | LiQ 1 nm |
| E8 | HTM-a: p-doped(5%) 20 nm | HTM-a 220 nm | HTM-6 10 nm | TMM-1: TMM-2(28%):TEG(12%) 30 nm | ETM: LiQ(50%) 30 nm | LiQ 1 nm |
| E9 | HTM-a: p-doped(5%) 20 nm | HTM-a 220 nm | HTM-7 10 nm | TMM-1: TMM-2(28%):TEG(12%) 30 nm | ETM: LiQ(50%) 30 nm | LiQ 1 nm |

| **Table 3: Materials used** | | |
|---|---|---|
| 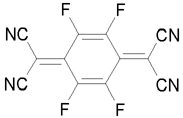 | 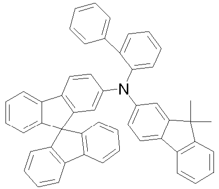 | 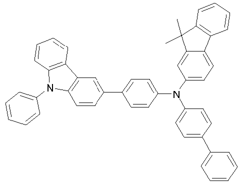 |
| p-dopant F4TCNQ | HTM-a | HTM-b |
| 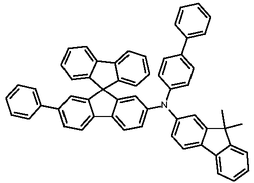 | 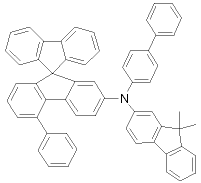 | 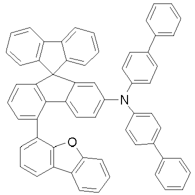 |
| HTM-c | HTM-1 | HTM-2 |
| 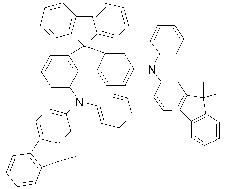 | 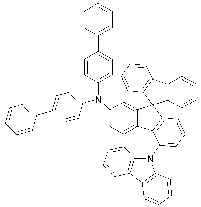 | 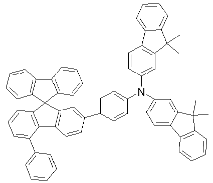 |
| HTM-3 | HTM-4 | HTM-5 |
| 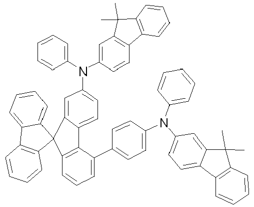 | 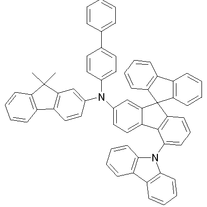 | 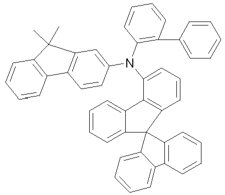 |
| HTM-6 | HTM-7 | EBM |
| 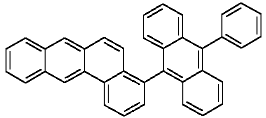 | 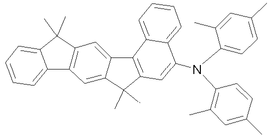 | 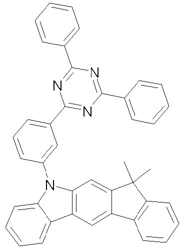 |
| H | SEB | TMM-1 |
| 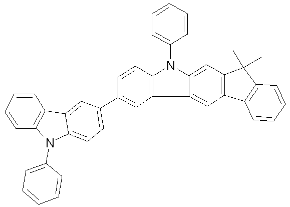 | 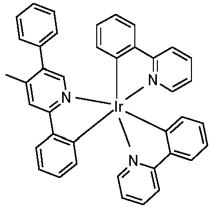 | 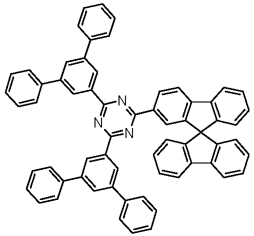 |
| TMM-2 | TEG | ETM |
| 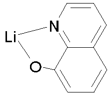 | | |
| LiQ | | |

## Claims

1. Compound of formula (I-A) 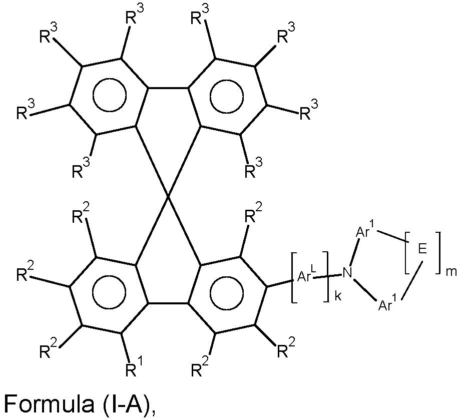 where the variables are defined as follows:
Index k shown in formula (I-A) is 0; and the group Ar^{L} shown in formula (I-A) is not present and the nitrogen atom and the spirobifluorene group are directly connected;
Ar^{L} is selected from aromatic ring systems having 6 to 40 aromatic ring atoms, which may be substituted by one or more radicals R⁴, and heteroaromatic ring systems having 5 to 40 aromatic ring atoms, which may be substituted by one or more radicals R⁴;
Ar¹ is, identically or differently, selected from aromatic ring systems having 6 to 40 aromatic ring atoms, which may be substituted by one or more radicals R⁴, and heteroaromatic ring systems having 5 to 40 aromatic ring atoms, which may be substituted by one or more radicals R⁴;
R¹ is 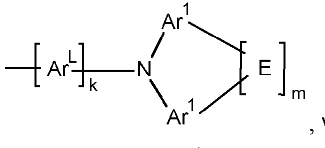 where k is 0 or 1, where in the case of k=0, the group Ar^{L} is not present and the nitrogen atom and the spirobifluorene group are directly connected;
R², R³ are selected, identically or differently on each occurrence, from H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, SCN, SF₅, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals selected from radicals R² and R³ may be connected to each other to form a ring; where the said alkyl, alkoxy, alkenyl and alkynyl groups and the said aromatic and heteroaromatic ring systems may in each case be substituted by one or more radicals R⁵, and where one or more CH₂ groups in the said alkyl, alkoxy, alkenyl and alkynyl groups may in each case be replaced by -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO or SO₂;
R⁴ is, identically or differently at each occurrence, selected from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁴ may be connected to each other to form a ring; where the said alkyl, alkoxy, alkenyl and alkynyl groups and the said aromatic and heteroaromatic ring systems may in each case be substituted by one or more radicals R⁵, and where one or more CH₂ groups in the said alkyl, alkoxy, alkenyl and alkynyl groups may in each case be replaced by -R⁵C=CR⁵-, -C≡C- , Si(R⁵)₂, C=O, C=NR⁵, -C(=O)O-, -C(=O)NR⁵-, NR⁵, P(=O)(R⁵), -O-, -S-, SO or SO₂;
-R⁵ is, identically or differently at each occurrence, selected from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁵ may be connected to each other to form a ring; where the said alkyl, alkoxy, alkenyl and alkynyl groups and the said aromatic and heteroaromatic ring systems may in each case be substituted by one or more radicals R⁶, and where one or more CH₂ groups in the said alkyl, alkoxy, alkenyl and alkynyl groups may in each case be replaced by -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO or SO₂;
R⁶ is selected, identically or differently at each occurrence, from H, D, F, CN, alkyl groups having 1 to 20 C atoms, aromatic ring systems having 6 to 40 C atoms, and heteroaromatic ring systems having 5 to 40 aromatic ring atoms; where two or more radicals R⁶ may be connected to each other to form a ring; and where the said alkyl groups, aromatic ring systems and heteroaromatic ring systems may be substituted by F and CN;
m is 0, so that the group E is not present and the groups Ar¹ are not connected.

2. Compound according to claim 1, **characterized in that** Ar^{L} is selected from divalent groups derived from benzene, biphenyl, terphenyl, naphthyl, fluorenyl, indenofluorenyl, spirobifluorenyl, dibenzofuranyl, dibenzothiophenyl, and carbazolyl, which may each be substituted by one or more radicals R⁴.

3. Compound according to claim 1 or 2, **characterized in that** groups Ar¹ are, identically or differently, selected from radicals derived from the groups phenyl, biphenyl, terphenyl, quaterphenyl, naphthyl, fluorenyl, benzofluorenyl, spirobifluorenyl, indenofluorenyl, dibenzofuranyl, dibenzothiophenyl, carbazolyl, benzofuranyl, benzothiophenyl, indolyl, quinolinyl, pyridyl, pyrimidyl, pyrazinyl, pyridazinyl and triazinyl, which are each optionally substituted by one or more radicals R⁴, or from combinations of 2 or 3 radicals derived from these groups, which are each optionally substituted by one or more radicals R⁴.

4. Compound according to one or more of claims 1 to 3, **characterized in that** group R¹ is 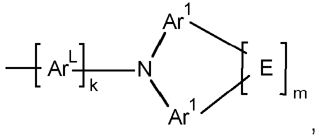 where index k is 0 and the nitrogen atom and the spirobifluorenyl group are directly connected.

5. Compound according to claim 4, **characterized in that**:
- Ar^{L} is selected from divalent groups derived from benzene, biphenyl, terphenyl, naphthyl, fluorenyl, indenofluorenyl, spirobifluorenyl, dibenzofuranyl, dibenzothiophenyl, and carbazolyl, which may each be substituted by one or more radicals R⁴;
- groups Ar¹ are, identically or differently, selected from radicals derived from the groups phenyl, biphenyl, terphenyl, quaterphenyl, naphthyl, fluorenyl, benzofluorenyl, spirobifluorenyl, indenofluorenyl, dibenzofuranyl, dibenzothiophenyl, carbazolyl, benzofuranyl, benzothiophenyl, indolyl, quinolinyl, pyridyl, pyrimidyl, pyrazinyl, pyridazinyl and triazinyl, which are each optionally substituted by one or more radicals R⁴, or from combinations of 2 or 3 radicals derived from these groups, which are each optionally substituted by one or more radicals R⁴;
- R² is H;
- R³ is H;
- R⁴ is selected, identically or differently, from H, F, CN, Si(R⁵)₃, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where two or more radicals R⁴ may be connected to each other to form a ring; where the said alkyl groups and the said aromatic and heteroaromatic ring systems may in each case be substituted by one or more radicals R⁵; and
- R⁵ is selected, identically or differently, from H, F, CN, Si(R⁶)₃, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where two or more radicals R⁵ may be connected to each other to form a ring; where the said alkyl groups and the said aromatic and heteroaromatic ring systems may in each case be substituted by one or more radicals R⁶.

6. Compound according to one or more of claims 1 to 5, **characterized in that** groups R¹ are groups which conform to the following groups
| | |
|---|---|
| 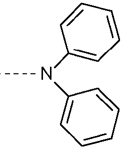 | 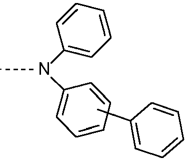 |
| R-165 | R-166 |
| 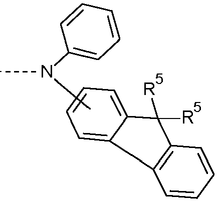 | 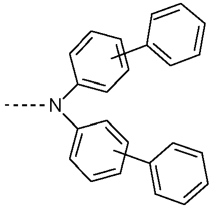 |
| R-167 | R-168 |
| 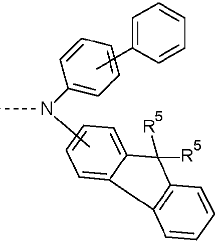 | 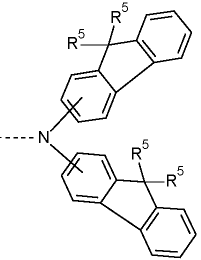 |
| R-169 | R-170 |
| 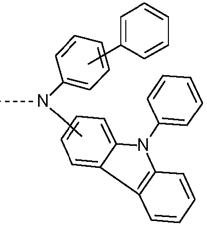 | 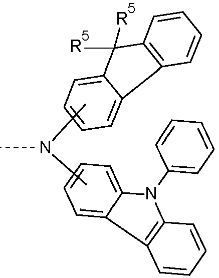 |
| R-171 | R-172 |
| 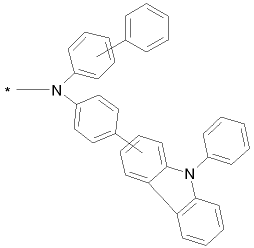 | 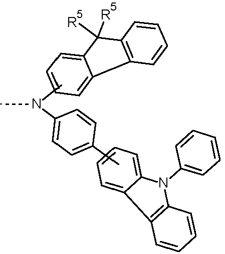 |
| R-173 | R-174 |
| 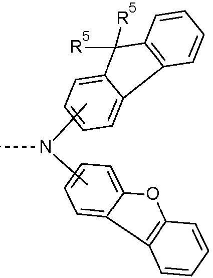 | 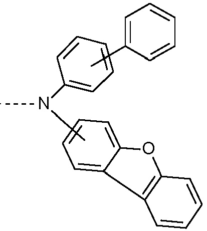 |
| R-175 | R-176 |
| 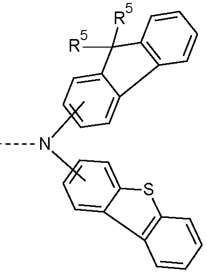 | 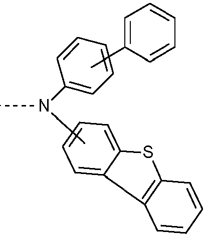 |
| R-177 | R-178 |
| 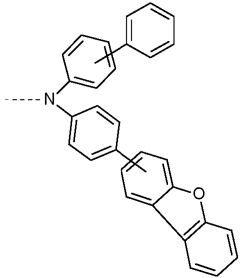 | 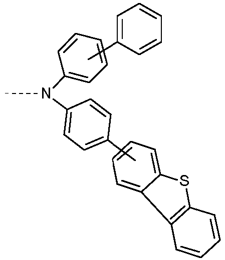 |
| R-179 | R-180 |
| 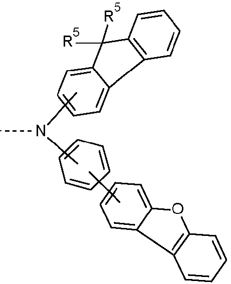 | 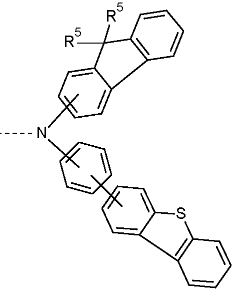 |
| R-181 | R-182 |
| 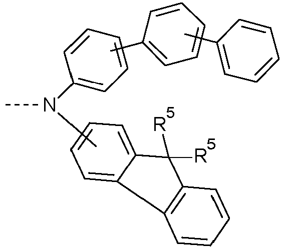 | 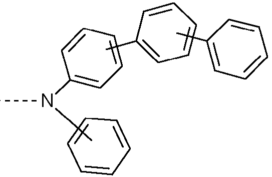 |
| R-183 | R-184 |
| 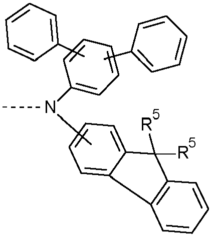 | 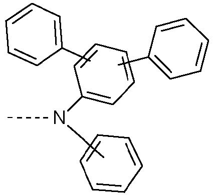 |
| R-185 | R-186 |
| 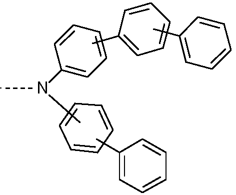 | 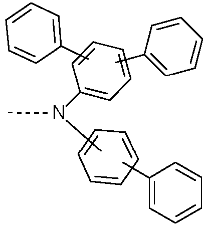 |
| R-187 | R-188 |
| 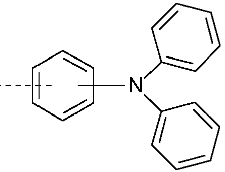 | 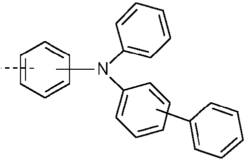 |
| R-189 | R-190 |
| 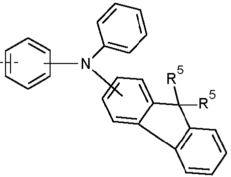 | 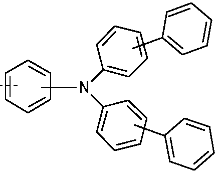 |
| R-191 | R-192 |
| 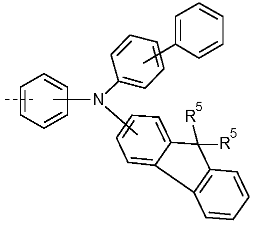 | 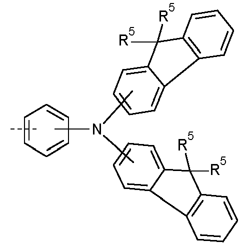 |
| R-193 | R-194 |
| 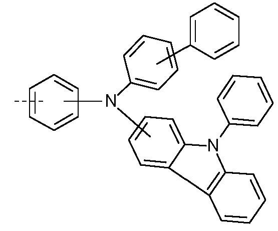 | 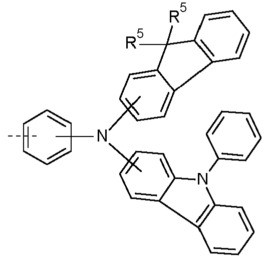 |
| R-195 | R-196 |
| 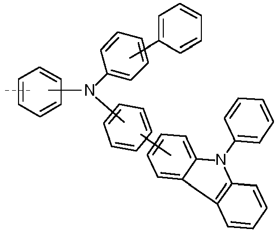 | 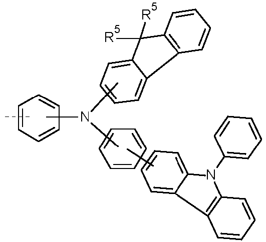 |
| R-197 | R-198 |
| 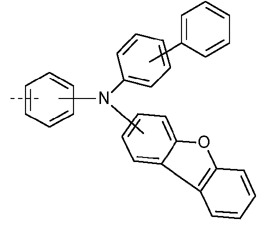 | 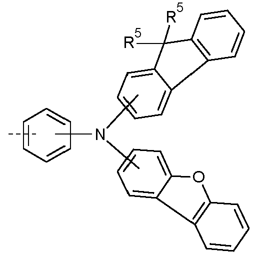 |
| R-199 | R-200 |
| 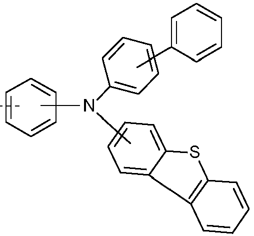 | 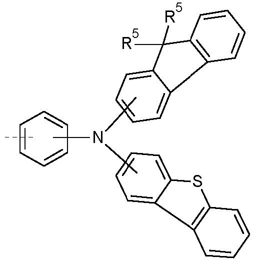 |
| R-201 | R-202 |
| 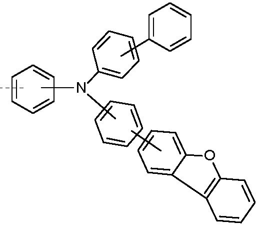 | 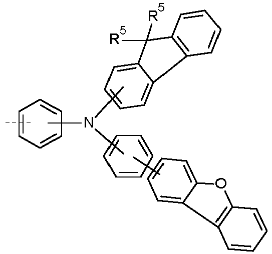 |
| R-203 | R-204 |
| 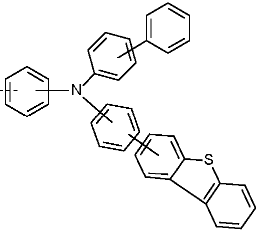 | 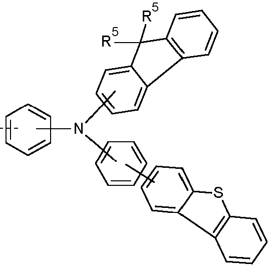 |
| R-205 | R-206 |
| 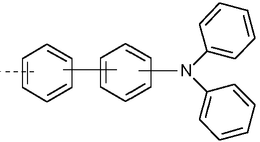 | 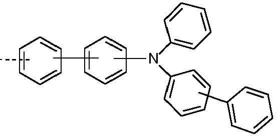 |
| R-207 | R-208 |
| 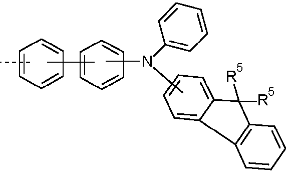 | 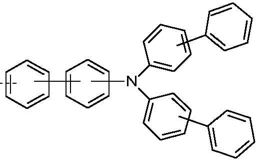 |
| R-209 | R-210 |
| 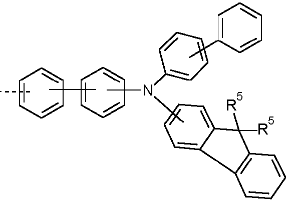 | 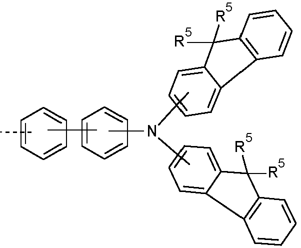 |
| R-211 | R-212 |
where the groups may be substituted at the free positions with groups R⁵, and where the dotted line symbolizes the bonding position to the spirobifluorene moiety of formula (I-A).

7. Compound according to one or more of claims 1 to 6, **characterized in that** R² is H.

8. Compound according to one or more of claims 1 to 7, **characterized in that** R³ is selected, identically or differently, from H, F, methyl, tert-butyl, and phenyl.

9. Process for preparation of a compound according to one or more of claims 1 to 8, **characterized in that** it comprises the reactions steps
1) metallation of a biphenyl derivative which has one reactive group in a position which is ortho to the phenyl-phenyl bond, and which bears two additional reactive groups in other positions, where the metallation takes place in the position which is ortho to the phenyl-phenyl bond;
2) addition of the metallated biphenyl derivative to a fluorenone derivative;
3) cyclisation of the resulting addition product to a spirobifluorene derivative, where the cyclisation takes place under acidic conditions or with a Lewis acid, and where the spirobifluorene derivative bears two reactive groups; and
4) coupling of the spirobifluorene derivative with groups selected from aromatic ring systems, heteroaromatic ring systems and amine groups, in the positions of the two reactive groups.

10. Oligomer, polymer or dendrimer, comprising one or more compounds of formula (I-A) according to one or more of claims 1 to 8, where the bond(s) to the polymer, oligomer or dendrimer may be localised at any desired positions in formula (I-A) substituted by R¹, R², R³ or R⁴.

11. Formulation, comprising at least one compound of formula (I-A) according to one or more of claims 1 to 8 or at least one polymer, oligomer or dendrimer according to claim 10, and at least one solvent.

12. Electronic device, comprising at least one compound according to one or more of claims 1 to 8, or at least one polymer, oligomer or dendrimer according to claim 10.

13. Electronic device according to claim 12, **characterized in that** it is an organic electroluminescent device, comprising anode, cathode and at least one emitting layer, where at least one organic layer of the device, which is an emitting layer, a hole transport layer, an electron blocking layer or a hole injection layer, comprises the at least one compound.

14. Use of a compound according to one or more of claims 1 to 8, or of a polymer, oligomer or dendrimer according to claim 10, in an electronic device.
